# EUROPEAN PATENT APPLICATION

(11) **EP 1 881 003 A1**
(43) Date of publication of application: **23.01.2008**
(21) Application number: 06732234.7
(22) Date of filing: 21.04.2006
(51) Int. Cl.: C07K 7/02, C07C 269/06, C07C 271/22, C07B 61/00, C12N 15/09, C12N 15/63

(54) **COMPOUND HAVING AMINO ACID RESIDUE OR PEPTIDE RESIDUE AND PROCESS FOR PRODUCING THE SAME**

(30) Priority: 22.04.2005 JP 2005125206; 23.05.2005 JP 2005148880; 25.11.2005 JP 2005340398
(71) Applicant: JAPAN CROWN CORK CO. LTD., Tokyo 100-8522 (JP)
(72) Inventor: IKEDA, Hisafumi, Kyoto-shi, Kyoto 615006 (JP); TONOSAKI, Madoka c/o CREDIA JAPAN CO., LTD., Higashigawa, Mikurumamichi 6020841 (JP)
(74) Representative: HOFFMANN EITLE
(86) International application number: PCT/JP2006/308476
(87) International publication number: WO 2006/115230

(57) **Abstract**

An object of the present invention is to provide a compound with a structure wherein amino acid or oligopeptide side chains are bonded to a main chain.

The present invention provides a compound represented by the following formula (1). 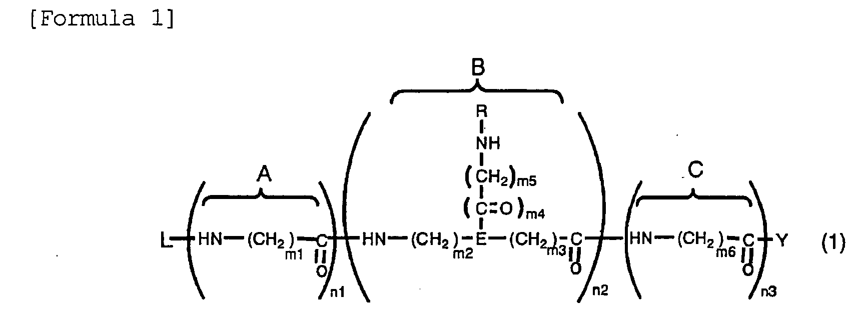 wherein n1 to n3, and m1 to m6 are certain integers;
Y is a hydroxyl or an amino group;
E is N or CH;
R is an amino acid residue, or a peptide residue consisting of 2 to 100 amino acid residues; and
L is a hydrogen atom, a group containing a lipid group, a group containing a fatty acid residue, or a group containing a fluorescent group.

## Description

### TECHNICAL FIELD

The present invention relates to compounds that contain amino acid or peptide residues, and that are capable of effectively expressing the functionalities of said residues. The present invention further relates to processes for producing such compounds.

### BACKGROUND OF THE INVENTION

Amino acids and oligopeptides are known for having various useful actions according to their kind. For example, arginine and oligopeptide are known for their ability to permeate cell membranes, and have therefore been used as reagents for introducing intended substances into cells. It is also known that compounds that contain a plurality of such amino acids or oligopeptides bound together may advantageously reinforce desired actions, improve safety, etc.

However, among such compounds that contain amino acids or oligopeptides, those with a structure wherein side chains with amino acids or oligopeptides are bonded to a main chain have not been proposed.

### DISCLOSURE OF THE INVENTION

### PROBLEMS TO BE SOLVED BY THE INVENTION

Objects of the present invention are to provide compounds with a structure wherein amino acid or oligopeptide side chains are bonded to a main chain, with such compounds being protected by protecting groups, and processes for producing such compounds.

### MEANS FOR SOLVING THE PROBLEMS

The present invention provides compounds represented by the following formulae (1) and (1a), and processes for producing these compounds:
Item 1 A compound represented by the following formula (1) 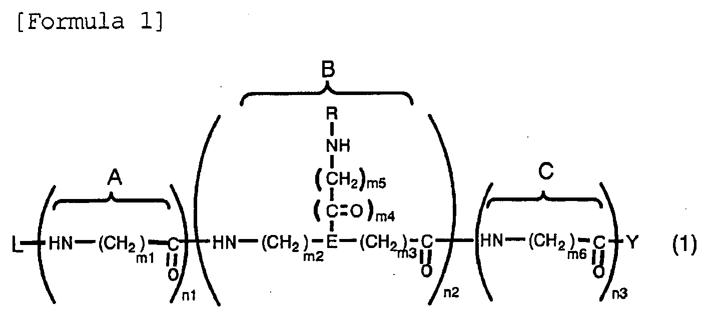 wherein n1 is an integer from 0 to 10, n2 is an integer from 1 to 50, and n3 is an integer from 1 to 10; m1 is an integer from 0 to 100, m2 is an integer from 0 to 100, m3 is an integer from 0 to 100, m4 is an integer of 0 or 1, m5 is an integer from 0 to 100, and m6 is an integer from 0 to 100;
   Y is a hydroxyl group or an amino group;
   E is N or CH;
   R is an amino acid residue, or a peptide residue consisting of 2 to 100 amino acid residues;
   L is a hydrogen atom, a group containing a lipid group, a group containing a fatty acid residue, or a group containing a fluorescent group;
   when n1 is at least 2, each m1 in repeating unit A may be the same or different;
   when n2 is at least 2, each of m2 to m5 and R may be the same or different in each repeating unit B; and
   when n3 is at least 2, each m6 in repeating unit C may be the same or different.
Item 2 A compound of Item 1, wherein L in formula (1) is a group containing a phospholipid group.
Item 3 A compound of Item 1, wherein R in formula (1) is an amino acid residue selected from the group consisting of arginine, lysine and serine; or a peptide residue comprising at least one of these amino acid residues.
Item 4 A compound of Item 1, wherein R in formula (1) is a peptide residue comprising at least one amino acid residue selected from the group consisting of arginine, lysine and serine; said peptide residue consisting of a total of 2 to 20 amino acid residues.
Item 5 A compound of Item 1, wherein R in formula (1) represents a peptide residue consisting of 2 to 5 arginine residues.
Item 6 A compound of Item 1, wherein in formula (1) n1 is an integer from 0 to 2, n2 is an integer from 1 to 10, and n3 is an integer from 0 to 2.
Item 7 A compound of Item 1, wherein in formula (1) E is N; m2 is 2, m3 and m4 are 1, and m5 is 5.
Item 8 A compound of Item 1, wherein in formula (1) E is CH; m2, m3 and m4 are 0, and m5 is 4.
Item 9 A compound represented by the following formula (1a): 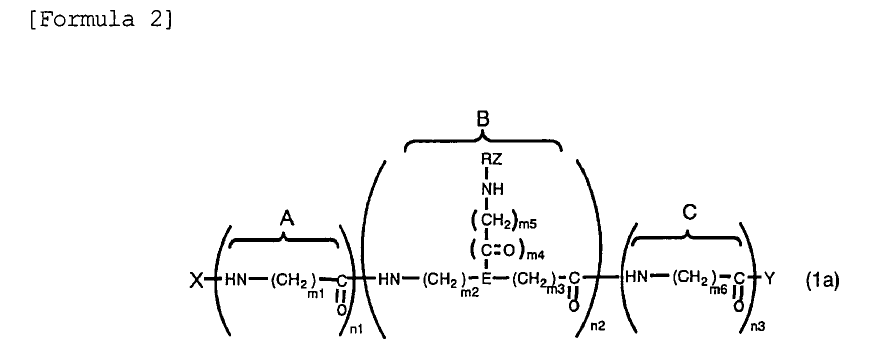 wherein n1 to n3, m1 to m6, E and R are as defined in Item 1;
   Y is a hydroxyl group;
   X is a protecting group;
   Z is a protecting group Za different from protecting group X, or a protecting group Zb identical to protecting group X;
   RZ represents such a protecting group Z bonded to a functional group of amino acid or peptide residue R;
   when n1 is at least 2, each m1 in repeating unit A may be the same or different;
   when n2 is at least 2, each of m2 to m5 and RZ may be the same or different in each repeating unit B; and
   when n3 is at least 2, each m6 in repeating unit C may be the same or different.
Item 10 A compound of Item 9, wherein in formula (1a) X is a tertiary-butoxycarbonyl group or a 9-fluorenylmethoxycarbonyl group.
Item 11 A compound of Item 9, wherein R in formula (1) is an amino acid residue selected from the group consisting of arginine, lysine and serine, or a peptide residue comprising at least one of these amino acid residues; said peptide residue consisting of a total of 2 to 20 amino acid residues.
Item 12 A compound of Item 9, where R in formula (1) is a peptide residue comprising at least one amino acid residue selected from the group consisting of arginine, lysine and serine; said peptide residue consisting of a total of 2 to 20 amino acid residues.
Item 13 A compound of Item 9, wherein R in formula (1) represents a peptide residue consisting of 2 to 5 arginine residues.
Item 14 A compound of Item 9, wherein in formula (1) n1 is an integer from 0 to 2, n2 is an integer from 1 to 10, and n3 is an integer from 0 to 2.
Item 15 A compound of Item 9, wherein in formula (1) E is N; m2 is 2, m3 and m4 are 1, and m5 is 5.
Item 16 A compound of Item 9, wherein in formula (1) E is CH; m2, m3 and m4 are 0, and m5 is 4.
Item 17 A conjugated compound synthesized by a condensation reaction between a compound of Item 9 and a compound containing an amino group or a hydroxyl group.
Item 18 A conjugated compound of Item 17, wherein the compound containing an amino group is a PNA monomer or a PNA oligomer.
Item 19 A process for producing a compound represented by the following formula (1) 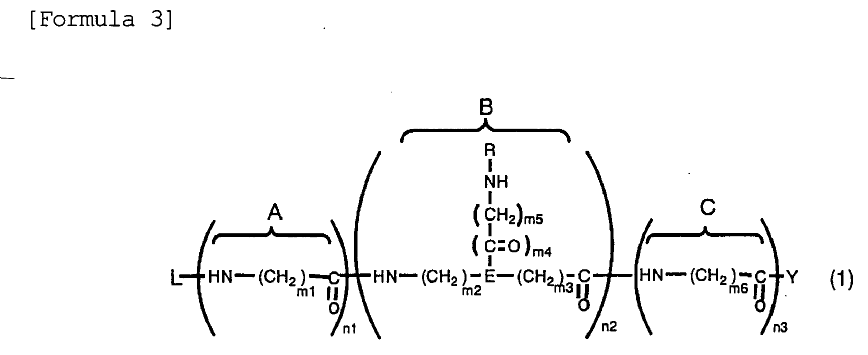 wherein n1 is an integer from 0 to 10, n2 is an integer from 1 to 50, and n3 is an integer from 1 to 10; m1 is an integer from 0 to 100, m2 is an integer from 0 to 100, m3 is an integer from 0 to 100, m4 is an integer of 0 or 1, m5 is an integer from 0 to 100, and m6 is an integer from 0 to 100;
   Y is a hydroxyl group or an amino group;
   E is N or CH;
   R is an amino acid residue, or a peptide residue consisting of 2 to 100 amino acid residues;
   L is a hydrogen atom, a group containing a lipid group, a group containing a fatty acid residue, or a group containing a fluorescent group;
   when n1 is at least 2, each m1 in repeating unit A may be the same or different;
   when n2 is at least 2, each of m2 to m5 and R may be the same or different in each repeating unit B; and
   when n3 is at least 2, each m6 in repeating unit C may be the same or different;
   the process comprising the steps 1-1 to 1-5 below:
   Step 1-1: a step of condensing a compound represented by the following formula (I) 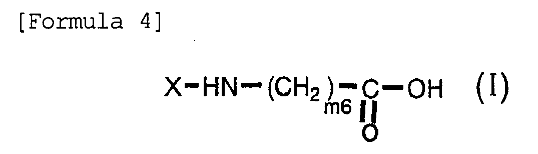 wherein m6 is as defined above,
      with a solid-phase resin or a solid-phase compound, removing protecting group(s) from the compound condensed with solid-phase resin or solid-phase compound, and subjecting the compound represented by formula (I) to such condensation n3-1 more times so as to polymerize it, thereby obtaining a compound represented by the following formula (i) 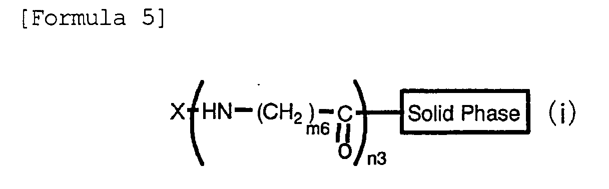 wherein n3, m6 and X are as defined above; and Solid Phase represents a solid-phase resin or a solid-phase compound;
   Step 1-2: a step of condensing n2 times a compound represented by the following formula (II) 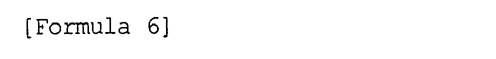 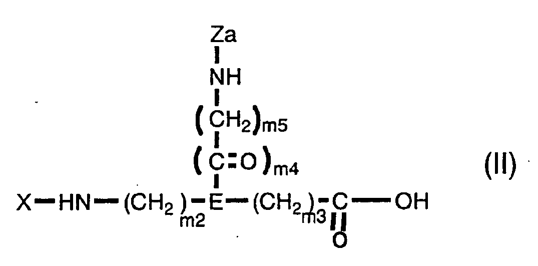 wherein m2 to m5 and X are as defined above, Za is a protecting group different from protecting group X,
      with the compound represented by formula (i), thereby obtaining a compound represented by the following formula (ii) 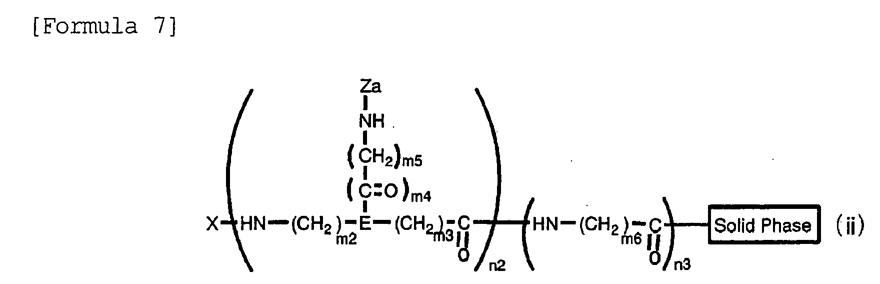 wherein n2, n3, m2 to m6, X, Za and Solid Phase are as defined above;
   Step 1-3: a step of condensing n1 times a compound represented by the following formula (III) 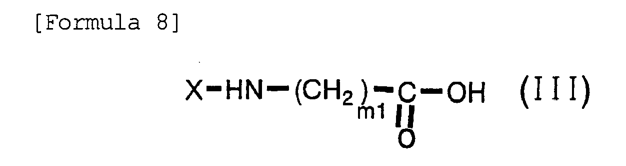 wherein m1 and X are as defined above,
      with the compound represented by formula (ii), thereby obtaining a compound represented by the following formula (iii) 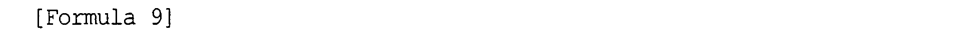 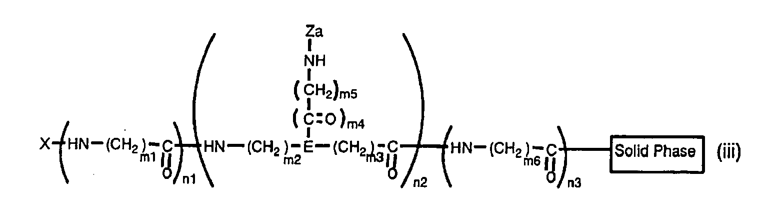 wherein n1 to n3, m1 to m6, X, Za and Solid Phase are as defined above;
   Step 1-4: a step of condensing, 1 to 100 times with the compound represented by formula (iii), one or more amino acids wherein protecting group Za is bonded to a functional group other than a carboxyl group bonded to an α carbon atom, thereby obtaining a compound represented by the following formula (iv) 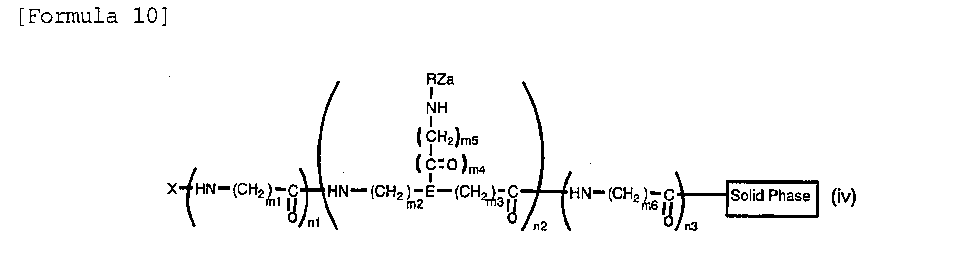 wherein n1 to n3, m1 to m6, X, RZa and Solid Phase are as defined above; and
   Step 1-5: a step of obtaining a compound represented by formula (1) by removing the solid-phase resin or solid-phase compound from the compound represented by formula (iv) to form terminal - COOH or -CONH₂ groups, and removing protecting groups X and Za.
Item 20 A process for producing a compound represented by the following formula (1a) 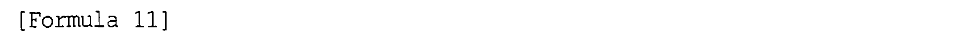 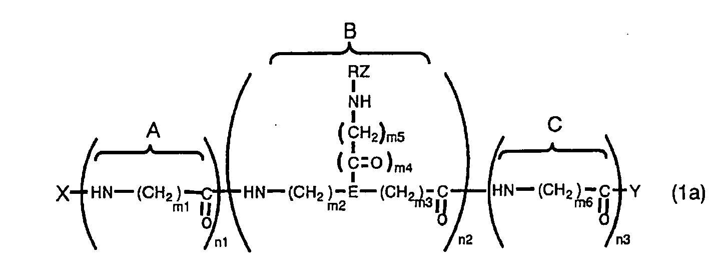 wherein n1 to n3, m1 to m6, E, X and RZ are as defined above;
   Y is a hydroxyl group;
   when n1 is at least 2, each m1 in repeating unit A may be the same or different;
   when n2 is at least 2, each of m2 to m5 and RZ may be the same or different in each repeating unit B; and
   when n3 is at least 2, each m6 in repeating unit C may be the same or different;
   the process comprising the steps 3-1 to 3-5 below:
   Step 3-1: a step of subjecting a compound represented by the following formula (I) 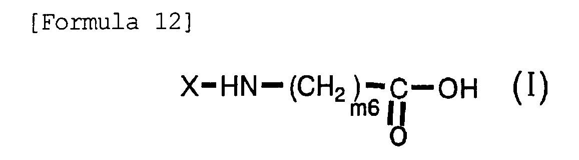 wherein m6 is as defined above,
      to condensation polymerization with a solid-phase resin or a solid-phase compound containing an amino group, removing protecting group X from the compound condensed with the solid-phase resin or solid-phase compound, and subjecting the compound represented by formula (I) to such condensation n3-1 more times so as to polymerize it, thereby obtaining a compound represented by the following formula (i) 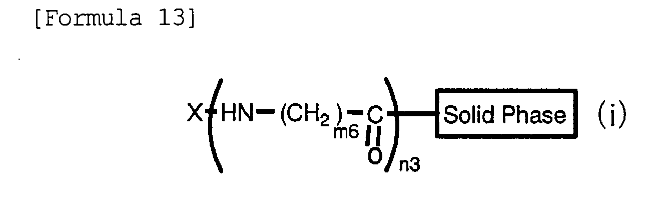 wherein n3, m6 and X are as defined above; and Solid Phase represents a solid-phase resin or solid-phase compound;
   Step 3-2: a step of condensing n2 times a compound represented by the following formula (II) 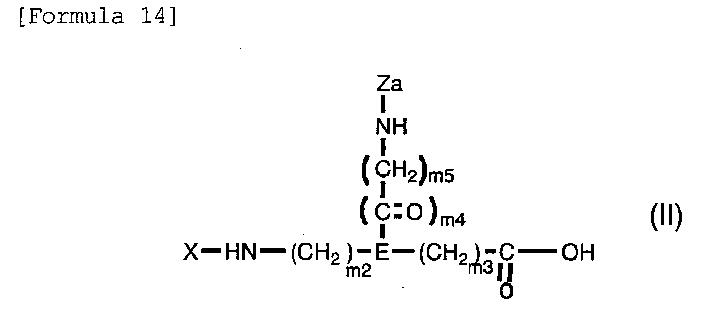 wherein m2 to m5, X and Za are as defined above,
      with the compound represented by formula (i), thereby obtaining a compound represented by the following formula (ii) 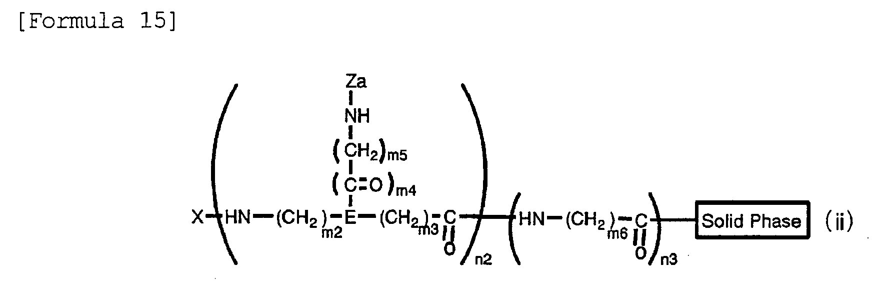 wherein n2, n3, m2 to m6, X, Za and Solid Phase are as defined above;
   Step 3-3: a step of condensing n1 times a compound represented by the following formula (III) 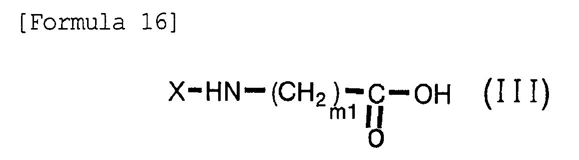 wherein m1 and X are as defined above,
      with the compound represented by formula (ii), thereby obtaining a compound represented by the following formula (iii) 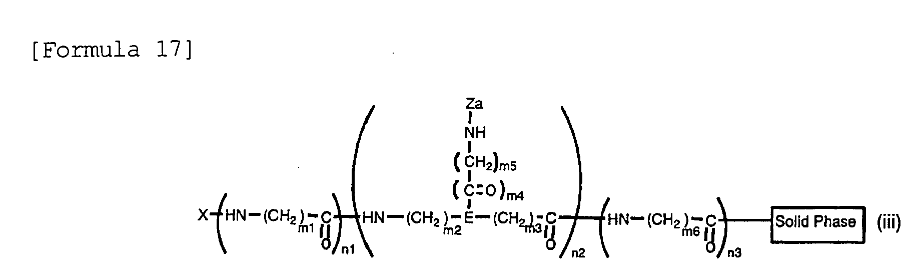 wherein n1 to n3, m1 to m6, X, Za and Solid Phase are as defined above;
   Step 3-4: a step of obtaining a compound represented by the following formula (iv) 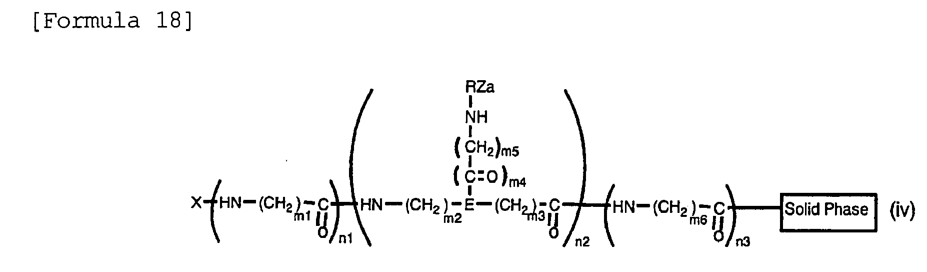 wherein n1 to n3, m1 to m6, X, RZa and Solid Phase are as defined above,
      by condensing, 1 to 100 times the compound represented by formula (iii), with one or more amino acids in whichprotecting group Z is bonded to an amino group; and
   Step 3-5: a step of obtaining a compound represented by formula (1a) by substituting as necessary protecting group Za of the compound represented by formula (iv) with protecting group Zb, and removing the solid-phase resin or solid-phase compound to form terminal -COOH without removing protecting groups X and Z.
      The term "membrane permeability" used hereinafter in the specification refers to the ability to permeate a cell membrane.

### BRIEF DESCRIPTION OF THE DRAWINGS

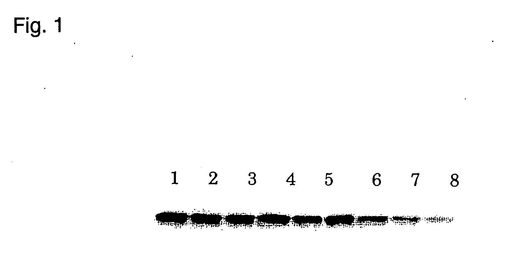

[FIG. 1] FIG. 1 shows test results (detection result of bcl-2 protein expression by a Western blot method) in Reference Test Example 4. In the figure, each lane represents the following.
Lane 1: negative control;
Lane 2: transfection of bcl-2 siRNA (added 45 pmpl/well), XtremeGene;
lane 3: transfection of bcl-2 siRNA (added 90 pmpl/well), XtremeGene;
lane 4: transfection of GFP siRNA (added 42.5 pmpl/well), carrier for nucleic acid transfection of Reference Example3;
lane 5: transfection of bcl-2 siRNA (added amount: 13.5 pmpl/well), carrier for nucleic acid transfection of Reference Example 3;
lane 6: transfection of bcl-2 siRNA (added amount: 45 pmpl/well), carrier for nucleic acid transfection of Reference Example 3;
lane 7: transfection of bcl-2 siRNA (added amount: 135 pmpl/well), carrier for nucleic acid transfection of Reference Example 3;
lane 8: transfection of bcl-2 siRNA (added amount: 225 pmpl/well), carrier for nucleic acid transfection of Reference Example 3 ;
lane 9: transfection of bcl-2 siRNA (added amount: 450 pmpl/well), carrier for nucleic acid transfection of Reference Example 3

### BEST MODE FOR CARRYING OUT THE INVENTION

### 1. Compound represented by formula (1)

The present invention provides a compound represented by the following formula (1) with a structure wherein amino acid or oligopeptide side chains are bonded to a main chain. 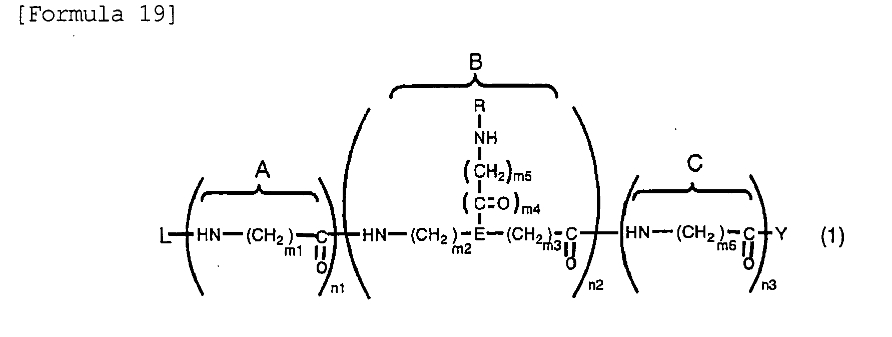 wherein n1 is the number of times that repeating unit A repeats, and is an integer from 0 to 10, preferably an integer from 0 to 5, and more preferably an integer from 0 to 2.

In repeating unit A, m1 is an integer from 0 to 100, preferably an integer from 0 to 30, and more preferably an integer from 0 to 11. When n1 is an integer of at least 2, i.e., when repeating unit A repeats at least twice, each m1 in repeating unit A may be the same or different.

Further, in formula (1), n2 is the number of times that repeating unit B repeats, and is an integer from 1 to 50, preferably an integer from 1 to 20, and more preferably an integer from 1 to 10.

In repeating unit B, m2 is an integer from 0 to 100, preferably an integer from 0 to 20, and more preferably an integer from 0 to 2. m3 is an integer from 0 to 100, preferably an integer from 0 to 20, and more preferably an integer from 0 to 2. m4 is an integer of 0 or 1. m5 is an integer from 0 to 100, preferably an integer from 0 to 30, and more preferably an integer from 0 to 11.

In repeating unit B, E is N or CH. In repeating unit B, when E is N, preferable examples of compounds are those wherein m2 is 0 to 2, with 2 being preferable; m3 is 0 or 1, with 0 being preferable; m4 is 1; and m5 is 0 to 11, with 5 being preferable. Further, in repeating unit B, when E is CH, preferable examples of compounds are those wherein m2 is 0 to 2, with 0 being preferable; m3 is 0 or 1, with 0 being preferable; m is 0; and m5 is 0 to 11, with 5 being preferable.

In repeating unit B, R is an amino acid residue, or a peptide residue consisting of 2 to 100 amino acid residues. R representing an amino acid residue is not limited, and may be a natural amino acid residue or a non-natural amino acid residue. To impart membrane permeability to a compound represented by formula (1), preferable residues are arginine, lysine, and serine, with arginine residue being more preferable.

The type of amino acid residues constituting the peptide residue represented by R is not limited as long as the peptide residue consists of 2 to 100 amino acid residues. To impart membrane permeability to a compound represented by formula (1), for example, usable peptide residue may be those containing at least one amino acid residue selected from the group consisting of arginine, lysine, and serine; preferably those consisting of arginine, lysine, and serine as constituent amino acid residues; and more preferably those consisting of arginine and/or lysine as constituent amino acid residues.

When such peptide residue includes a lysine residue as a constituent amino acid residue, at least one of the amino groups at the α and ε positions of the lysine residue can form a peptide bond to a carboxyl group of an adjacent amino acid.

The number of amino acid residues constituting a peptide residue is preferably 2 to 50, more preferably 2 to 20, and still more preferably 2 to 3.

A preferable example of the form of R is a peptide residue consisting of 2 to 5 arginine residues, with a tri-arginine residue consisting of three arginine residues being particularly preferable. The compound represented by formula (1) can have far better membrane permeability by containing such peptide residues.

Repeating unit B contains an amino acid or peptide residue in which the carboxyl group of the constituent amino acid at the C-terminal is bonded in a dehydrated and condensed form to an amino group on the side chain of the repeating unit. That is, the amino acid or peptide residue represented by R is equivalent to a group in which -OH has been eliminated from the carboxyl group of the amino acid or the constituent amino acid at the C-terminal of the peptide.

In repeating unit B, when m2 is an integer of at least 2, i.e., when repeating unit B repeats at least twice, each of m2 to m5 and R may be the same or different in each repeating unit B.

In formula (1), n3 is the number of times that repeating unit C repeats, and is an integer from 0 to 10, preferably an integer from 0 to 5, and more preferably an integer from 0 to 2.

In repeating unit C, m6 is an integer from 0 to 100, preferably an integer from 0 to 30, and more preferably an integer from 0 to 11. When n3 is an integer of at least 2, i.e., when repeating unit C repeats at least twice, each m6 in repeating unit C may be the same or different.

In formula (1), specific examples of n1 to n3 include n1 being an integer from 0 to 2, n2 being an integer from 1 to 10, and n3 being an integer from 0 to 2, and preferably n1 being an integer of 1 or 2, n2 being an integer from 4 to 6, and n3 being an integer of 1 or 2.

In formula (1), Y is a hydroxyl group or an amino group.

In formula (1), L is a hydrogen atom, a group containing a lipid group, a group containing a fatty acid residue, or a group containing a fluorescent group.

Lipids constituting a group containing a lipid group are not limited, but preferable examples include phospholipids such as phosphatidyl ethanolamine, phosphatidyl choline, phosphatidyl serine, phosphatidyl inositol, sphingomyelin, sphingosine, ceramide, plasmalogen, phosphatidylgrycerol, phosphatidic acids, etc.; glyceroglycolipids such as galactosyldiglycerol, 6-sulfoquinovosyl diacylglycerol, etc.; sphingo-glycolipids such as galactocerebroside, etc.; steroids; prostaglandins; etc. Among these, preferable are phospholipids, with phosphatidyl ethanolamine being particularly preferable. Fatty acid residues constituting these lipid groups are not limited, but examples include saturated or unsaturated fatty acid residues having 4 to 30 (preferably 12 to 20) carbon atoms. Specific examples of such fatty acid residues include groups such as lauroyl, myristoyl, palmitoyl, stearoyl, oleoyl, linoleyl, etc. Among these fatty acid residues, preferable are palmitoyl and oleoyl groups.

A group containing a lipid group may be a lipid group itself, but also may be lipid groups with a linker bonded thereto. Usable linkers are those with a structure shown in (a) and (b) below.

### [Formula 20]

-CO-(CH₂)ᵢ-CO- (a)

-O(CH₂CH₂O)ⱼ- (b)

wherein i is an integer from 1 to 20, preferably an integer from 1 to 8, and j is an integer from 1 to 1,000, preferably an integer from 1 to 21.

Specific examples of groups in which a linker is bonded to a lipid group are those represented by the formula (L1) below when constituent lipids contain an amino group; and those represented by the formula (L2) below when constituent lipids contain a carboxyl group. 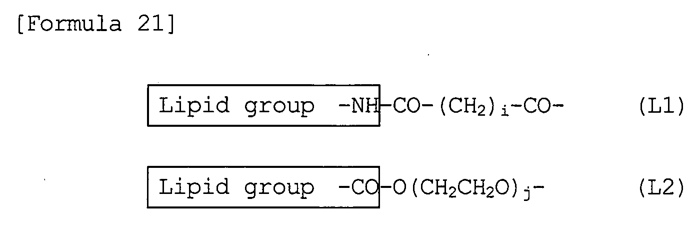 In formulae (L1) and (L2), i and j are as defined above.

A group containing a lipid group may be those in which the above lipid groups are bonded.

When L is a group containing a fatty acid residue, such a group may be a fatty acid residue itself, or a group in which a linker is bonded to a fatty acid residue. The term "fatty acid residue" used herein refers to any group in which -OH is eliminated from a carboxyl group of a fatty acid. Specific examples of such fatty acid residues include saturated or unsaturated fatty acid residues having 4 to 30 (preferably 12 to 20) carbon atoms. Specific examples of such fatty acid residues include groups such as lauroyl, myristoyl, palmitoyl, stearoyl, oleoyl, linoleyl, etc. Among these fatty acid residues, preferable are palmitoyl and oleoyl groups.

In the case of a group in which a linker is bonded to a fatty acid residue, usable linkers are those with structures shown in (a) and (b) above. Specific examples of such a group in which a linker is bonded to a fatty acid residue include those represented by the following formula (L3). 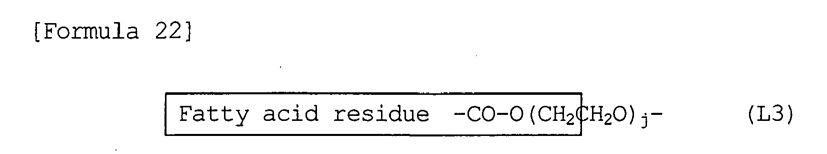 wherein j is as defined above

A group containing a fatty acid residue may be one in which at least 2 (e.g., 2 to 8, preferably 2 to 4) fatty acid residues are bonded. Specific examples of such groups in which at least 2 fatty acid residues are bonded include those shown below.

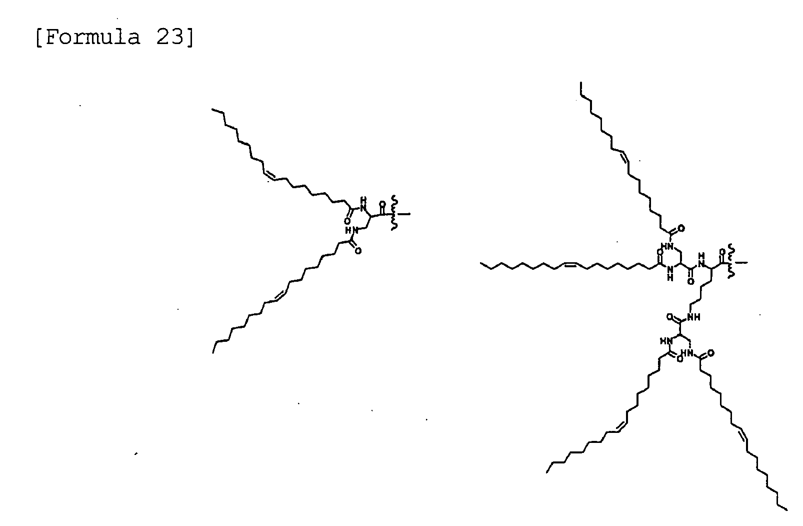

When L is a group containing a fluorescent group, such a group may be a fluorescent group itself, or a group in which a linker is bonded to a lipid group. The term "fluorescent group" used herein refers to any group with fluorescence-emitting properties. Usable fluorescent groups include known fluorescent compounds. The structure below shows an example of such a fluorescent group.

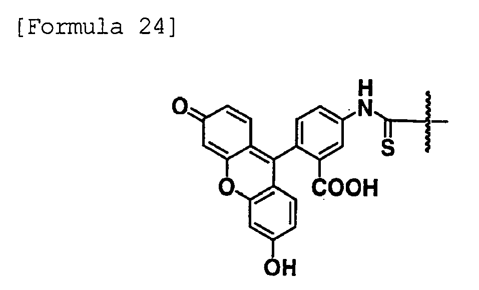

In the case of a group in which a linker is bonded to a fatty acid residue, usable linkers are those with a structure shown in (a) and (b) above.

When L in formula (1) is a group containing a lipid group or a group containing a fatty acid residue, the compounds represented by formula (1) are likely to have improved membrane permeability and enhanced compatibility with fatty components.

Further, when L in formula (1) is a group containing a fluorescent group, the compounds represented by formula (1) can be fluorescent-labeled, thereby enabling the detection of the compound according to the presence or absence of the fluorescence.

### 2. Compound represented by formula (1a)

The present invention further provides a compound represented by the following formula (1a) with a structure wherein amino acid or oligopeptide side chains are bonded to a main chain. 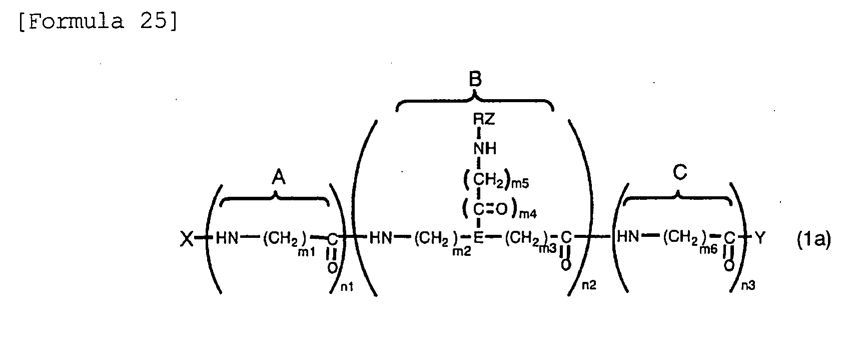 wherein n1 to n3, m1 to m6, E and R are as defined above. In formula (1a), Y is a hydroxyl group.
In formula (1a), X is a protecting group. Further, in formula (1a), Z is a protecting group different from the protecting group X, which is indicated as Za, or a protecting group identical with the protecting group X, which is indicated as Zb. The term "protecting group" used herein refers to a group that protects a part in a specific region of the compound represented by formula (1a) so as to not be influenced by reactions such as oxidation, reduction, hydrolysis, condensation, etc., occurring in other regions of the compound, and the group reverting to a hydrogen atom or a hydroxyl group by the protecting group's removal under certain conditions. Representative examples of such protecting groups include tertiary-butoxycarbonyl (Boc group) and 9-fluorenylmethoxycarbonyl (Fmoc group), but more protecting groups can be exemplified as follows. Preferable protecting group X is Boc group or Fmoc group. Preferable protecting group Z is protecting group different from the protecting group X, which is protecting group Za.

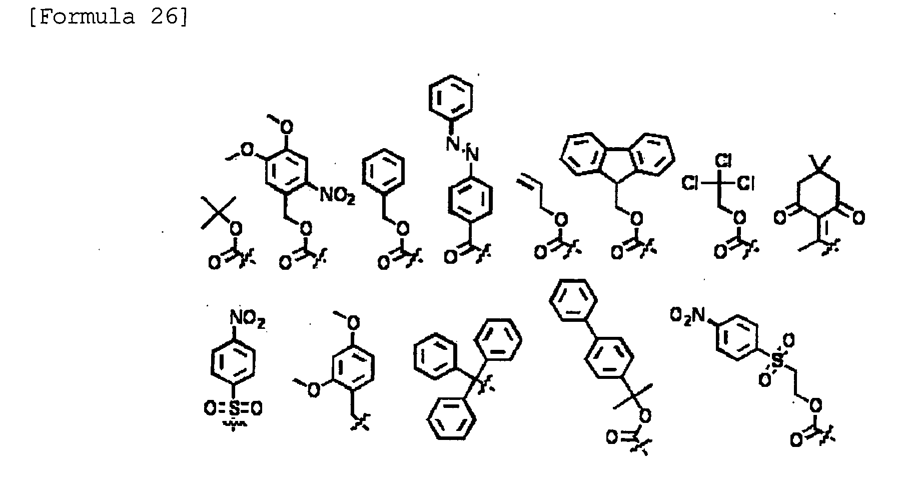

In particular, when a protecting group X is a Boc group or Fmoc group, a protecting group Z is preferably an Alloc group as shown below.

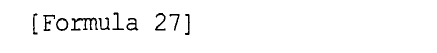

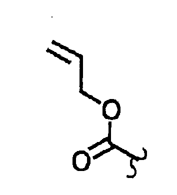

In formula (1a), RZ represents protecting group Z bonded to a functional group of the above-mentioned amino acid or peptide residue represented by R. When at least two functional groups are contained in the above-mentioned amino acid or peptide residue represented by R, each protecting group Z bonded to a functional group may be the same or different type. Examples of functional groups of amino acid residue or peptide residue include amino, carboxyl, guanidyl, midazold, thiol, etc.

When n2 is an integer of at least 2, i.e., when repeating unit B repeats at least twice, each RZ in repeating unit B may be the same or different.

The compound represented by formula (1a) has all functional groups protected by protecting group X or Z, and has a free carboxyl group at the repeating unit C terminal. Consequently, condensation reaction between a target compound containing an amino group or hydroxyl group and the compound represented by formula (1a) readily occurs, and forms a conjugated compound in which the compound represented by formula (1a) and the target compound are bonded. Therefore, the use of the compound represented by formula (1a) as a starting material enables repeating units A, B and C to easily bond to a target compound. Alternatively, when protecting groups of the compound represented by formula (1a) substituted by hydrogen atoms, the compound represented by formula (1) can also be produced.

Target compounds for the bonding to the compound represented by formula (1a) are not limited as long as they contain an amino group or hydroxyl group, but specific examples can include PNA (peptide nucleic acid) oligomers, PNA monomers, peptides, lipids, fatty acids, etc.

The compound represented by formula (1a), for example, undergoes a condensation reaction with PNA monomers or PNA oligomers as shown by formulae (15) to (17) and formulae (18) and (19) below, thereby efficiently enabling the production of PNA monomers or PNA oligomers with repeating units A, B and C added thereto. 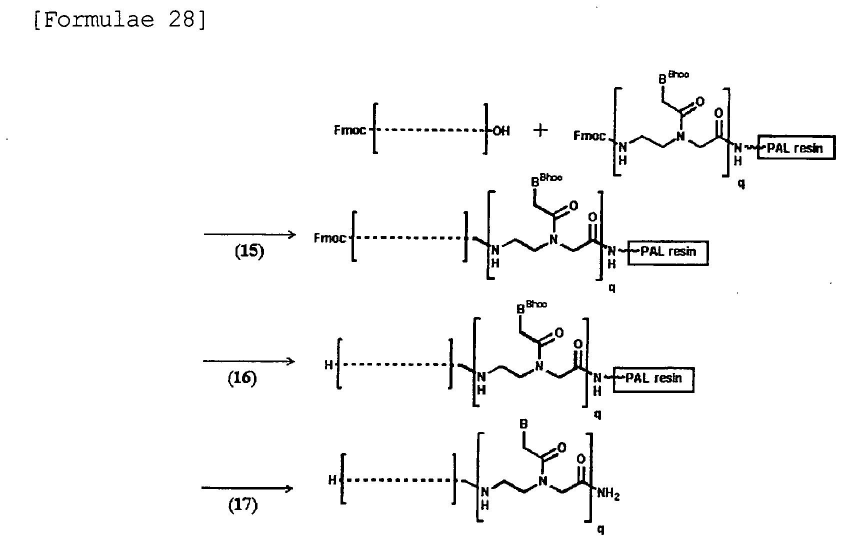 wherein Fmoc-[···]-OH represents a compound in which protecting group X in formula (1a) is Fmoc; B are nucleobases such as adenine, guanine, cytosine, thymine, etc.; Bhoc are protecting groups other than the Fmoc groups; and q is an integer of at least 1, and preferably an integer from 1 to 50. 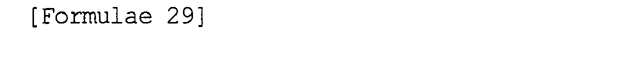 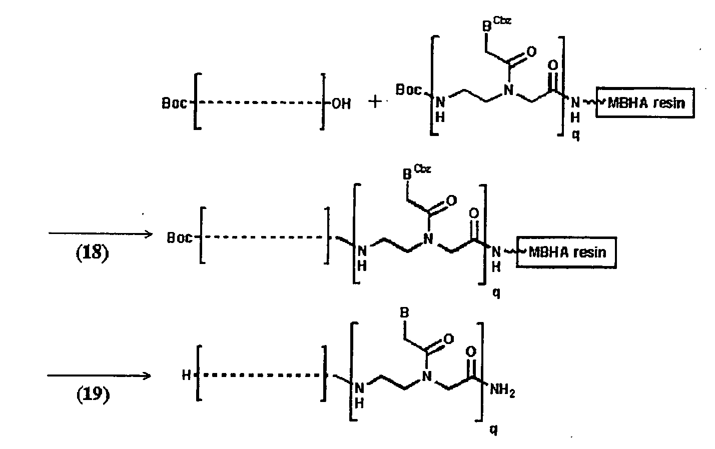 wherein Boc-[ ... ]-OH is a compound in which protecting group X in formula (1a) is Boc; B are nucleobases such as adenine, quanine, cytosine, thymine, etc.; Cbz is a protecting group other than Boc; and Q is an integer of at least 1, and preferably an integer from 1 to 50.

### 3. Process for producing a compound represented by formula (1)

The first process for producing a compound represented by formula (1) is a stepwise procedure comprising the following steps 1-1 to 1-5. Each of steps 1-1 to 1-5 is described in detail.

### Step 1-1

In step 1-1, a compound represented by formula (i) is synthesized using a compound represented by the following formula (I) and a solid-phase resin or a solid-phase compound. 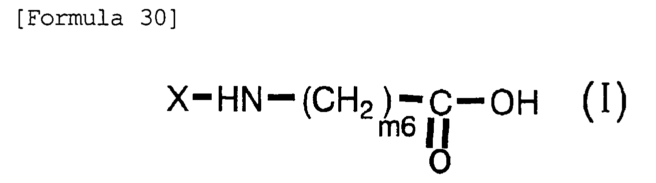 wherein m6 is as defined above. 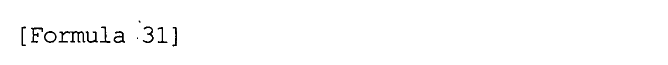 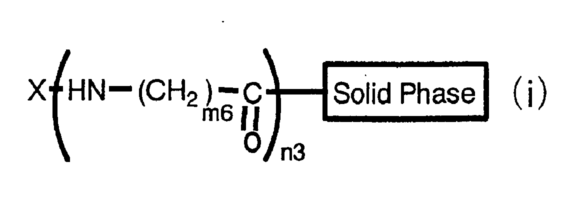 wherein n3, m6 and X are as defined above. Solid Phase is a solid-phase resin or a solid-phase compound.

The compounds represented by formula (I) are known compounds or those produced in accordance with a known production process.

The solid-phase resin or solid-phase compound used in this step is not limited, but examples include MBHA (methylbenzhydrylamine resin), PAL (peptide amide linker), oxime (P-nitrobenzophenone oxime), PAM (4-hydroxymethylphenylacetoamidemethyl resin), Merrifield resins, etc.

The compound represented by formula (I) above is condensed with a solid-phase resin or a solid-phase compound.

The condensation reaction between the compound represented by formula (I) and the solid-phase resin or solid-phase compound is typically carried out by admixing 0.01 to 100 mol, and preferably 0.1 to 10 mol of the solid-phase resin or solid-phase compound, per mol of the compound represented by formula (I).

The condensation reaction is typically carried out between the compound represented by formula (I) and the solid-phase resin or solid-phase compound in a solvent. Usable solvents can be selected from a wide variety of known solvents as long as they do not inhibit the reaction. Examples of such solvents include dimethylformamide (DMF), N-methylpyrrolidone (NMP), etc.

The condensation reaction between the compound represented by formula (I) and the solid-phase resin or solid-phase compound is preferably carried out using a coupling reagent and a reaction accelerator. Examples of the coupling reagent include O-(azabenzotriazol-1-yl)-N,N,N',N'-tetramethyluronium hexafluoro phosphate (HATU), O-(benzotriazol-1-yl)-N,N,N', N'-tetramethyluronium hexafluorophosphate (HBTU), 1-ethyl-3-(3-dimethylaminopropyl)carbodiimide (EDCI), dicyclohexylcarbodiimide (DCC), etc. HATU is preferably used among these. Examples of the reaction accelerator include N,N-diisopropylethylamine (DIEA), triethylamine (TEA), etc. A preferable reaction accelerator is DIEA.

The amount of coupling reagents to be typically used is 0.01 to 100 mol in total, and preferably 0.1 to 10 mol in total, per mol of a solid-phase resin or a solid-phase compound.

The amount of the above-mentioned reaction accelerators to be typically used is 0.01 to 100 mol in total, and preferably 0.1 to 10 mol in total, per mol of a solid-phase resin or a solid-phase compound.

The condensation reaction is typically performed between the compound represented by formula (I) and the solid-phase resin or solid-phase compound at 5 to 80°C, and preferably 10 to 30°C, for 0.1 to 48 hours, and preferably 0.1 to 1 hour, if required, while stirring.

A compound with the structure below can be thus obtained by condensing a single compound represented by formula (I) with a solid-phase resin or a solid-phase compound. Such a compound condensed with a solid-phase resin or a solid-phase compound is hereinafter referred to as a "solid-phase bonded compound."

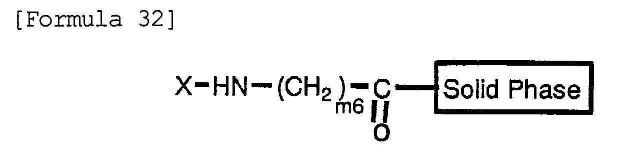

Protecting group X is eliminated from the thus obtained solid-phase bonded compound. The elimination of protecting group X from the solid-phase bonded compound is carried out by a suitably selected method according to the type of protecting group X. When protecting group X is a Boc group, an example of an elimination method is treatment in a TFA (trifluoroacetic acid) solution (95 vol.% TFA/5 vol.% m-cresol) at 10 to 30°C for 0.1 to 1 hour. When protecting group X is an Fmoc group, an example is treatment in a piperidine solution (20 vol.% piperidine/80 vol.% DMF) at 10 to 30°C for 0.01 to 0.5 hour. Further, when protecting group X is an Alloc group, an example is treatment in a Pd(PPh3)4 (tetrakis-triphenylphosphine-palladium complex, 312 mg) solution (55 vol.% chloroform/30 vol.% acetate/15 vol.% N-methylmorphorine) at 10 to 30°C for 0.1 to 1 hour.

A condensation reaction is then performed between the solid-phase bonded compound from which protecting group X was eliminated and the compound represented by formula (I). The conditions for the condensation reaction are the same as those employed in the above condensation reaction, except that the solid-phase bonded compound is used in place of the solid-phase resin or solid-phase compound.

The compound represented by formula (i) is obtained by an elimination reaction of protecting group X from the above-mentioned solid-phase bonded compound, and n3-1 times of the condensation reaction between the compound represented by formula (I) and the solid-phase bonded compound from which protecting group X was eliminated.

### Step 1-2

In step 1-2, a compound represented by the following formula (ii) is synthesized using the compound represented by formula (i) obtained in step 1-1 and a compound represented by the following formula (II). 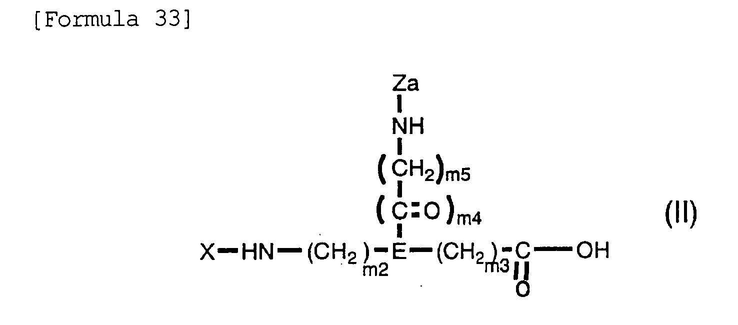 wherein m2 to m5, X and Za are as defined above. Further, the compounds represented by formula (II) are known compounds or those produced in accordance with a known production process. 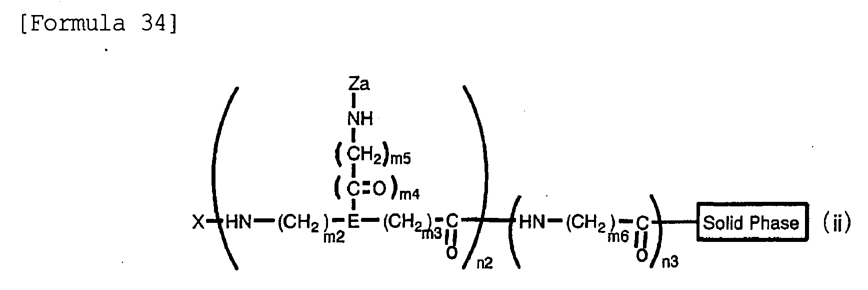 wherein n2, n3, m2 to m6, X, Za and Solid Phase are as defined above.

In the second step, protecting group X is first eliminated from the solid-phase bonded compound. To eliminate protecting group X, the same conditions as those in step 1-1 above can be employed.

A condensation reaction is then performed between the solid-phase bonded compound from which protecting group X was eliminated and the compound represented by formula (II).

The condensation reaction can be carried out between the solid-phase bonded compound from which protecting group X was eliminated and the compound represented by formula (II), under the same conditions for the condensation reaction in step 1-1 above. More specifically, the condensation reaction of step 1-2 can be performed under the same conditions for the condensation reaction in step 1-1 above, with the solid-phase bonded compound being used in place of the solid-phase resin or solid-phase compound, and further the compound represented by formula (II) being used in place of the compound represented by formula (I).

The compound represented by formula (ii) is obtained by the elimination reaction of protecting group X from the above-mentioned solid-phase bonded compound, and n2 times of the condensation reaction between the compound represented by formula (II) and the solid-phase bonded compound from which protecting group X was eliminated.

### Step 1-3

In step 1-3, a compound represented by the following formula (iii) is synthesized using the compound represented by formula (ii) obtained in step 1-2 and the compound represented by the following formula (III). 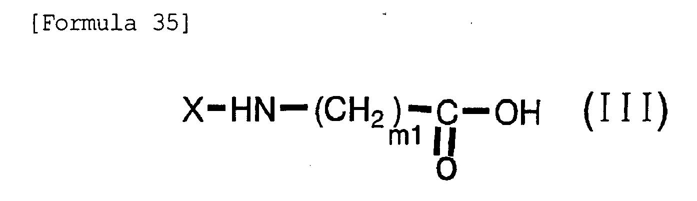 wherein m1 and X are as defined above. The compounds represented by formula (III) are known compounds or those produced in accordance with a known production process. 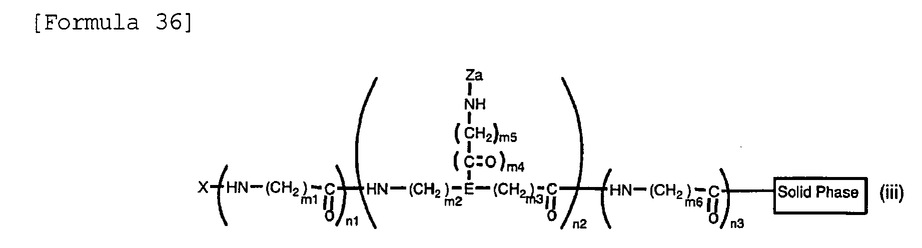 wherein n1 to n3, m1 to m6, X, Za and Solid Phase are as defined above.

In step 1-3, an elimination reaction of protecting group X from the solid-phase bonded compound is first carried out. To eliminate protecting group X, the same conditions as those in step 1-1 above can be employed.

A condensation reaction is then performed between the solid-phase bonded compound from which protecting group X was eliminated and the compound represented by formula (III).

The condensation reaction can be performed between the solid-phase bonded compound from which protecting group X was eliminated and the compound represented by formula (III), under the same conditions for the condensation reaction in step 1-1 above. More specifically, the condensation reaction of step 1-3 can be performed under the same conditions for the condensation reaction in step 1-1 above, with the solid-phase bonded compound being used in place of the solid-phase resin or solid-phase compound, and further the compound represented by formula (III) being used in place of the compound represented by formula (I).

The compound represented by formula (iii) can be obtained by the elimination reaction of protecting group X from the above-mentioned solid-phase bonded compound, and n3 times of the condensation reaction between the compound represented by formula (III) and the solid-phase bonded compound from which protecting group X was eliminated.

### Step 1-4

In step 1-4, a compound represented by the following formula (iv) is synthesized using the compound represented by formula (iii) obtained in step 1-3 and one or more amino acids wherein protecting group Za is bonded to a functional group other than a carboxyl group bonded to an α carbon atom. 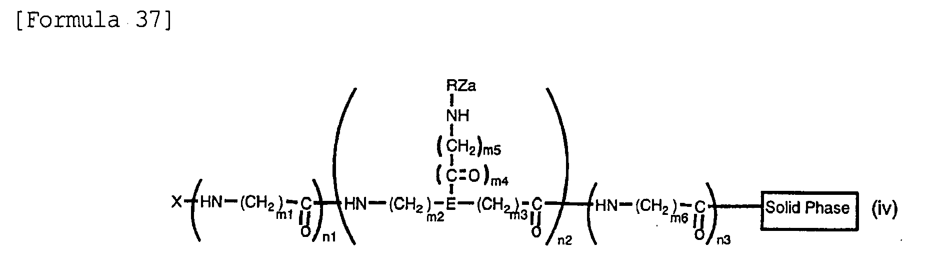 wherein n1 to n3, m1 to m6, X, RZa and Solid Phase are as defined above.

Further, the above-mentioned amino acid used in this step can be one wherein protecting group Za is bonded to a functional group other than a carboxyl group bonded to an α carbon atom.

For example, when an amino acid has no functional group other than a carboxyl group and amino group bonded to an α carbon atom, an amino acid in which protecting group Za is bonded to an amino group bonded to an α carbon atom can be used.

Further, when an amino acid in which functional groups are bonded other than an α carbon atom, such as lysine, arginine, serine, etc., amino acids in which protecting groups Za are bonded to all functional groups other than a carboxyl group bonded to an α carbon atom can be used. In such a case, protecting groups Za for an amino group bonded to an α carbon atom are preferably different in kind from protecting groups Za for functional groups bonded to parts other than α carbon atoms. Thus, when there are at least 2 protecting groups Zb in the above amino acid, the use of different kinds of protecting groups enables the selective elimination of only protecting groups Za bonded to an amino group used in the condensation reaction, leaving protecting groups Za bonded to other functional groups intact.

In step 1-4, an elimination reaction of protecting group Za bonded to an amino group used in the condensation reaction with the solid-phase bonded compound is first performed. To eliminate protecting group Za, the same conditions as those in step 1-1 above can be employed.

A condensation reaction is carried out between the above amino acid and the solid-phase bonded compound from which protecting group Za was eliminated in the above-mentioned manner.

The condensation reaction can be performed between the solid-phase bonded compound from which protecting group Za was eliminated and the above amino acid, under the same conditions as those for the condensation reaction in step 1-1 above. More specifically, the condensation reaction of step 1-4 can be performed under the same conditions as those for the condensation reaction in step 1-1 above, with the solid-phase bonded compound being used in place of the solid-phase resin or solid-phase compound, and further the above amino acid being used in place of the compound represented by formula (I).

The compound represented by formula (iv) can be obtained by the elimination reaction of protecting group Za from the above-mentioned solid-phase bonded compound, and 1 to 100 times of the condensation reaction between the above amino acid and the solid-phase bonded compound from which protecting group Za was eliminated. For example, the elimination of the above protecting group Za and a single performance of the condensation reaction synthesize a compound wherein R is an amino acid residue. Further, the elimination of the above protecting group Za and three times of the condensation reaction synthesize a compound wherein R is a peptide residue consisting of 3 amino acid residues.

### Step 1-5

The solid-phase resin or solid-phase compound is then removed from the compound represented by formula (iv) obtained in step 1-4 to form terminal -COOH or -CONH₂ group. Also, the protecting group X and Za are substituted by hydrogen atoms. Further, if required, a group containing a lipid group, fatty acid residue, or fluorescent group is caused to bond to a terminal amino group in repeating unit A of the compound represented by formula (1). The compound represented by formula (1) is thus synthesized.

When, for example, Merrifield resin is used as a solid-phase resin or a solid-phase compound, the compound represented by formula (1) wherein Y is a carboxyl group can be obtained by exposing the compound represented by formula (iv) under highly acidic conditions. When, for example, MBHA resin is used as a solid-phase resin or solid-phase compound, the compound represented by formula (1) wherein Y is an amino group can be obtained by exposing the compound represented by formula (iv) under highly acidic conditions.

Further, the second process for producing the compound represented by formula (1) is a stepwise procedure comprising the following steps 2-1 to 2-5. Each of steps 2-1 to 2-5 is described in detail.

### Step 2-1

In step 2-1, a compound represented by formula (i) is synthesized using the compound represented by the above formula (I) and a solid-phase resin or a solid-phase compound.

The compound represented by the above formula (I) is first condensed with a solid-phase resin or a solid-phase compound to obtain a solid-phase bonded compound, from which protecting group X is then eliminated. The elimination of protecting group X is carried out under the same conditions as in step 1-1 above.

Subsequently, a condensation reaction is performed n3-1 times between the solid-phase bonded compound from which protecting group X was eliminated and the compound represented by formula (I). The condensation reaction is carried out under the same conditions as in step 1-1 above. The compound represented by formula (i) is thus obtained.

### Step 2-2

In step 2-2, a compound represented by the following formula (II') is synthesized using a compound represented by the above formula (II) and an amino acid in which protecting group Za is bonded to a functional group other than a carboxyl group bonded to an α carbon atom. 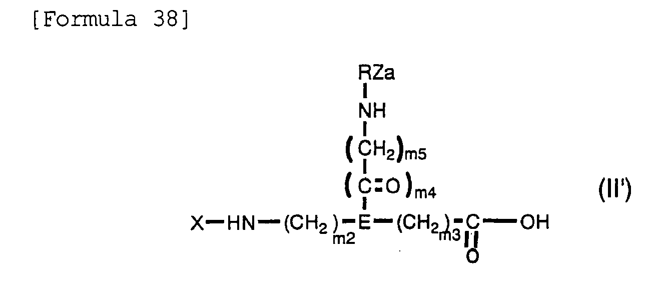 wherein m2 to m5, X and RZa are as defined above.

In step 2-2, the carboxyl group of the compound represented by formula (II) is preferably protected by a protecting group different from protecting groups X and Za.

Protecting group Za of the compound represented by formula (II) is first eliminated. The elimination of protecting group Za is carried out under the same conditions as in step 1-4 above.

A condensation reaction is then performed between the above amino acid and the compound represented by formula (II) from which protecting group Za was eliminated The condensation reaction is carried out under the same conditions as in step 1-4 above.

As described above, the compound represented by formula (II') can be obtained by eliminating protecting group Za bonded to an amino group used in the condensation reaction, and repeating 1 to 100 times the condensation reactions between the above amino acid and the compound from which protecting group Za bonded to the amino group used in the condensation reaction was eliminated.

### Step 2-3

In step 2-3, a compound represented by the following formula (ii') is synthesized using the compound represented by formula (i) obtained in step 2-1 and the compound represented by formula (II') obtained in step 2-2. 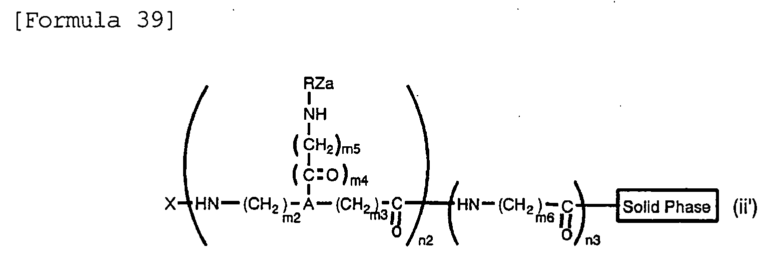 wherein n2, n3, m2 to m6, RZa and Solid Phase are as defined above.

Protecting group X is first eliminated from the solid-phase bonded compound. The elimination of protecting group X can employ the same conditions as in step 1-2 above.

A condensation reaction is then performed between the solid-phase bonded compound from which protecting group X was eliminated and the compound represented by formula (II'). The condensation reaction can be performed under the same conditions as in step 1-2 above.

The compound represented by formula (ii') can be obtained by eliminating protecting group X from the above solid-phase bonded compound, and by repeating a total of n2 times the condensation reaction between the compound represented by formula (II') and the solid-phase bonded compound from which protecting group X was eliminated.

### Step 2-4

In step 2-4, a compound represented by formula (iv) is synthesized using the compound represented by formula (ii') obtained in step 2-3 and a compound represented by formula (III).

Protecting group X is first eliminated from the solid-phase bonded compound. The elimination of protecting group X can employ the same conditions as in step 1-3 above.

A condensation reaction is then performed between the solid-phase bonded compound from which protecting group X was eliminated and the compound represented by formula (III). The condensation reaction can be performed under the same conditions as in step 1-3 above.

The compound represented by formula (iv) can be obtained by eliminating protecting group X from the above solid-phase bonded compound, and by repeating a total of n1 times the condensation reaction between the compound represented by formula (III) and the solid-phase bonded compound from which protecting group X was eliminated.

### Step 2-5

Consequently, the solid-phase resin or solid-phase compound is removed from the compound represented by formula (iv) obtained in step 2-4 to form terminal -COOH or -CONH₂ groups. Also, the protecting groups X and Za are removed. Further, if required, a group containing a lipid group, fatty acid residue, or fluorescent group is caused to bond to a terminal amino group in repeating unit A of the compound represented by formula (1). The compound represented by formula (1) is thus synthesized. Step 2-5 is performed in the same manner as in step 1-5 above.

Furthermore, the third process for producing the compound represented by formula (1) is a process by which protecting groups X and Z are eliminated from the compound represented by formula (1a). The elimination reaction of protecting groups X and Z can be performed under suitable conditions determined according to the kind of protecting groups.

### 4. Process for producing a compound represented by formula (1a)

The first process for producing a compound represented by formula (1a) is a stepwise procedure comprising the following steps 3-1 to 3-5. Each of steps 3-1 to 3-5 is described in detail.

### Step 3-1

In step 3-1, a compound represented by formula (i) is synthesized using a compound represented by the above formula (I) and a solid-phase resin or a solid-phase compound.

The solid-phase resin or solid-phase compound used for the synthesis of the compound represented by formula (1a) is suitably selected according to the kind of protecting groups X and Z contained in the compound represented by formula (1a). For example, when at least one of the protecting groups X and Z contained in the compound represented by formula (1a) is a Boc group, oxime resin is desirably used as a solid-phase resin or oxylbenzyl is desirably used as a solid-phase compound. When at least one of the protecting groups X and Z contained in the compound represented by formula (1a) is an Fmoc group, PAM resin is desirably used as a solid-phase resin.

The compound represented by formula (I) is first condensed with a solid-phase resin or solid-phase compound to obtain a solid-phase bonded compound, and the elimination reaction of protecting group X from the obtained solid-phase bonded compound is then performed. The elimination of protecting group X is carried out under the same conditions as in step 1-1 above.

Subsequently, a condensation reaction is performed n3-1 times between the solid-phase bonded compound from which protecting group X was eliminated and the compound represented by formula (I). The condensation reaction is carried out under the same conditions as in step 1-1 above. The compound represented by formula (i) is thus obtained.

### Step 3-2

In step 3-2, a compound represented by formula (ii) is synthesized using the compound represented by formula (i) obtained in step 3-1, and a compound represented by formula (II).

Protecting group X is first removed from the solid-phase bonded compound. The elimination of protecting group X can be performed under the same conditions as in step 1-2 above.

A condensation reaction is then carried out between the solid-phase bonded compound from which protecting group X was eliminated and the compound represented by formula (II). The condensation reaction can be performed under the same conditions as in step 1-2 above.

The compound represented by formula (ii) is obtained by eliminating protecting group X from the above solid-phase bonded compound, and repeating a total of n2 times the condensation reaction between the solid-phase bonded compound from which protecting group X was eliminated and the compound represented by formula (II).

### Step 3-3

In step 3-3, a compound represented by formula (iii) is synthesized using the compound represented by formula (ii) obtained in step 3-2, and a compound represented by formula (III).

Protecting group X is first eliminated from the solid-phase bonded compound. The elimination method of protecting group X can employ the same conditions as in step 1-3 above.

A condensation reaction is then performed between the solid-phase bonded compound from which protecting group X was eliminated and the compound represented by formula (III). The condensation reaction can be carried out under the same conditions as in step 1-3 above.

The compound represented by formula (iii) is obtained by eliminating protecting group X from the above solid-phase bonded compound, and repeating a total of n1 times the condensation reaction between the solid-phase bonded compound from which protecting group X was eliminated and the compound represented by formula (III).

### Step 3-4

In step 3-4, a compound represented by formula (iv) is synthesized using the compound represented by formula (iii) obtained in step 3-3, and an amino acid in which protecting group Za is bonded to a functional group other than a carboxyl group bonded to an α carbon atom.

The amino acid used in step 3-4 is the same amino acid used in step 1-4 above.

Protecting group Za is first eliminated from the compound represented by formula (iii). The elimination of protecting group Za can be performed under the same conditions as in step 1-4 above.

A condensation reaction is then carried out between the above amino acid and the compound represented by formula (iii) from which protecting group Za was eliminated. The condensation reaction can be performed under the same conditions as in step 1-4 above.

As described above, the compound represented by formula (iv) is obtained by eliminating protecting group Za bonded to the amino group used in the condensation reaction, and repeating a total of 1 to 100 times the condensation reaction between the above amino acid and the compound from which protecting group Za bonded to the amino group used in the condensation reaction was eliminated.

### Step 3-5

Protecting group Za is bonded to the compound represented by formula (iv) obtained in the above step 3-4. Therefore, if required, protecting group Za is replaced with a protecting group identical to protecting group X (protecting group Zb) in accordance with a known method.

The solid-phase resin or solid-phase compound is then removed from the compound represented by formula (iv) obtained in step 3-4 to form a terminal -COOH, without removing protecting groups X and Za.

For example, when oxime resin is used as a solid-phase resin or a solid-phase compound, the compound represented by formula (1a) can be obtained by exposing the compound represented by formula (iv) to alkaline conditions.

Further, the second process for producing the compound represented by formula (1a) is a procedure comprising the following steps 4-1 to 4-5. Each of steps 4-1 to 4-5 is described in detail below.

### Step 4-1

In step 4-1, a compound represented by formula (i) is synthesized using a compound represented by the above formula (I) and a solid phase-resin or a solid-phase compound. Step 4-1 can be performed under the same conditions as in step 3-1 above.

### Step 4-2

In step 4-2, a compound represented by formula (II') is synthesized using a compound represented by the above formula (II) and an amino acid in which protecting group Za is bonded to a functional group other than a carboxyl group bonded to an α carbon atom.

The amino acid used in step 1-2 is the same amino acid used in step 3-4 above.

Protecting group Za is first eliminated from the compound represented by formula (II). The elimination of protecting group Za can be performed under the same conditions as in step 3-4 above.

A condensation reaction is then performed between the above amino acid and the compound represented by formula (II) from which protecting group Za was eliminated. The condensation reaction is carried out under the same conditions as in step 3-4 above.

As described above, the compound represented by formula (II') is obtained by eliminating protecting group Za bonded to the amino group used in the condensation reaction, and repeating a total of 1 to 100 times the condensation reaction between the above amino acid and the compound from which protecting group Za bonded to the amino group used in the condensation reaction was eliminated.

### Step 4-3

In step 4-3, a compound represented by formula (ii') is synthesized using the compound represented by formula (i) obtained in step 4-1 and the compound represented by formula (II') obtained in step 4-2.

Protecting group X is first eliminated from the solid-phase bonded compound. The elimination method of protecting group X can employ the same conditions as in step 3-2 above.

A condensation reaction is then carried out between the solid-phase bonded compound from which protecting group X was eliminated, and the compound represented by formula (II'). The condensation reaction can be performed under the same conditions as in step 3-2 above.

The compound represented by formula (ii') is obtained by eliminating protecting group X from the above solid-phase bonded compound, and repeating a total of n2 times the condensation reaction between the solid-phase bonded compound from which protecting group X was eliminated and the compound represented by formula (II').

### Step 4-4

In step 4-4, a compound represented by formula (iv) is synthesized using the compound represented by formula (ii') obtained in step 4-3 and a compound represented by formula (III).

Protecting group X is first eliminated from the solid-phase bonded compound. The elimination method of protecting group X can employ the same conditions as in step 3-3 above.

A condensation reaction is then performed between the solid-phase bonded compound from which protecting group X was eliminated and the compound represented by formula (III). The condensation reaction can be carried out under the same conditions in step 3-3 above.

The compound represented by formula (iv) is obtained by eliminating protecting group X from the above solid-phase bonded compound, and repeating a total of n1 times the condensation reaction between the solid-phase bonded compound from which protecting group X was eliminated and the compound represented by formula (III).

### Step 4-5

The protecting group Za is bonded to the compound represented by formula (iv) obtained in step 4-4 above. Therefore, if required, protecting group Za is replaced with a protecting group identical to protecting group X (protecting group Zb) in accordance with a known method.

Next, the solid-phase resin or solid-phase compound is removed from the compound represented by formula (iv) obtained in step 4-4 to form a terminal -COOH without removing protecting groups X and Zb. Step 4-5 is performed in the same manner as in step 3-5 above.

### EXAMPLES

The present invention will be described in more detail hereinafter with reference to the Examples and Test Examples. However, the present invention is not limited to these Examples and Test Examples.

### Reference synthesis example 1:

### Synthesis of compound represented by general formula (11)

The compound represented by the following general formula (11) (hereafter referred to as compound (11)) was synthesized.

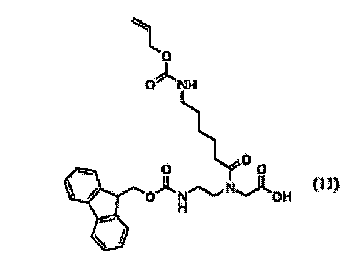

Specifically, DCC (1,3-dicyclohexylcarbodiimide) (845 mg, 4.5 mmol) was added with water-cooling to a methylene chloride solution (12 ml) of Alloc(allyloxycarbonyl)-HN-C₅H₁₀-COOH (891 mg, 3.0 mmol) and Pfp-OH (pentafluorophenol) (754 mg, 4.5 mmol), and this reaction mixture was stirred at 0°C for 30 minutes, and then at room temperature for 15 hours. The reaction mixture was filtered, and the filtrate was concentrated under reduced pressure. The residue was purified by silica gel column chromatography (CH₂Cl₂) and then recrystallized from hexane to give white powdery Alloc-HN-C₅H₁₀-COO-Pfp (537.5 mg, 98%). ¹H-NMR (CDCl₃) δ 5.92 (m, 1 H), 5.26 (m, 2 H), 4.96 (br t, 1 H), 4.57 (br d, 2 H), 3.22 (q, J = 6.2 Hz, 2 H), 2.69 (t, J = 7.2 Hz, 2 H), 2.0 - 1.8 (m, 2 H), 1.75-1.1 (m, 8 H); LRMS (FAB⁺) calcd for C₁₆H₁₇F₅NO₄ [(M+H)⁺] 382.3 observed 382.

Subsequently, NaHCO₃ (67.2 mg, 0.8 mmol) was added to a mixed solution of acetone (6.0 ml) and water (1.0 ml). Alloc-HN-C₅H₁₀-O-Pfp (763 mg, 2.0 mmol) and the compound represented by the following general formula (12) (681 mg, 2.0 mmol) were dissolved therein, and stirring was performed at room temperature for 6 hours.

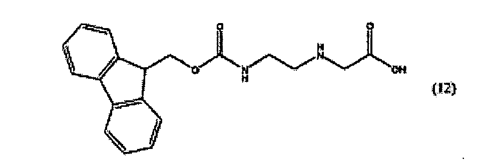

The pH of the cooled reaction solution was adjusted to 3.0 with water-cooled 1 N hydrochloric acid, and an aqueous 1% citric acid solution was further added thereto. Extraction was then performed with ethyl acetate, and the organic layer was washed with saturated brine. The organic layer was dried over anhydrous magnesium sulfate, concentrated, and then purified by silica gel column chromatography (1 to 5% MeOH/CH₂Cl₂) . Subsequently, the resulting product was dissolved in methylene chloride and then concentrated under reduced pressure to give amorphous powdery compound (11) (157.3 mg, 80%). ¹H-NMR (CDCl₃) δ 7.75 (d, J = 6.8 Hz, 2 H), 7.59 (d, J = 7.6 Hz, 2 H), 7.35 (m, 4 H), 5.8 (m, 1 H), 5.8 (ma) and 5.6 (mi) (m, 1 H), 5.2 (m, 2 H), 4.6 (mi) and 4.53 (ma) (br d, 2 H), 4.37 (br d, 2 H), 4.22 (mi) and 4.04 (ma) (br d, 2 H), 3.50 (m, 2 H), 3.33 (m, 2 H), 3.16 (m, 2 H), 2.37 (ma) and 2.2 (mi) (br t, 2 H), 1.8 - 1.2 (m, 6 H); LRMS (FAB⁺) calcd for C₂₉H₃₆N₃O₇ [(M+H)⁺] 538.6 observed 538.

### Example 1:

### Synthesis of compound represented by general formula (1-A)

The compound represented by the following general formula (1-A) (hereafter referred to as compound (1-A)) was synthesized.

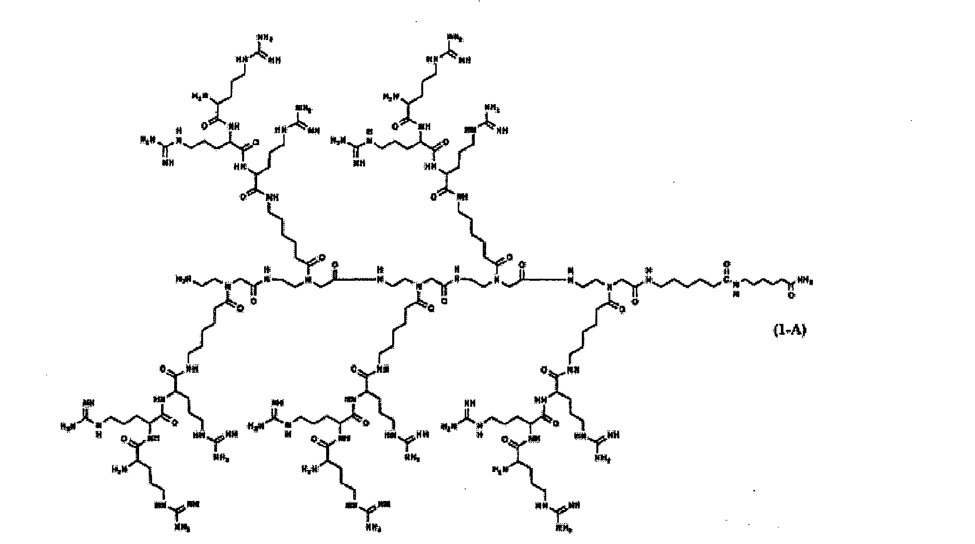

According to the standard tBoc method (cf. Koch, T.; Hansen, H.F.; Andersen, P.; Larsen, T.; Batz, H.G.; Otteson, K.; Orum, H. J. Peptide Res. 1997, 49, 80-88.), first, a condensation reaction was carried out at room temperature for 30 minutes by applying Boc-HN-C₅H₁₀-COOH (t-butoxycarbonyl-6-aminocaproic acid; 34.3 mg, 109.8 µmol), a condensing agent HATU (2-(1H-9-azabenzotriazol-1-yl)-1,1,3,3-tetramethyluronium hexafluorophosphate) (41.7 mg, 109.8 µmol) and DIEA (diisopropylethylamine) (50 µL) to a solid-phase carrier MBHA (4-methyl-benzhydrylamine) resin (120 mg, 73.2 µmol) (reaction step r1-1). After the Boc group was depotected by TFA treatment (95% TFA/5% m-cresol), stepwise extension reactions were carried out at room temperature for 30 minutes using Boc-HN-C₅H₁₀-COOH (34.3 mg, 109.8 µmol), a condensing agent HATU (41.7 mg, 109.8 µmol) and DIEA (50 µL) (reaction steps r1-2 and r1-3).

Subsequently, after the Boc group was deprotected by TFA treatment (95% TFA/5% m-cresol) (reaction step r1-4), the compound (58.6 mg, 109.8 µmol) represented by the following formula (13) was condensed at room temperature for 30 minutes using a condensing agent HATU (41.7 mg, 109.8 µmol) and DIEA (50 µL). This operation was performed five times in total (reaction steps r1-5 and 1-6).

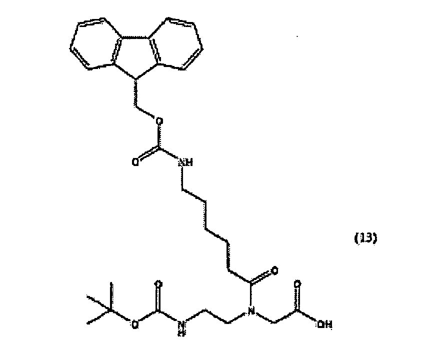

Next, the Fmoc groups were deprotected by pyperidine treatment (20% piperidine in DMF) at room temperature for 5 minutes (reaction step r1-7). Fmoc-Arg (Mts:N-mesitylene-2-sulfonyl)-OH·IPE(isopropylethylamine) (747 mg, 1098 µmol) was then condensed at room temperature for 30 minutes using a condensing agent HATU (417 mg, 1098 µmol) and DIEA (192 µL). This operation was performed three times in total (reaction steps r1-8 to r1-10).

Finally, after the Fmoc groups were deprotected by pyperidine treatment, excision from the solid-phase carrier MBHA and deprotection of the Arg-protecting Mts groups were performed by a standard method (TFA/TFMSA/p-cresol/thioanisole = 60/25/10/10) to give the target (compound (1-A)) (reaction step rl-11). MALDI-TOF MS: calcd. 3653.53 (M+H⁺), found 3654.57.

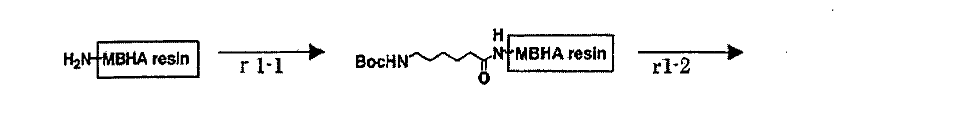

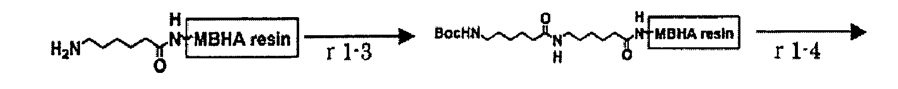

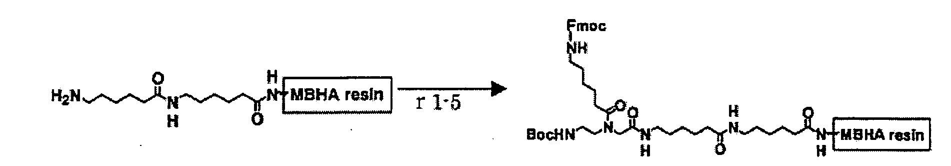

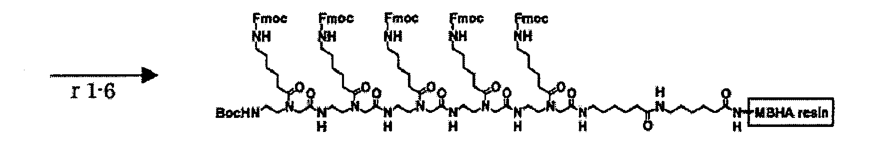

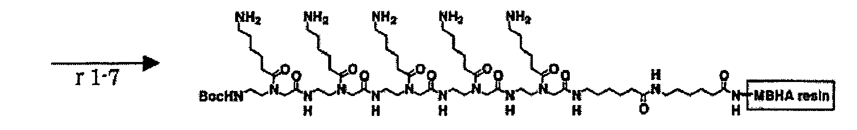

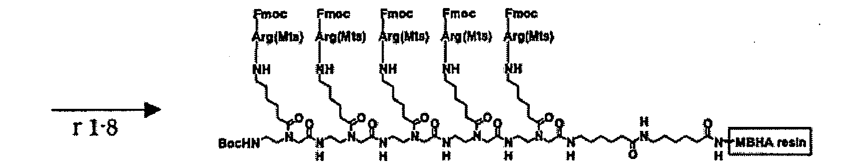

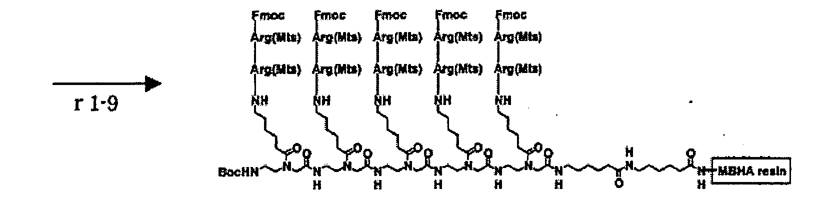

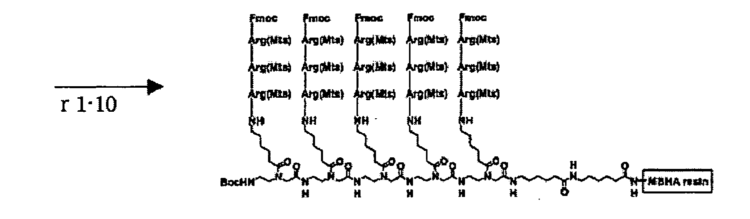

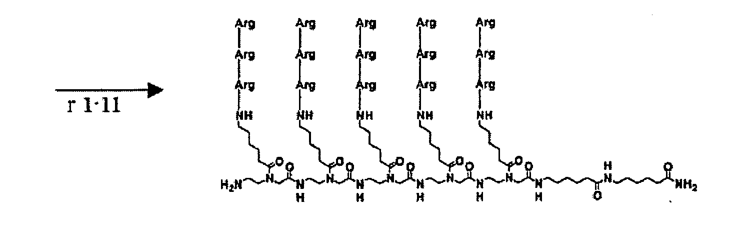

### Example 2:

### Synthesis of compound represented by general formula (1-B)

The compound represented by the following general formula (1-B) (hereafter referred to as compound (1-B)) was synthesized.

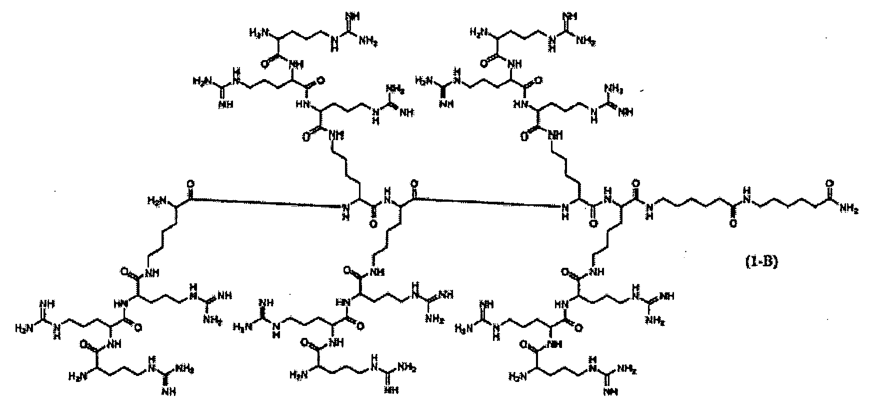

Specifically, according to the standard tBoc method (cf. Koch, T.; Hansen, H.F.; Andersen, P.; Larsen, T.; Batz, H.G.; Otteson, K.; Orum, H. J. Peptide Res. 1997, 49, 80-88.), first, a condensation reaction was carried out at room temperature for 30 minutes by applying a linker ω-amino acid Boc-HN-C₅H₁₀-COOH (34.3 mg, 109.8 µmol), a condensing agent HATU (41.7 mg, 109.8 µmol) and DIEA (50 µL) to a solid-phase carrier MBHA (120 mg, 73.2 µmol) (reaction step r2-1). After the Boc group was depotected by TFA treatment (95% TFA/5% m-cresol), stepwise extension reactions were carried out at room temperature for 30 minutes using Boc-HN-C₅H₁₀-COOH (34.3 mg, 109.8 µmol), a condensing agent HATU (41.7 mg, 109.8 µmol) and DIEA (50 µL) (reaction steps r2-2 and r2-3).

Subsequently, after the Boc group was depotected by TFA treatment (95% TFA/5% m-cresol) (reaction step r2-4), Boc-Lys(Fmoc)-OH (53.4 mg, 109.8 µmol) was condensed at room temperature for 30 minutes using a condensing agent HATU (41.7 mg, 109.8 µmol) and DIEA (50 µL) . This operation was performed five times in total (reaction steps r2-5 and r2-6).

Next, the Fmoc groups were deprotected by pyperidine treatment (20% piperidine in DMF) at room temperature for 5 minutes (reaction step r2-7). Fmoc-Arg(Mts)-OH·IPE (747 mg, 1098 µmol) was then condensed at room temperature for 30 minutes using a condensing agent HATU (417 mg, 1098 µmol) and DIEA (192 µL). This operation was performed three times in total (reaction steps r2-8 to r2-10).

Finally, after the Fmoc groups were deprotected by pyperidine treatment, excision from the solid-phase carrier MBHA and deprotection of the Arg-protecting Mts groups was performed by a standard method (TFA/TFMSA/p-cresol/thioanisole = 60/25/10/10) to give the target (compound (1-B)) (reaction step r2-11). MALDI-TOF MS: calcd. 3228.00(M+H⁺), found 3227.91.

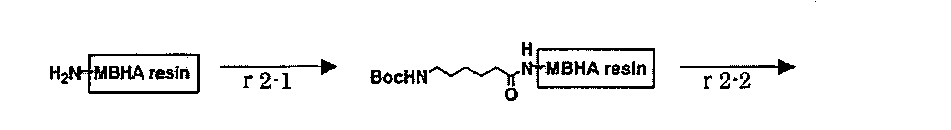

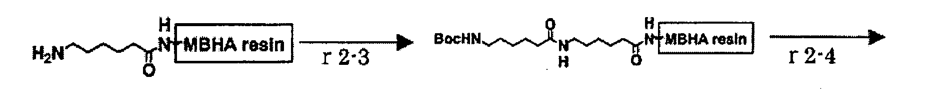

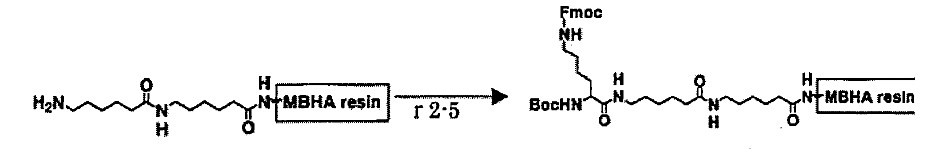

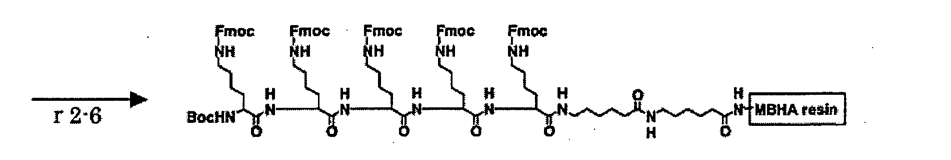

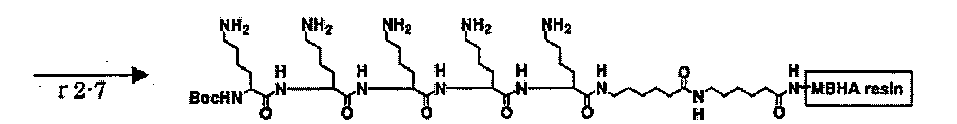

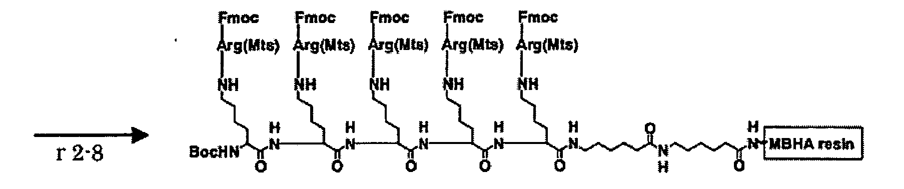

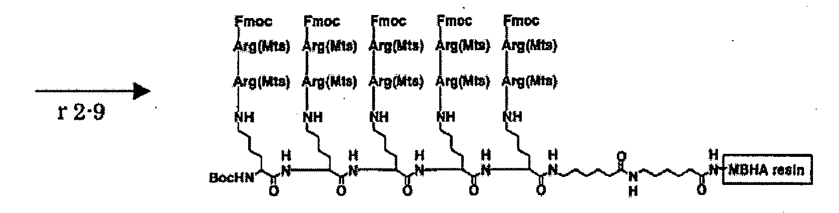

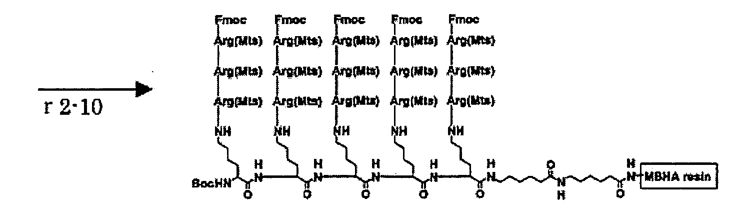

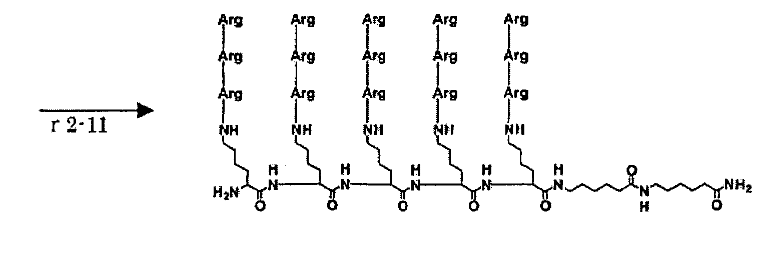

### Example 3:

### Synthesis of compound represented by general formula (1-C)

The compound represented by the following general formula (1-C) (hereafter referred to as compound (1-C)) was synthesized.

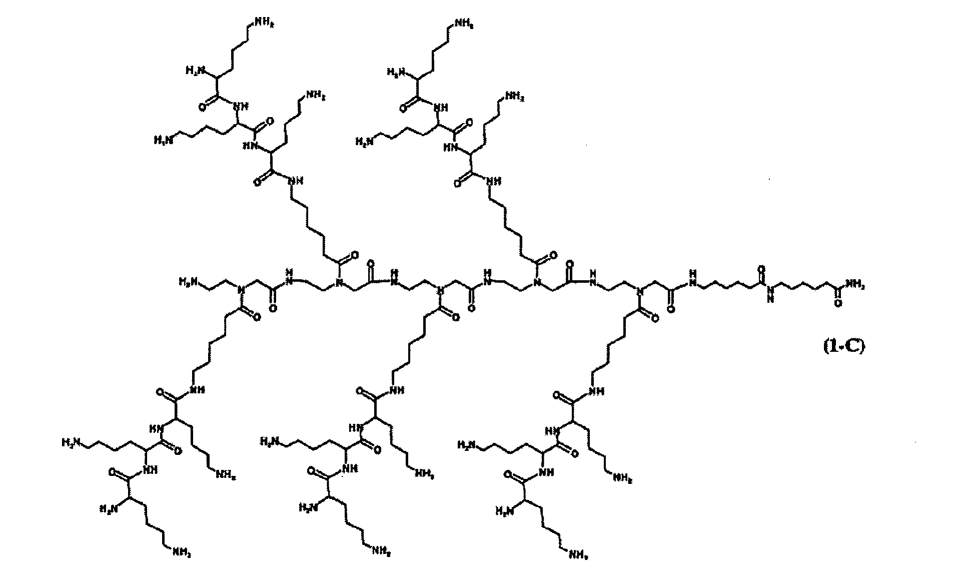

Specifically, according to the standard tBoc method (cf. Koch, T.; Hansen, H.F.; Andersen, P.; Larsen, T.; Batz, H.G.; Otteson, K.; Orum, H. J. Peptide Res. 1997, 49, 80-88.), first, a condensation reaction was carried out at room temperature for 30 minutes by applying a linker ω-amino acid Boc-HN-C₅H₁₀-COOH (34.3 mg, 109.8 µmol), a condensing agent HATU (41.7 mg, 109.8 µmol) and DIEA (50 µL) to a solid-phase carrier MBHA (120 mg, 73.2 µmol) (reaction step r3-1). After the Boc group was depotected by TFA treatment (95% TFA/5% m-cresol), stepwise extension reactions were carried out at room temperature for 30 minutes using Boc-HN-C₅H₁₀-COOH (34.3 mg, 109.8 µmol), a condensing agent HATU (41.7 mg, 109.8 µmol) and DIEA (50 µL) (reaction steps r3-2 and r3-3).

Subsequently, after the Boc group was depotected by TFA treatment (95% TFA/5% m-cresol) (reaction step r3-4), the compound (58.6 g, 109.8 µmol) represented by the above formula (13) was condensed at room temperature for 30 minutes using a condensing agent HATU (41.7 mg, 109.8 µmol) and DIEA (50 µL). This operation was performed five times in total (reaction steps r3-5 and r3-6).

Next, the Fmoc groups were deprotected by pyperidine treatment (20% piperidine in DMF) at room temperature for 5 minutes (reaction step r3-7). Fmoc-Lys(Boc)-OH (534 mg, 1098 µmol) was then condensed at room temperature for 30 minutes using a condensing agent HATU (417 mg, 1098 µmol) and DIEA (192 µL). This operation was performed three times in total (reaction steps r3-8 to r3-10).

Finally, after the Fmoc groups were deprotected by pyperidine treatment, excision from the solid-phase carrier MBHA and deprotection of the Lys-protecting Boc groups were performed by a standard method (TFA/TFMSA/p-cresol/thioanisol = 60/25/10/10) to give the target (compound (1-C)) (reaction step r3-11). MALDI-TOF MS: calcd. 3233.32 (M+H⁺), found 3233.60.

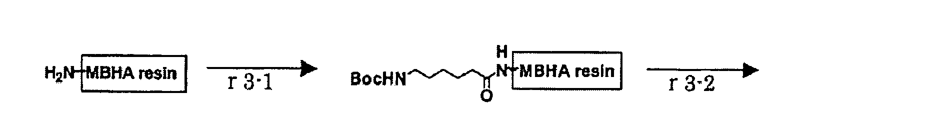

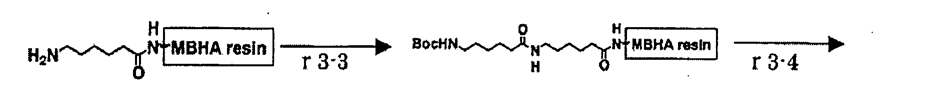

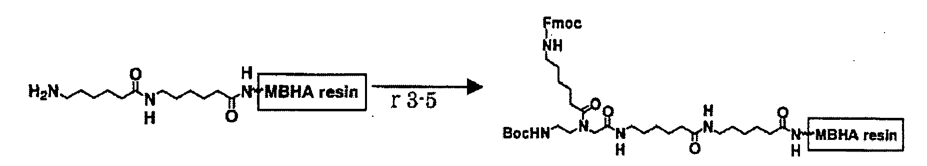

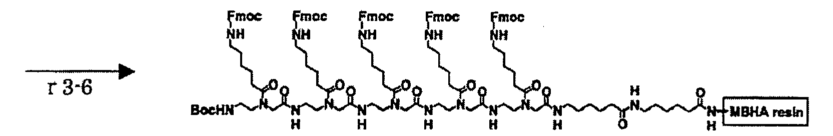

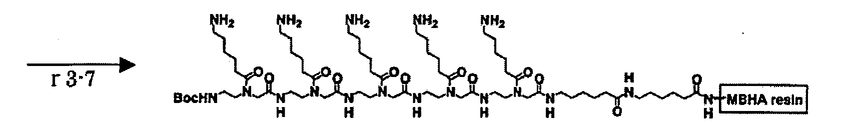

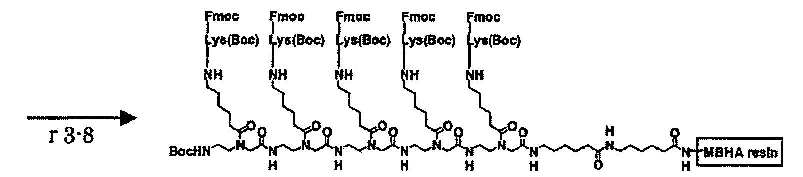

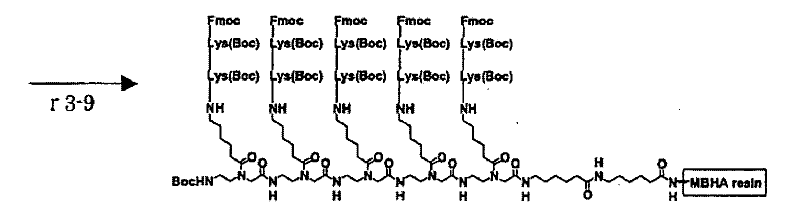

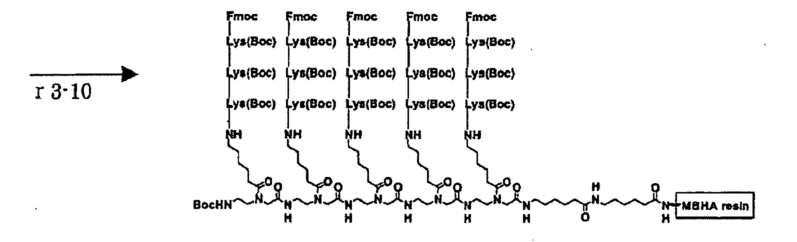

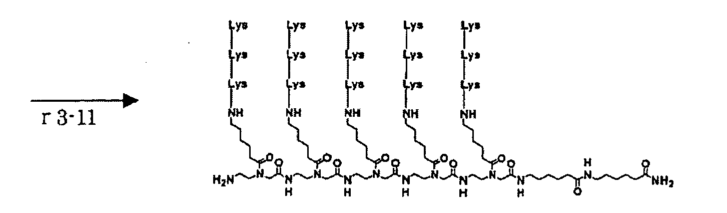

### Example 4:

### Synthesis of compound represented by general formula (1-D)

The compound represented by the following general formula (1-D) (hereafter referred to as compound (1-D)) was synthesized.

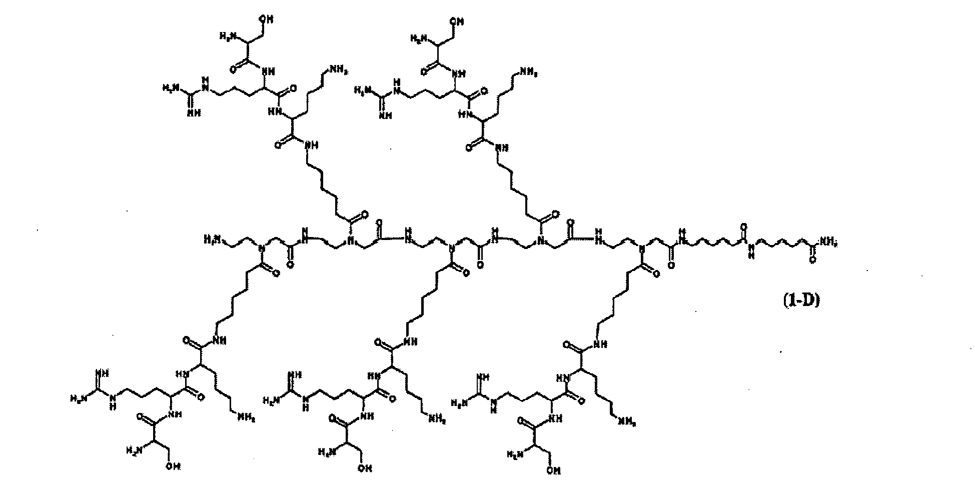

Specifically, according to the standard tBoc method (cf. Koch, T.; Hansen, H.F.; Andersen, P.; Larsen, T.; Batz, H.G.; Otteson, K.; Orum, H. J. Peptide Res. 1997, 49, 80-88.), first, a condensation reaction was carried out at room temperature for 30 minutes by applying a linker ω-amino acid Boc-HN-C₅H₁₀-COOH (34.3 mg, 109.8 µmol), a condensing agent HATU (41.7 mg, 109.8 µmol) and DIEA (50 µL) to a solid-phase carrier MBHA (120 mg, 73.2 µmol) (reaction step r4-1). After the Boc group was depotected by TFA treatment (95% TFA/5% m-cresol), stepwise extension reactions were carried out at room temperature for 30 minutes using Boc-HN-C₅H₁₀-COOH (34.3 mg, 109.8 mmol), a condensing agent HATU (41.7 mg, 109.8 mmol) and DIEA (50 µL) (reaction steps r4-2 and r4-3).

Subsequently, after the Boc group was depotected by TFA treatment (95% TFA/5% m-cresol) (reaction step r4-4), the compound (58.6 mg, 109.8 µmol) represented by the above formula (13) was condensed at room temperature for 30 minutes using a condensing agent HATU (41.7 mg, 109.8 µmol) and DIEA (50 µL). This operation was performed five times in total (reaction steps r4-5 and r4-6).

Next; the Fmoc groups were deprotected by pyperidine treatment (20% piperidine in DMF) at room temperature for 5 minutes (reaction step r4-7). Fmoc-Lys(Boc)-OH (534 mg, 1098 µmol) was then condensed at room temperature for 30 minutes using a condensing agent HATU (417 mg, 1098 µmol) and DIEA (192 µL) (reaction step r4-8). Subsequently, the Fmoc groups were deprotected by pyperidine treatment, and Fmoc-Arg(Mts)-OH·IPE (747 mg, 1098 µmol) was condensed at room temperature for 30 minutes using a condensing agent HATU (417 mg, 1098 µmol) and DIEA (192 µL) (reaction step r4-9). Further, the Fmoc groups were deprotected by pyperidine treatment, and Fmoc-Ser(Trt)-OH (415 mg, 1098 µmol) was condensed at room temperature for 30 minutes using a condensing agent HATU (417 mg, 1098 µmol) and DIEA (192 µL) (reaction step r4-10).

Finally, after the Fmoc groups were deprotected by pyperidine treatment, excision from the solid-phase carrier MBHA and deprotection of the Lys-protecting Boc groups, Arg-protecting Mts groups and Ser-protecting Trt groups were performed by a standard method (TFA/TFMSA/p-cresol/thioanisol = 60/25/10/10) to give the target (compound (1-D)) (reaction step r4-11). MALDI-TOF MS: calcd. 3167.92 (M+H⁺), found 3166.89.

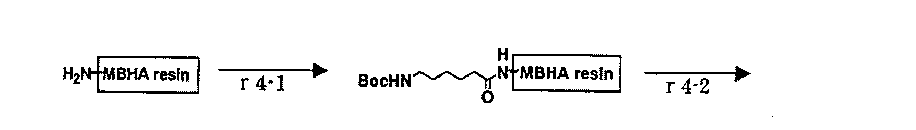

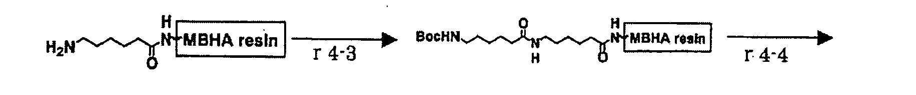

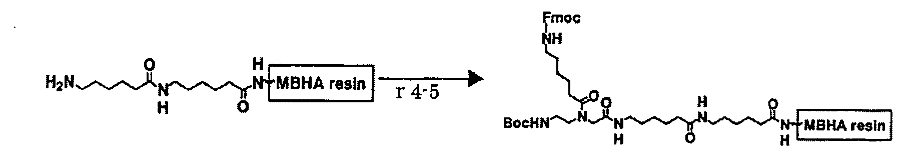

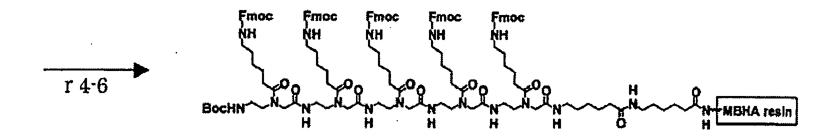

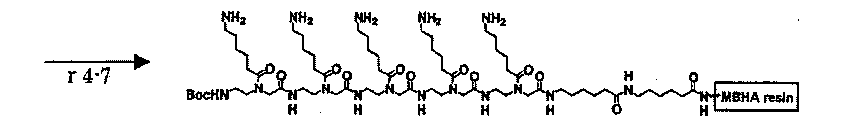

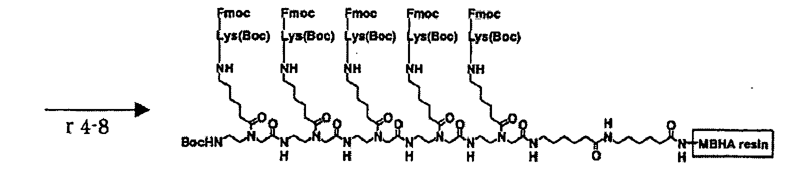

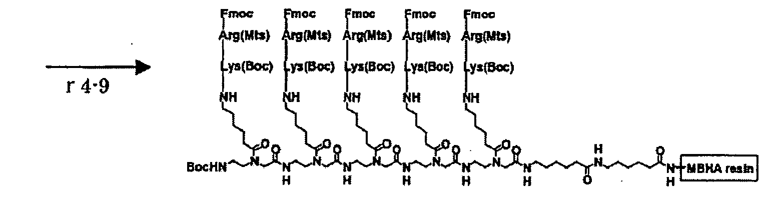

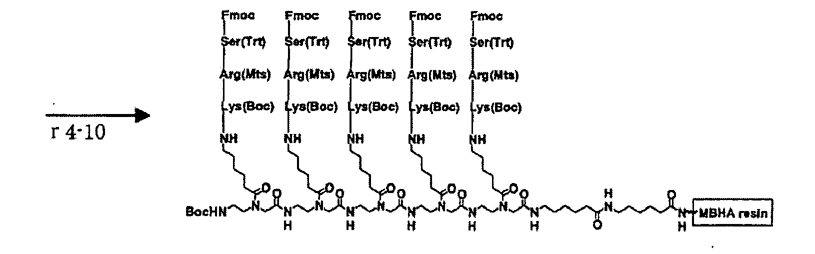

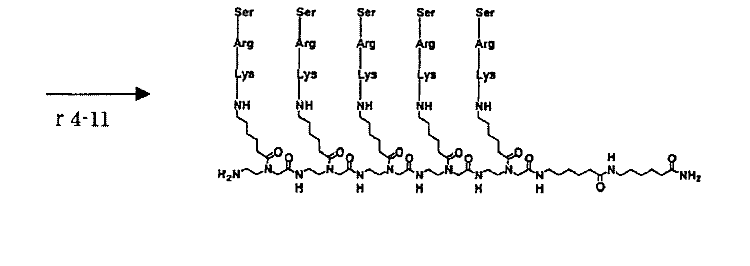

### Example 5:

### Synthesis of compound (1L-A)

The compound represented by the following general formula (1L-A) was synthesized.

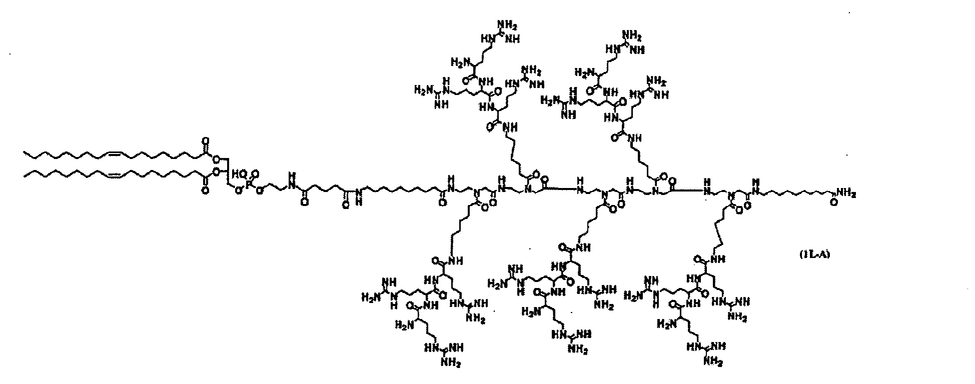

Specifically, according to the standard Fmoc method (cf. Carpino, L.A.; Han, G.Y. J. Org. Chem. 1972, 37, 3404-9.), first, the Fmoc group was deprotected by piperidine treatment (20% piperidine in DMF) of a solid-phase carrier PAL (5-(4'-aminomethyl-3',5'-dimethoxyphenoxy)-valeric acid) (180 mg, 72 µmol) at room temperature for 5 minutes. Condensation was then carried out at room temperature for 30 minutes by applying thereto a linker ω-amino acid Fmoc-HN-C₁₀H₂₀-COOH (305 mg, 720 µmol), a condensing agent HATU (274 mg, 720 µmol) and DIEA (126 µL) (reaction step r5-1)

Subsequently, after the Fmoc group was deprotected by pyperidine treatment, compound (11) (193 mg, 360 µmol) was condensed at room temperature for 30 minutes using a condensing agent HATU (137 mg, 360 µmol) and DIEA (63 µL). This operation was performed five times in total (reaction steps r5-2 to r5-4).

Next, after the Fmoc groups were deprotected by pyperidine treatment, condensation was carried out at room temperature for 30 minutes using Fmoc-HN-C₁₀H₂₀-COOH (305 mg, 720 µmol), a condensing agent HATU (274 mg, 720 µmol) and DIEA (126 µL) (reaction step r5-5).

Subsequently, the Fmoc groups were deprotected by piperidine treatment (reaction step r5-6). DOPE-NHS (dioleoylphosphatidylethanolamine-N-hydroxysuccinimide) (90.5 mg, 92.3 µmol), which is an activated ester of phospholipid, was then condensed using a DMF/DIEA (1 mL/50 µL) solution (reaction step r5-7).

Subsequently, treatment with a CHCl₃/AcOH/N-methylmorphorine solution of Pd(PPh₃)₄ (tetrakis (triphenylphosphine)palladium(O)) (312 mg, 2.8 mL, 1.5 mL, 0.75 mL) at room temperature for 30 minutes was performed twice to deprotect the Alloc groups (reaction step r5-8). Fmoc-Arg(Pbf)-OH.0.3IPE (733 mg, 1080 µmol) was then condensed at room temperature for 30 minutes using a condensing agent HATU (410 mg, 1080 µmol) and DIEA (188 µL). This operation was performed three times in total (reaction steps r5-9 to r5-11).

Finally, after the Fmoc groups were deprotected by pyperidine treatment, excision from the solid-phase carrier PAL and deprotection of the Arg-protecting Pbf groups were performed by TFA treatment (95% TFA/5% m-cresol) to give the target (compound (1L-A)) (reaction step r5-12). MALDI-TOF MS: calcd. 4632.90 (M+H⁺), found 4632.63.

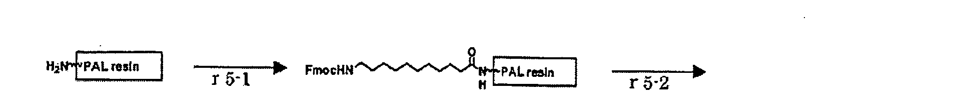

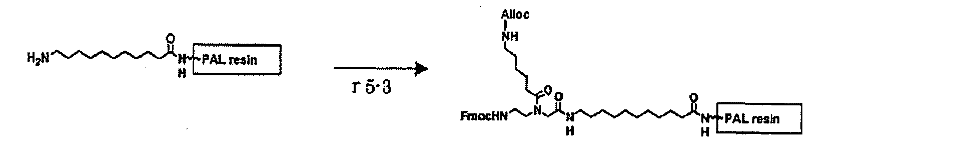

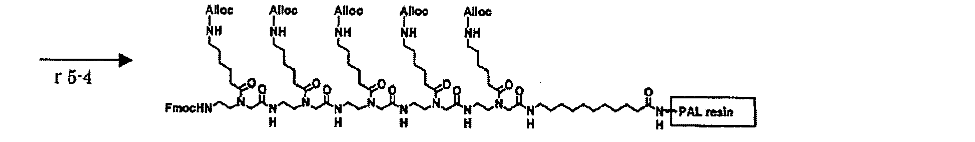

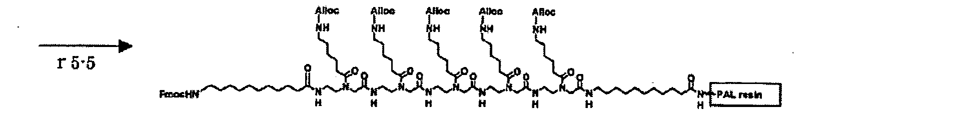

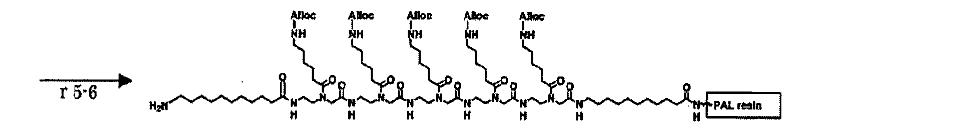

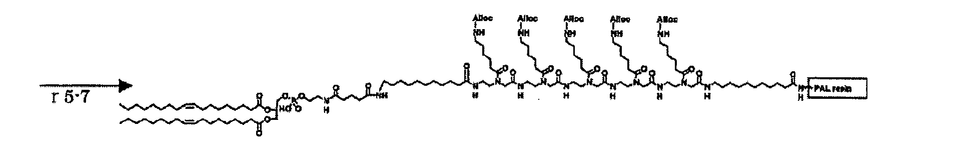

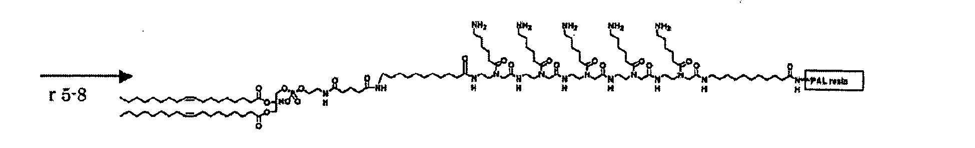

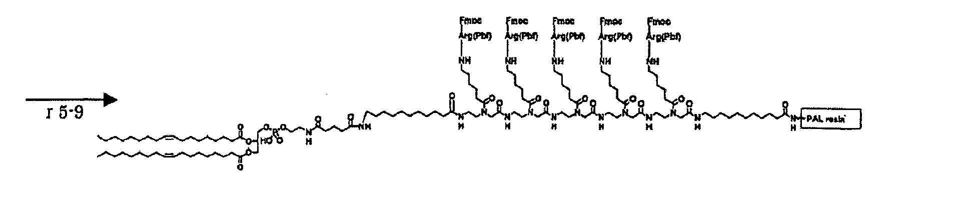

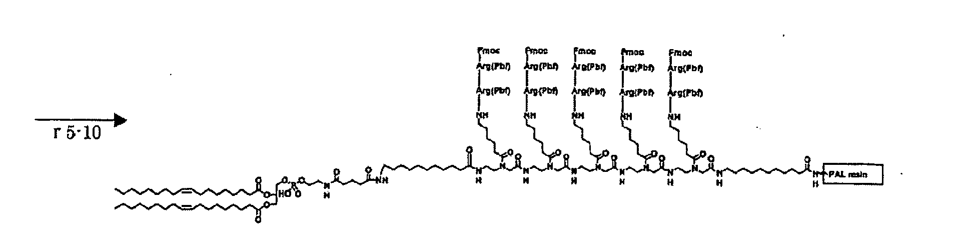

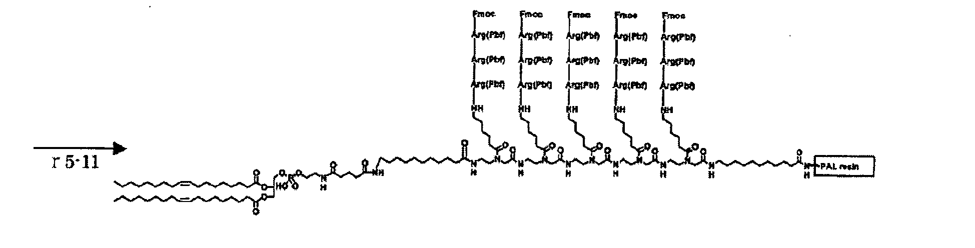

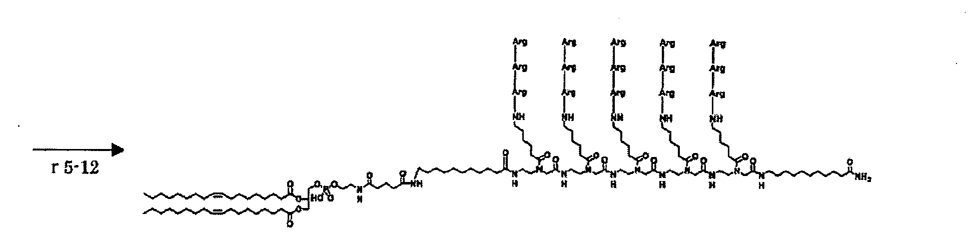

### Example 6:

### Synthesis of compound represented by general formula (1L-B)

The compound represented by the following general formula (1L-B) (hereafter referred to as compound (1L-B)) was synthesized.

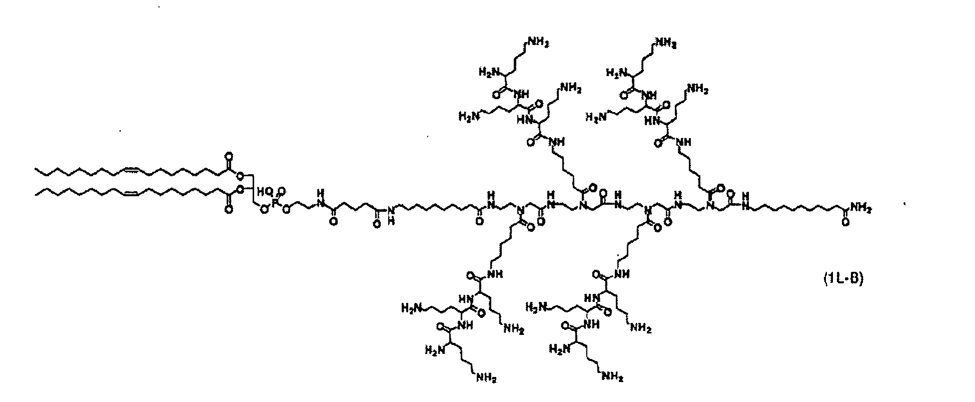

Specifically, according to the standard Fmoc method (cf. Carpino, L.A.; Han, G.Y. J. Org. Chem. 1972, 37, 3404-9.), first, the Fmoc group was deprotected by piperidine treatment (20% piperidine in DMF) of a solid-phase carrier PAL (180 mg, 72 µmol) at room temperature for 5 minutes. Condensation was then carried out at room temperature for 30 minutes by applying thereto a linker ω-amino acid Fmoc-HN-C₁₀H₂₀-COOH (305 mg, 720 µmol), a condensing agent HATU (274 mg, 720 µmol) and DIEA (126 µL) (reactions step r6-1).

Subsequently, after the Fmoc group was deprotected by pyperidine treatment, compound (11) (193 mg, 360 µmol) was condensed at room temperature for 30 minutes using a condensing agent HATU (137 mg, 360 µmol) and DIEA (63 µL). This operation was performed four times in total (reaction step r6-2 to r6-4).

Next, after the Fmoc group was deprotected by pyperidine treatment, condensation was carried out at room temperature for 30 minutes using Fmoc-HN-C₁₀H₂₀-COOH (305 mg, 720 µmol), a condensing agent HATU (274 mg, 720 µmol) and DIEA (126 µL) (reaction step r6-5).

Subsequently, the Fmoc group was deprotected by piperidine treatment (reaction step r6-6). A phospholipid active ester DOPE-NHS (dioleoylphosphatidylethanolamine-N-hydroxysuccinimide) (90.5 mg, 92.3 µmol) was then condensed using a DMF/DIEA (1 mL/50 µL) solution (reaction step r6-7).

Further, treatment with a CHCl₃/AcOH/N-methylmorphorine solution of Pd(PPh₃)₄ (312 mg, 2.8 mL, 1.5 mL, 0.75 mL) at room temperature for 30 minutes was performed twice to deprotect the Alloc groups (reaction step r6-8). Fmoc-Lys(Boc)-OH (525 mg, 1080 µmol) was then condensed at room temperature for 30 minutes using a condensing agent HATU (410 mg, 1080 µmol) and DIEA (188 µL). This operation was performed three times in total (reaction steps r6-9 to r6-11).

Finally, after the Fmoc groups were deprotected by pyperidine treatment, excision from the solid-phase carrier PAL and deprotection of the Lys-protecting Boc groups were performed by TFA treatment (95% TFA/5% m-cresol) to give the target (compound (1L-B)) (reaction step r6-12). MALDI-TOF MS: calcd. 3614.91 (M+H⁺), found 3611.60.

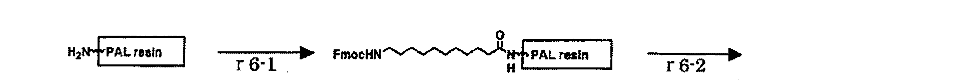

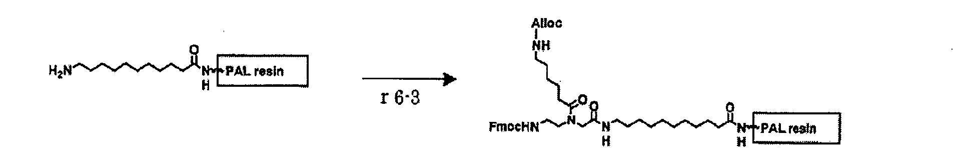

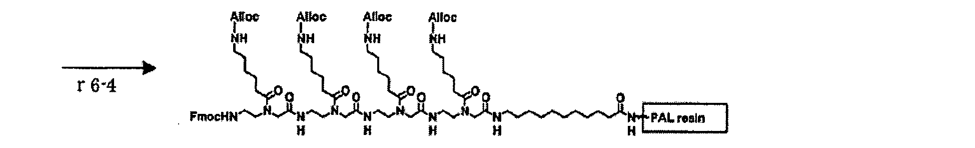

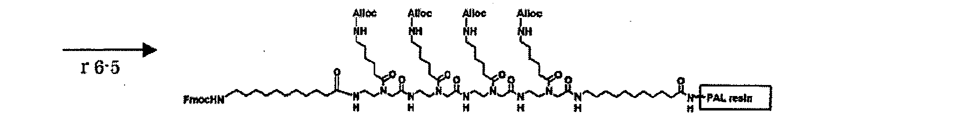

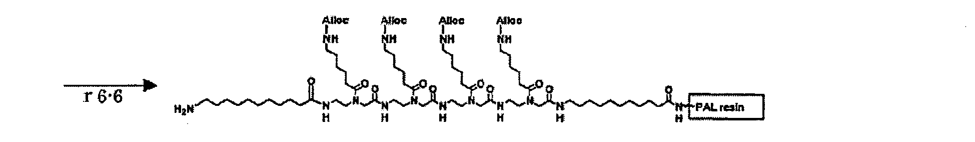

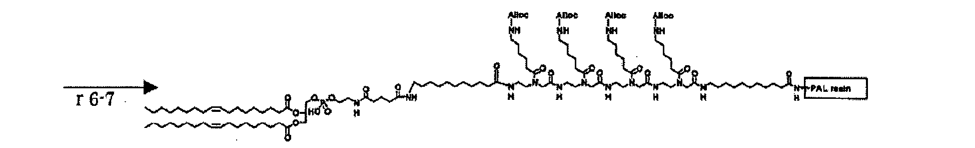

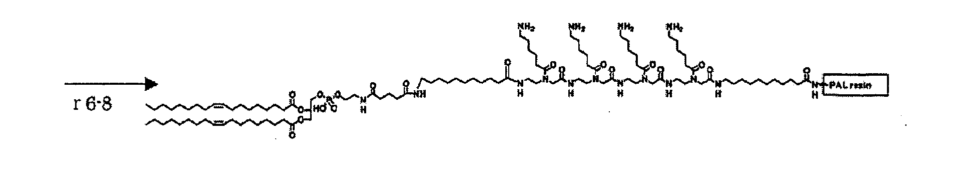

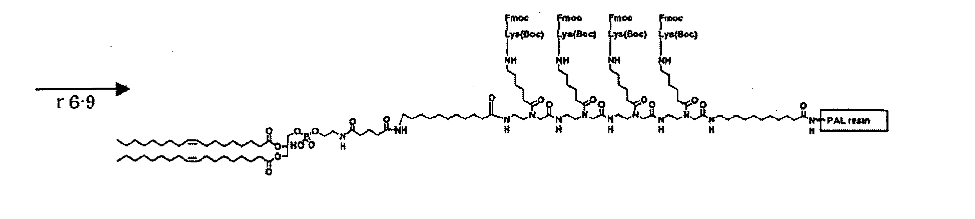

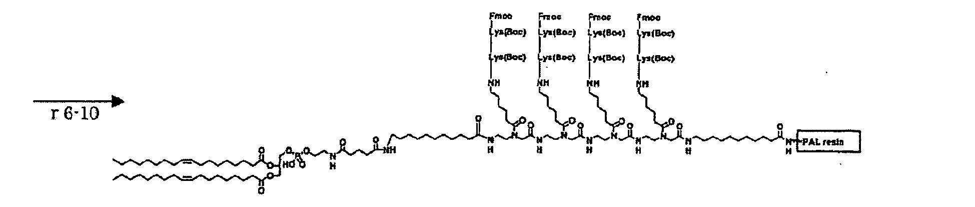

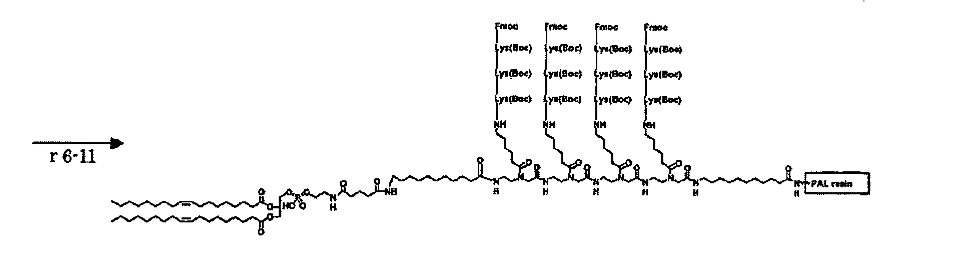

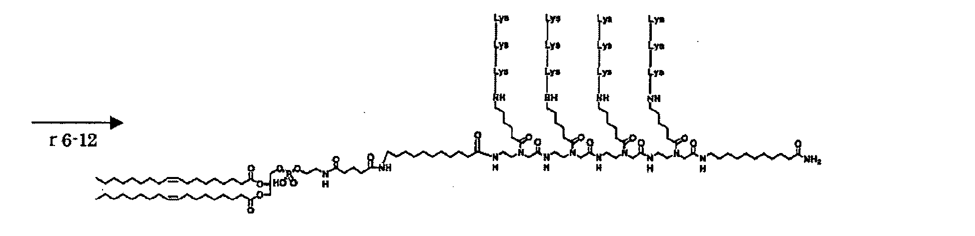

### Example 7:

### Synthesis of compound represented by general formula (1L-C)

The compound represented by the following general formula (1L-C) (hereafter referred to as compound (1L-C)) was synthesized.

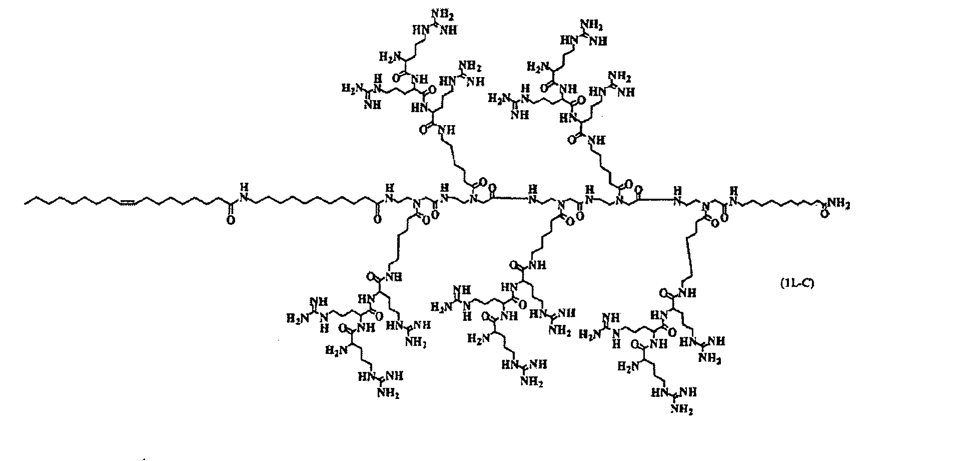

Specifically, according to the standard Fmoc method (cf. Carpino, L.A.; Han, G.Y. J. Org. Chem. 1972, 37, 3404-9.), first, the Fmoc group was deprotected by piperidine treatment (20% piperidine in DMF) of a solid-phase carrier PAL (180 mg, 72 µmol) at room temperature for 5 minutes. Condensation was then carried out at room temperature for 30 minutes by applying thereto a linker ω-amino acid Fmoc-HN-C₁₀H₂₀-COOH (305 mg, 720 µmol), a condensing agent HATU (274 mg, 720 µmol) and DIEA (126 µL) (reaction step r7-1).

Subsequently, the Fmoc group was deprotected by pyperidine treatment, and compound (11) (193 mg, 360 µmol) was then condensed at room temperature for 30 minutes using a condensing agent HATU (137 mg, 360 µmol) and DIEA (63 µL). This operation was performed five times in total (reaction steps r7-2 to r7-4).

Next, after the Fmoc group was deprotected by pyperidine treatment, codensation was carried out at room temperature for 30 minutes using Fmoc-HN-C₁₀H₂₀-COOH (305 mg, 720 µmol), a condensing agent HATU (274 mg, 720 µmol) and DIEA (126 µL) (reaction step r7-5).

Subsequently, the Fmoc group was deprotected by piperidine treatment (reaction step r7-6). Oleic acid (40 mg, 144 µmol) was then condensed using a condensing agent HATU (54.6 mg, 144 µmol) and DIEA (25 µL) (reaction step r7-7) .

Further, treatment with a CHCl₃/AcOH/N-methylmorphorine solution of Pd(PPh₃)₄ (312 mg, 2.8 mL, 1.5 mL, 0.75 mL) at room temperature for 30 minutes was performed twice to deprotect the Alloc groups (reaction step r7-8). Fmoc-Arg(Pbf)-OH·0.3IPE (733 mg, 1080 µmol) was then condensed at room temperature for 30 minutes using a condensing agent HATU (410 mg, 1080 µmol) and DIEA (188 µL). This operation was performed three times in total (reaction steps r7-9 to r7-11).

Finally, after the Fmoc groups were deprotected by pyperidine treatment, excision from the solid-phase carrier PAL and deprotection of the Arg-protecting Pbf groups were performed by TFA treatment (95% TFA/5% m-cresol) to give the target (compound (1L-C)) (reaction step r7-12).

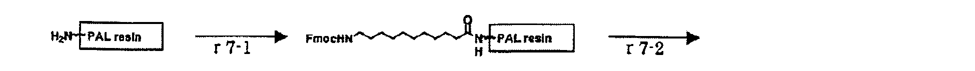

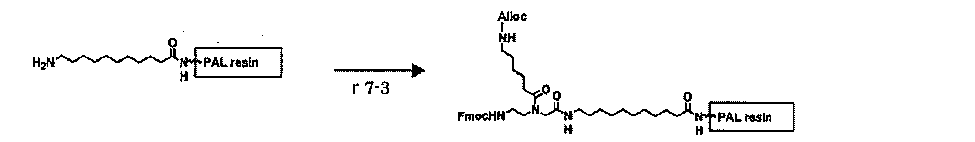

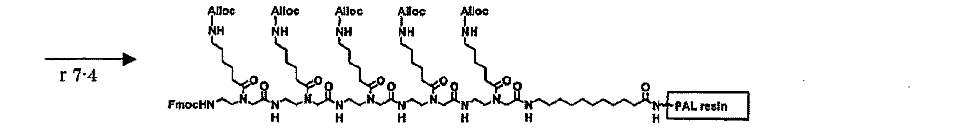

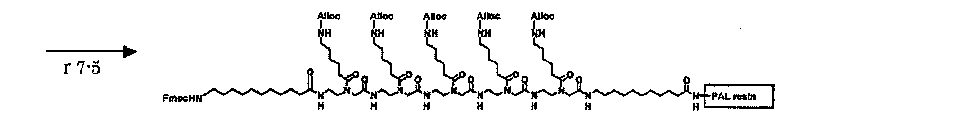

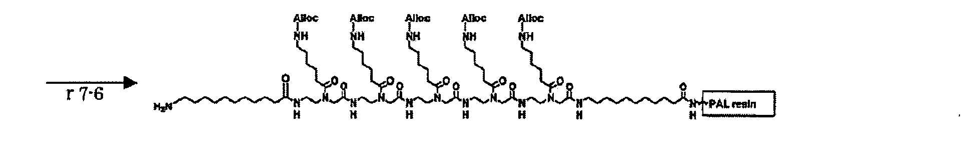

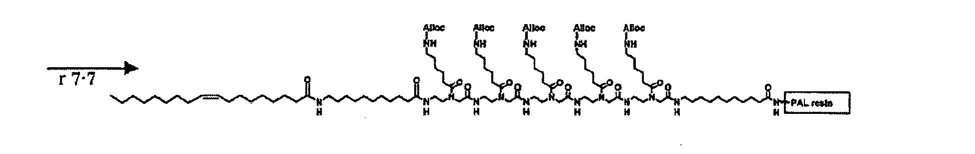

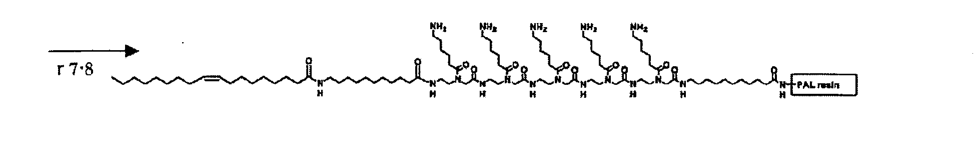

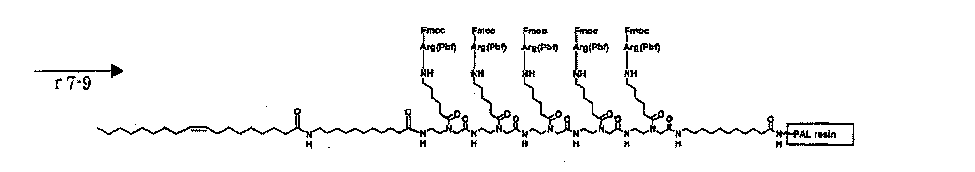

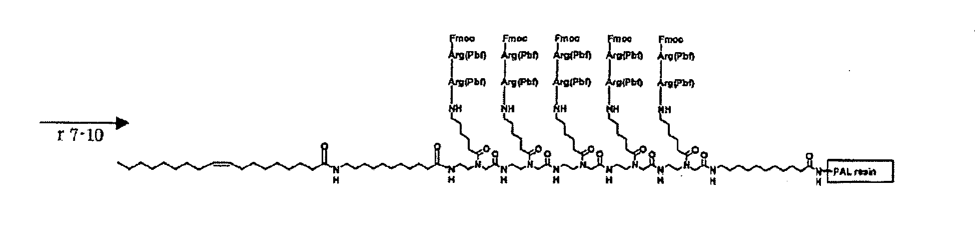

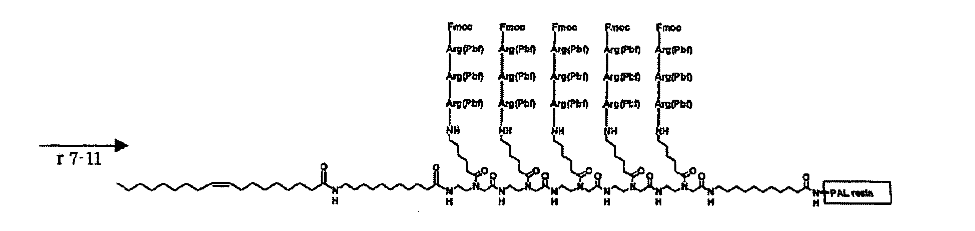

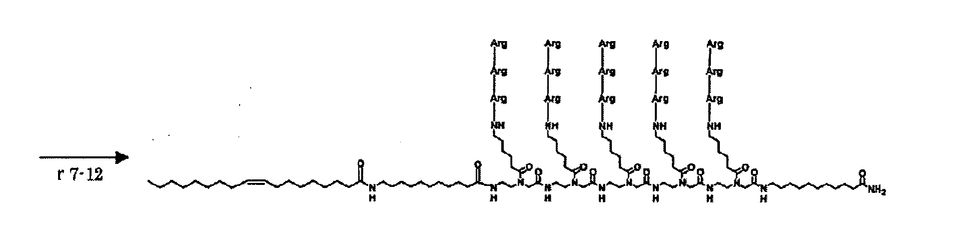

### Example 8:

### Synthesis of compound represented by general formula (1L-D)

The compound represented by the following general formula (1L-D) (hereafter referred to as compound (1L-D)) was synthesized.

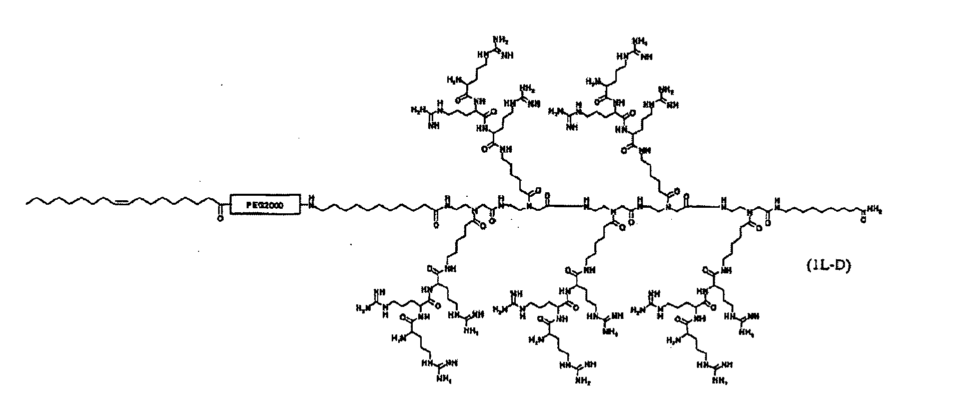

Specifically, according to the standard Fmoc method (cf. Carpino, L.A.; Han, G.Y. J. Org. Chem. 1972, 37, 3404-9.), first, the Fmoc group was deprotected by piperidine treatment (20% piperidine in DMF) of a solid-phase carrier PAL (180 mg, 72 µmol) at room temparature for 5 minutes. Condensation was then carried out at room temperature for 30 minutes by applying thereto a linker ω-amino acid Fmoc-HN-C₁₀H₂₀-COOH (305 mg, 720 µmol), a condensing agent HATU (274 mg, 720 µmol) and DIEA (126 µL) (reaction step r8-1).

Subsequently, the Fmoc group was deprotected by pyperidine treatment, and compound (11) (193 mg, 360 µmol) was then condensed at room temperature for 30 minutes using a condensing agent HATU (137 mg, 360 µmol) and DIEA (63 µL). This operation was performed five times in total (reaction steps r8-2 to r8-4).

Next, after the Fmoc group was deprotected by pyperidine treatment, condensation was carried out at room temperature for 30 minutes using Fmoc-HN-C₁₀H₂₀-COOH (305 mg, 720 µmol), a condensing agent HATU (274 mg, 720 µmol) and DIEA (126 µL) (reaction step r8-5).

Subsequently, the Fmoc group was deprotected by piperidine treatment (reaction step r8-6). A phospholipid active ester Ole-PEG-NHS (oleic acid-polyethylene glycol-N-hydroxysuccinimide) (306 mg, 144 µmol) was then condensed using a DMF/DIEA (1 mL/50 µL) solution (reaction step r8-7).

Further, treatment with a CHCl₃/AcOH/N-methylmorphorine solution of Pd(PPh₃)₄ (312 mg, 2.8 mL, 1.5 mL, 0.75 mL) at room temperature for 30 minutes was performed twice to deprotect the Alloc groups (reaction step r8-8). Fmoc-Arg(Pbf)-OH.0.3IPE (733 mg, 1080 µmol) was then condensed at room temperature for 30 minutes using a condensing agent HATU (410 mg, 1080 µmol) and DIEA (188 µL). This operation was performed three times in total (reaction steps r8-9 to r8-11).

Finally, after the Fmoc groups were deprotected by pyperidine treatment, excision from the solid-phase carrier PAL and deprotection of the Arg-protecting Pbf groups were performed by TFA treatment (95% TFA/5% m-cresol) to give the target (compound (1L-D)) (reaction step r8-12).

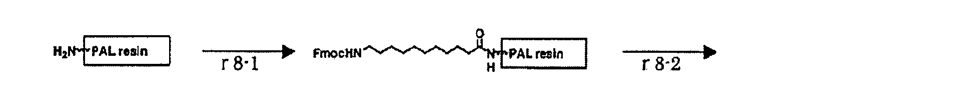

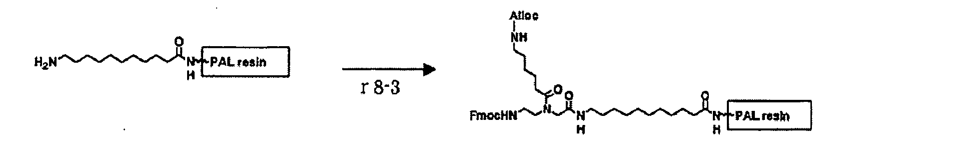

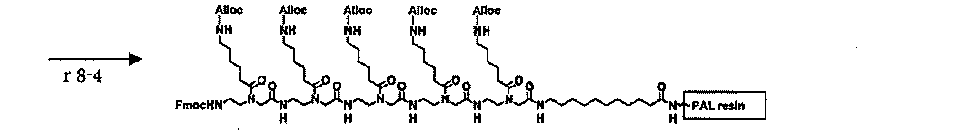

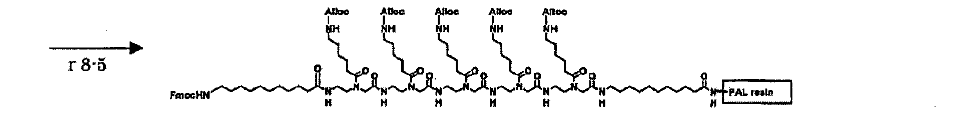

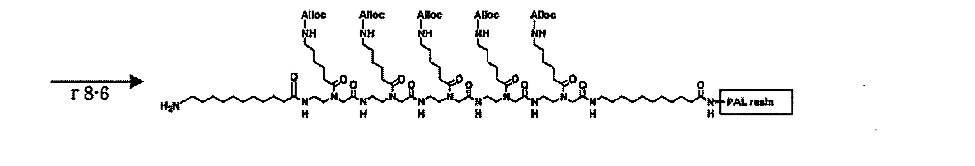

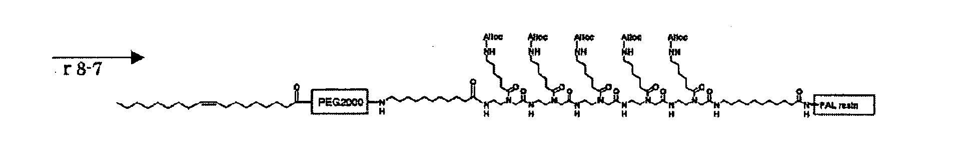

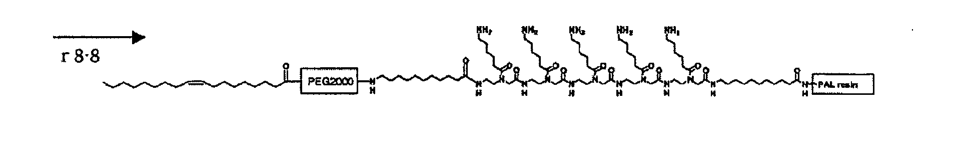

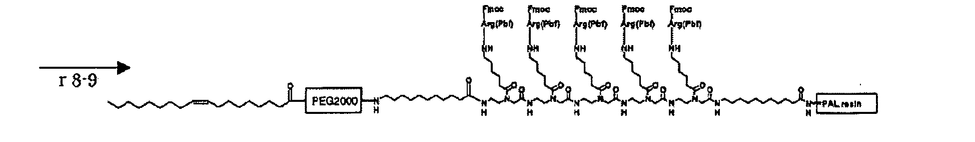

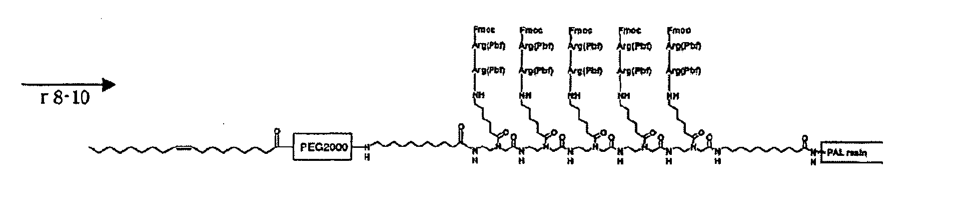

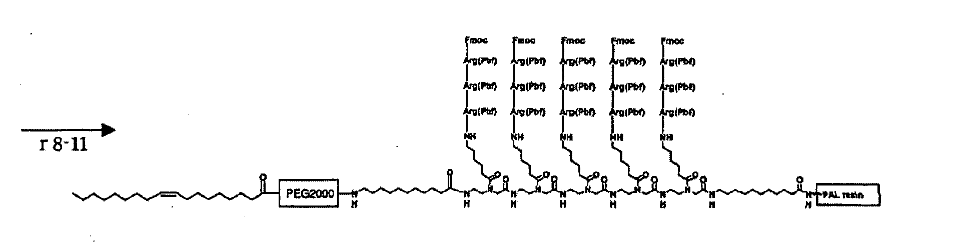

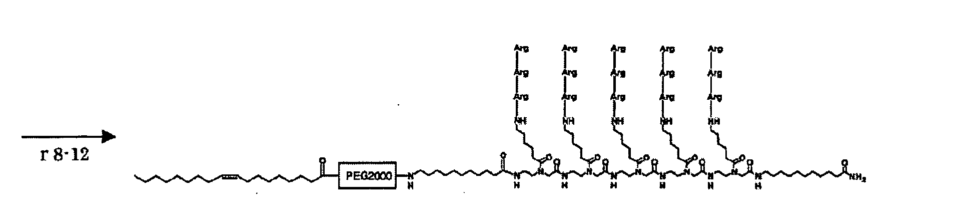

### Example 9:

### Synthesis of compound (1-E)

The compound represented by the following general formula (1-E) was synthesized.

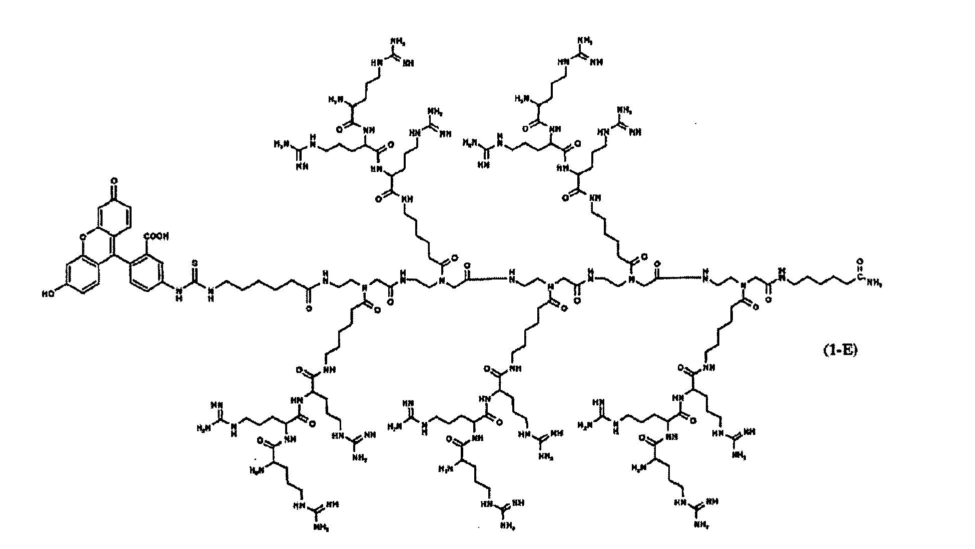

Specifically, according to the standard tBoc method (cf. Koch, T.; Hansen, H.F.; Andersen, P.; Larsen, T.; Batz, H.G.; Otteson, K.; Orum, H. J. Peptide Res. 1997, 49, 80-88.), first, a condensation reaction was carried out at room temperature for 120 minutes by applying a linker ω-amino acid Boc-HN-C₅H₁₀-COOH (6.93 mg, 30 µmol), a condensing agent HATU (11.4 mg, 30 µmol) and DIEA (10µL) to a solid-phase carrier MBHA (100 mg, 61 µmol) (reaction step r9-1).

Subsequently, after the Boc group was depotected by TFA treatment (95% TFA/5% m-cresol), a condensation reaction was carried out at room temperature for 30 minutes using Boc-HN-C₅H₁₀-COOH (13.9 mg, 60 µmol), a condensing agent HATU (22.8 mg, 60 µmol) and DIEA (20µL) (reaction steps r9-2 and 9-3).

Subsequently, the Boc group was deprotected by TFA treatment (95% TFA/5% m-cresol) (reaction step r9-6). The compound represented by the following general formula (91) (19.9 mg, 60 µmol) was then condensed at room temperature for 30 minutes using a condensing agent HATU (13.7 mg, 60 µmol) and DIEA (12.5 µL). This operation was performed five times in total (reaction steps r9-7 and r9-8).

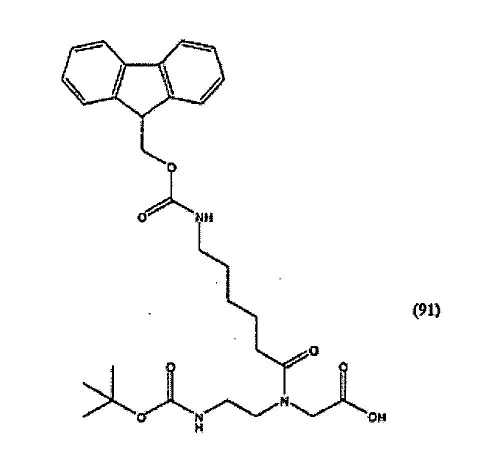

Next, the Fmoc group was deprotected by piperidine treatment (20% piperidine in DMF) at room temperature for 5 minutes (reaction step r9-9). Fmoc-Arg(Mts)-OH·IPE (153 mg, 225 µmol) was then condensed at room temperature for 30 minutes using a condensing agent HATU (85 mg, 225 µmol) and DIEA (39 µL). This operation was performed three times in total (reaction steps r9-10 to r9-12).

Further, after the Boc group was deprotected by TFA treatment, a DIEA/DMF (26.7 mL/1.5 mL) solution of FITC (29.2 mg, 75 µmol) was stirred at room temperature for 14 hours, and fluorescence-labeling was performed (reaction step r9-13).

Finally, after the Fmoc groups were deprotected by pyperidine treatment, excision from the solid-phase carrier MBHA and deprotection of the Arg-protecting Mts groups were performed by a standard method (TFA/TFMSA/p-cresol/thioanisol = 60/25/10/10) to give the target (compound (1-E)) (reaction step r9-14). MALDI-TOF MS: calcd. 4041.40, (M+H⁺), found 4042.91.

### Example 10:

### Synthesis of compound represented by general formula (1a-1)

According to the reaction steps given below, the compound represented by the following general formula (1a-1) was synthesized.

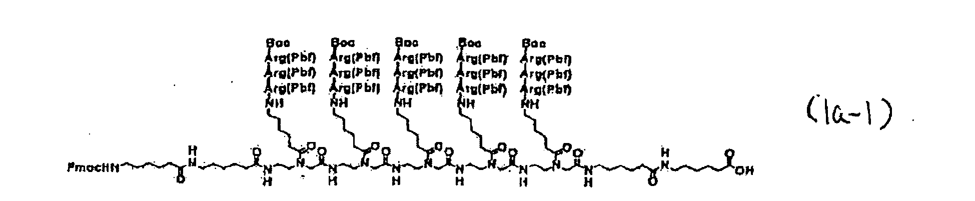

Specifically, according to the standard tBoc method (cf. Koch, T.; Hansen, H.F.; Andersen, P.; Larsen, T.; Batz, H.G.; Otteson, K.; Orum, H. J. Peptide Res. 1997, 49, 80-88.), first, a condensation reaction was carried out at room temperature for 15 hours by applying a linker ω-amino acid Boc-HN-C₅H₁₀-COOH (181 mg, 480 µmol) and a condensing agent DCC (124 mg, 480 µmol) to a solid-phase carrier Oxime (100 mg, 120 µmol). After the Boc group was depotected by TFA treatment (95% TFA/5% m-cresol), stepwise extension reactions were carried out at room temperature for 30 minutes using Boc-HN-C₅H₁₀-COOH (68 mg, 180 µmol), a condensing agent HATU (68 mg, 180 µmol) and DIEA (83 µL).

Subsequently, the Boc group was deprotected by TFA treatment (95% TFA/5% m-cresol). The compound represented by the following general formula (101) (19.9 mg, 180 µmol) was then condensed at room temperature for 30 minutes using a condensing agent HATU (41 mg, 180 µmol) and DIEA (37.5 µL). This operation was performed five times in total.

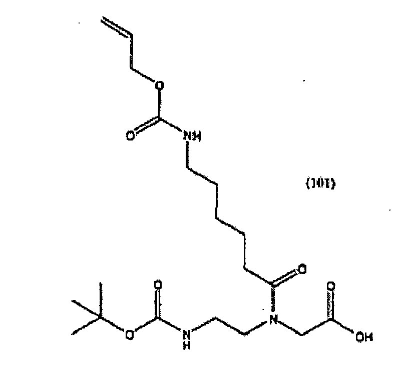

Next, after the Boc group was depotected by TFA treatment (95% TFA/5% m-cresol), stepwise extension reactions were carried out at room temperature for 30 minutes using Boc-HN-C₅H₁₀-COOH (68 mg, 180 µmol), a condensing agent HATU (68 mg, 180 µmol) and DIEA (83 µL).

Subsequently, after the Boc group was depotected by TFA treatment (95% TFA/5% m-cresol), stepwise extension reactions were carried out at room temperature for 30 minutes using Boc-HN-C₅H₁₀-COOH (68 mg, 180 µmol), a condensing agent HATU (68 mg, 180 µmol) and DIEA (83 µL).

Further, treatment with a CHCl₃/AcOH/N-methylmorphorine solution of Pd(PPh₃)₄ (312 mg, 2.8 mL, 1.5 mL, 0.75 mL) at room temperature for 30 minutes was performed twice to deprotect the Alloc groups. Alloc-Arg(Pbf)-OH (459 mg, 900 µmol) was then condensed at room temperature for 30 minutes using a condensing agent HATU (340 mg, 900 µmol) and DIEA (157 µL) . This operation was performed twice in total.

Further, treatment with a CHCl₃/AcOH/N-methylmorphorine solution of Pd(PPh₃)₄ (312 mg, 2.8 mL, 1.5 mL, 0.75 mL) at room temperature for 30 minutes was performed twice to deprotect the Alloc groups. Boc-Arg(Pbf)-OH·IPE (474 mg, 900 µmol) was then condensed at room temperature for 30 minutes using a condensing agent HATU (340 mg, 900 µmol) and DIEA (157 µL).

Finally, excision from the solid-phase carrier oxime was performed with an aqueous dioxane solution of 1N NaOH (70% dioxane/30% water) at room temperature for 5 minutes, and neutralization was then performed to give the target (compound represented by general formula (1a-1)). 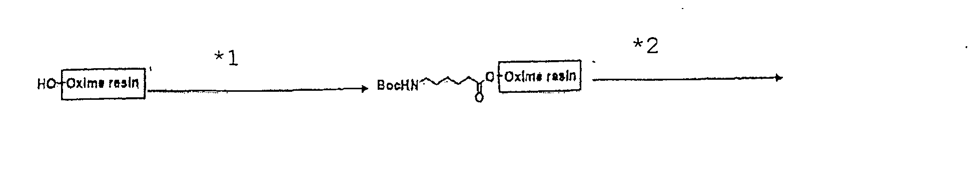 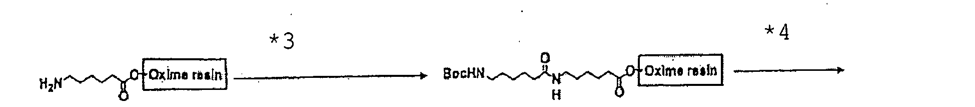 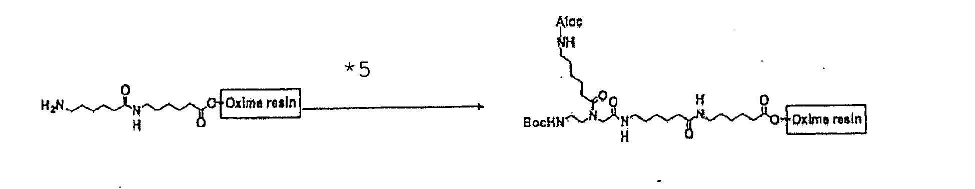 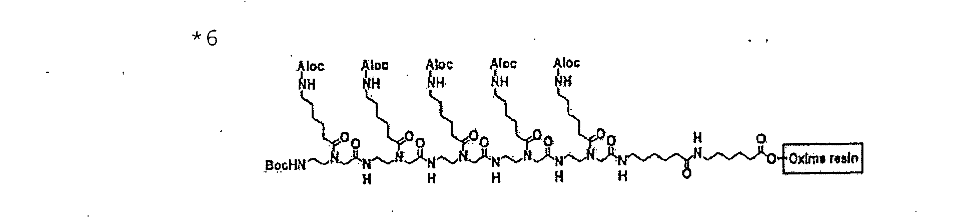 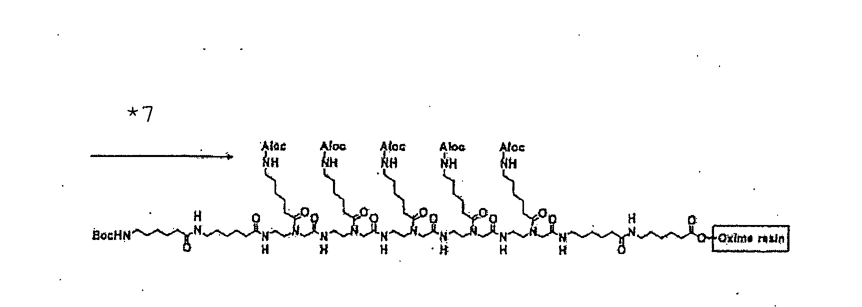 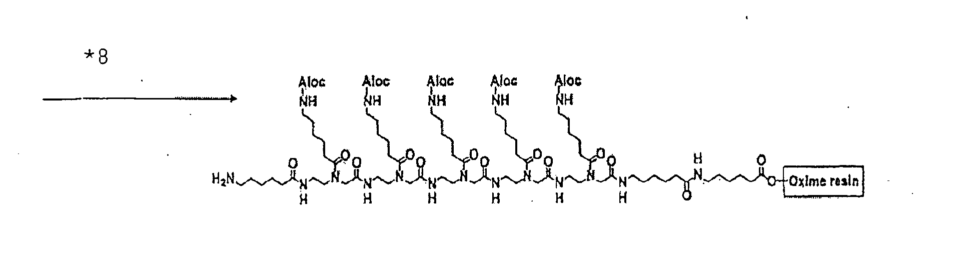
*1 Reaction with: 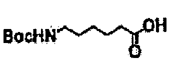 using DCC (dicyclohexylcarbodiimide) as a catalyst
*2 Substitution of a Boc group with a hydrogen atom using TFA containing 5% m-cresol
*3 Dehydration-condensation reaction with 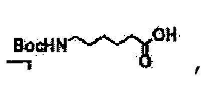 using HATU (2-(1H-9-azabenzotriazol-1-yl)-1,1,3,3-tetramethyluronium hexafluorophosphate) as a catalyst
*4 Substitution of a Boc group using a hydrogen atom with TFA containing 5% m-cresol
*5 Dehydration-condensation reaction with AlocHN-C5-^{Boc}NHC₂-NC₁COOH, using HATU (2-(1H-9-azabenzotriazol-1-yl)-1,1,3,3-tetramethyluronium hexafluorophosphate) as a catalyst
*6 Substitution of a Boc group with a hydrogen atom by treatment with TFA containing 5% m-cresol; dehydration-condensation reaction with AlocHN-C5-^{Boc}NHC₂-NC₁COOH, using HATU (2-(1H-9-azabenzotriazol-1-yl)-1,1,3,3-tetramethyluronium hexafluorophosphate) as a catalyst; both repeated four times
*7 Substitution of a Boc group with a hydrogen atom by treatment with TFA containing 5% m-cresol; dehydration-condensation reaction with 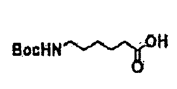 and the terminal amino group, using HATU (2-(1H-9-azabenzotriazol-1-yl)-1,1,3,3-tetramethyluronium hexafluorophosphate) as a catalyst
*8 Substitution of a Boc group with a hydrogen atom by treatment with TFA containing 5% m-cresol

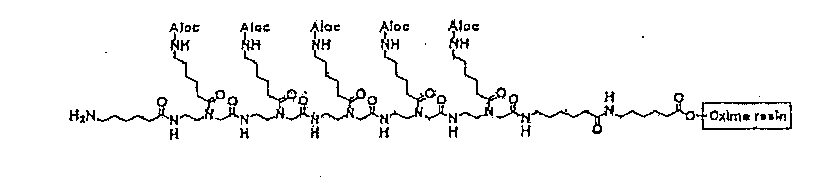

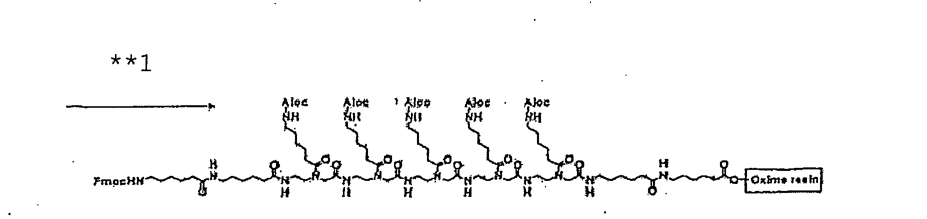

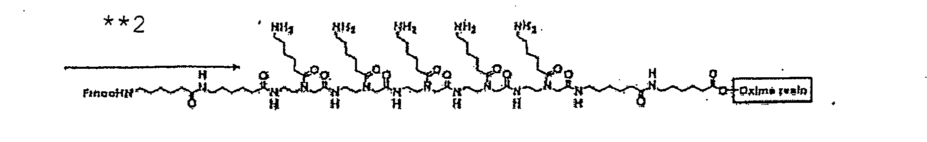

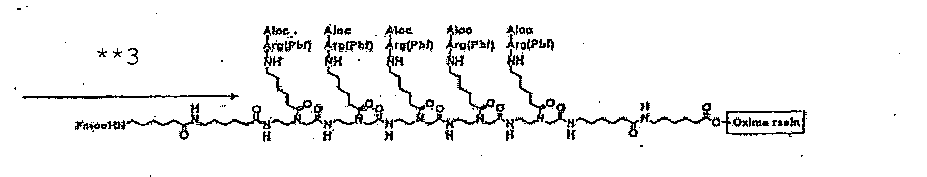

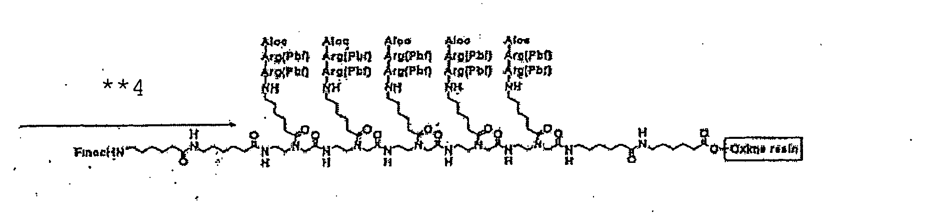

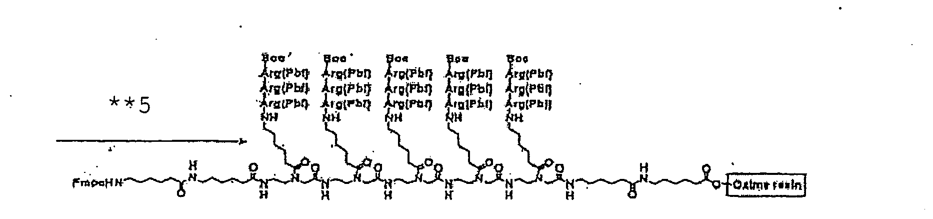

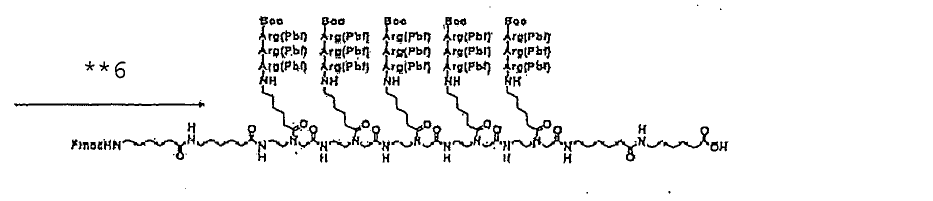

**7
**1 Dehydration-condensation reaction with 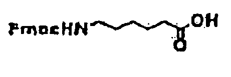 and the terminal amino group, using HATU (2-(1H-9-azabenzotriazol-1-yl)-1,1,3,3-tetramethyluronium hexafluorophosphate) as a catalyst
**2 Reduction to substitute Aloc groups with hydrogen atoms, using Pd(PPh₃)₄ and BH₃ as a catalyst
^{**}3 Dehydration-condensation reaction with Aloc-Arg(Pdf)-OH and the terminal amino group, using HATU (2-(1H-9-azabenzotriazol-1-yl)-1,1,3,3-tetramethyluronium hexafluorophosphate) as a catalyst
^{**}4 Substitution of Aloc groups with hydrogen atoms using Pd(PPh₃)₄ and BH₃ as a catalyst; dehydration-condensation reaction with Aloc-Arg(Pdf)-OH and the terminal amino group, using HATU (2-(1H-9-azabenzotriazol-1-yl)-1,1,3,3-tetramethyluronium hexafluorophosphate) as a catalyst
^{**}5 Substitution of Aloc groups with hydrogen atoms using Pd(PPh₃)₄ and BH₃ as a catalyst; dehydration-condensation reaction with Boc-Arg(Pdf)-OH and the terminal amino group, using HATU (2-(1H-9-azabenzotriazol-1-yl)-1,1,3,3-tetramethyluronium hexafluorophosphate) as a catalyst
**6 Release from Oxime resin in an NaOH solution by adding thereto hydrogen ions in water
**7 The above oligomer having five Boc groups on side chains and one Fmoc group on the straight chain enables, using MBHA resin which has different reaction rates in the side-chain and straight-chain directions in an isolated state, further condensation reactions with PNA monomers or PNA oligomers in the side-chain or straight-chain directions.

### Example 11:

### Synthesis of compound represented by general formula (1a-2)

According to the reaction step given below, the compound represented by the following general formula (1a-2) was synthesized. 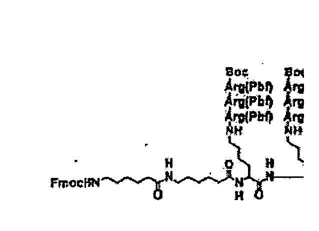 Specifically, according to the standard tBoc method (cf. Koch, T.; Hansen, H.F.; Andersen, P.; Larsen, T.; (1a-2) H.G.; Otteson, K.; Orum, H. J. s. 1997, 49, 80-88.), first, a condensation reaction was carried out at room temperature for 15 hours by applying a linker ω-amino acid Boc-HN-C₅H₁₀-COOH (181 mg, 480 µmol) and a condensing agent DCC (124 mg, 480 µmol) to a solid-phase carrier Oxime (100 mg, 120 µmol). After the Boc group was depotected by TFA treatment (95% TFA/5% m-cresol), stepwise extension reactions were carried out at room temperature for 30 minutes using Boc-HN-C₅H₁₀-COOH (68 mg, 180 µmol), a condensing agent HATU (68 mg, 180 µmol) and DIEA (83 µL).

Subsequently, the Boc group was deprotected by TFA treatment (95% TFA/5% m-cresol). The compound represented by the following general formula (111) (19.9 mg, 180 µmol) was then condensed at room temperature for 30 minutes using a condensing agent HATU (41 mg, 180 µmol) and DIEA (37.5 µL). This operation was performed five times in total.

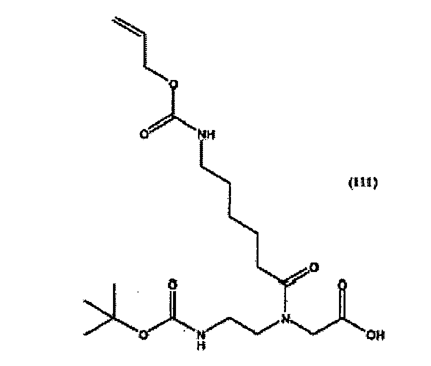

Subsequently, after the Boc group was depotected by TFA treatment (95% TFA/5% m-cresol), stepwise extension reactions were carried out at room temperature for 30 minutes using Boc-HN-C₅H₁₀-COOH (68mg, 180 µmol), a condensing agent HATU (68 mg, 180µmol) and DIEA (83µL).

Next, after the Boc group was depotected by TFA treatment (95% TFA/5% m-cresol), stepwise extension reactions were carried out using Fmoc-HN-C₅H₁₀-COOH (64mg, 180 µmol), a condensing agent HATU (68 mg, 180 µmol) and DIEA (83µL).

Further, treatment with a CHCl_{3/}AcOH/N-methylmorphorine solution of Pd(PPh₃)₄ (312 mg, 2.8 mL, 1.5 mL, 0.75 mL) at room temperature for 30 minutes was performed twice to deprotect the Alloc groups. Alloc-Arg(Pbf)-OH (459 mg, 900 µmol) was then condensed at room temperature for 30 minutes using a condensing agent HATU (340 mg, 900 µmol) and DIEA (157 µL). This operation was performed twice in total.

Further, treatment with a CHCl₃/AcOH/N-methylmorphorine solution of Pd(PPh₃)₄ (312 mg, 2.8 mL, 1.5 mL, 0.75 mL) at room temperature for 30 minutes was performed twice to deprotect the Alloc groups. Boc-Arg(Pbf)-OH.IPE (474 mg, 900 µmol) was then condensed at room temperature for 30 minutes using a condensing agent HATU (340 mg, 900 µmol) and DIEA (157 µL).

Finally, excision from the solid-phase carrier oxime was performed with an aqueous dioxane solution of 1N NaOH (70% dioxane/30% water) at room temperature for 5 minutes, and neutralization was then performed to give the target (compound represented by general formula (1a-2)). 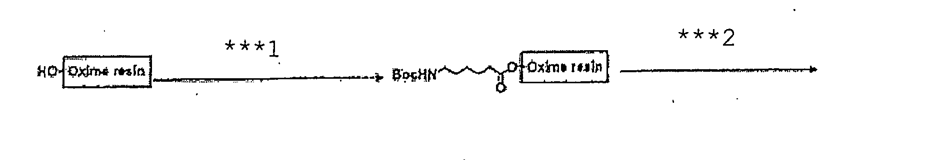 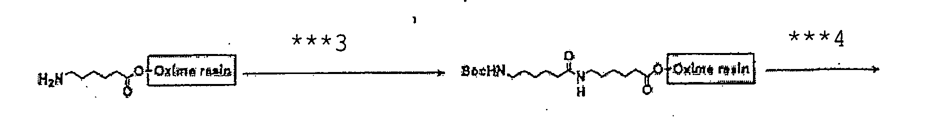 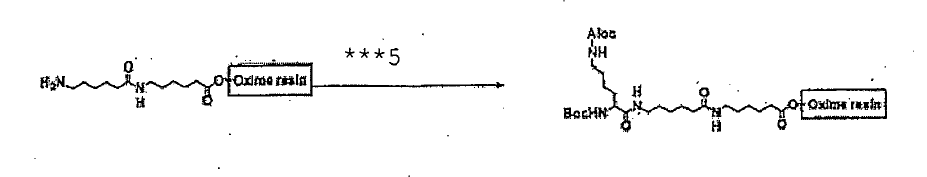 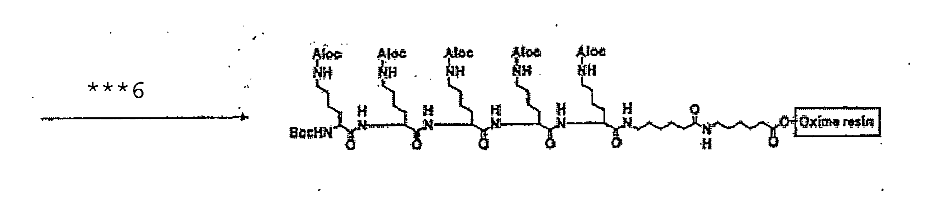 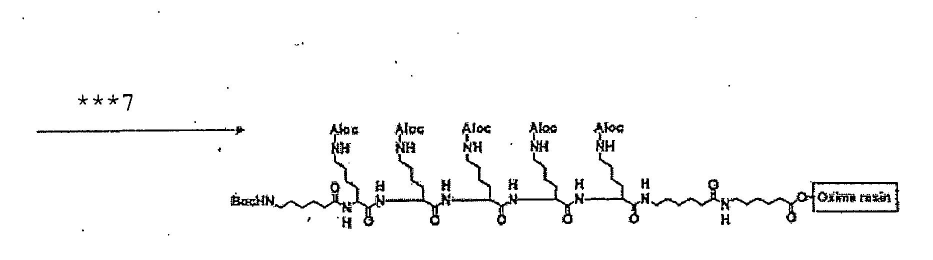 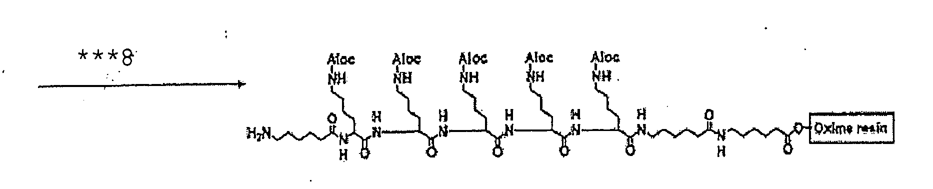
***1 Reaction with 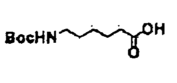 using DCC (dicyclohexylcarbodiimide) as a catalyst
^{***}2 Substitution of a Boc group with a hydrogen atom with TFA containing 5% m-cresol
***3 Dehydration-condensation reaction with 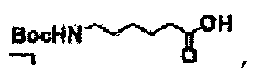 using HATU (2-(1H-9-azabenzotriazol-1-yl)-1,1,3,3-tetramethyluronium hexafluorophosphate) as a catalyst
***4 Substitution of a Boc group with a hydrogen atom with TFA containing 5% m-cresol
***5 Dehydration-condensation reaction with Boc-Lys(Aloc)-OH, using HATU (2-(1H-9-azabenzotriazol-1-yl)-1,1,3,3-tetramethyluronium hexafluorophosphate) as a catalyst
***6 Substitution of a Boc group with a hydrogen atom by treatment with TFA containing 5% m-cresol; dehydration-condensation reaction with Boc-Lys(Aloc)-OH, using HATU (2-(1H-9-azabenzotriazol-1-yl)-1,1,3,3-tetramethyluronium hexafluorophosphate) as a catalyst; repeated four times
***7 Substitution of a Boc group with a hydrogen atom by treatment with TFA containing 5% m-cresol; dehydration-condensation reaction with 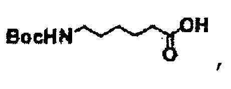 using HATU (2-(1H-9-azabenzotriazol-1-yl)-1,1,3,3-tetramethyluronium hexafluorophosphate) as a catalyst
***8 Substitution of a Boc group with a hydrogen atom by treatment with TFA containing 5% m-cresol 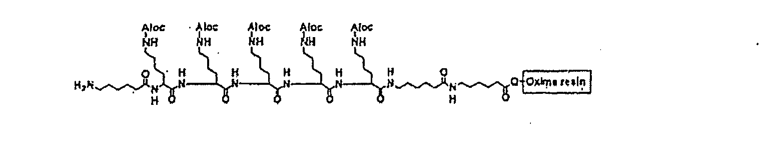 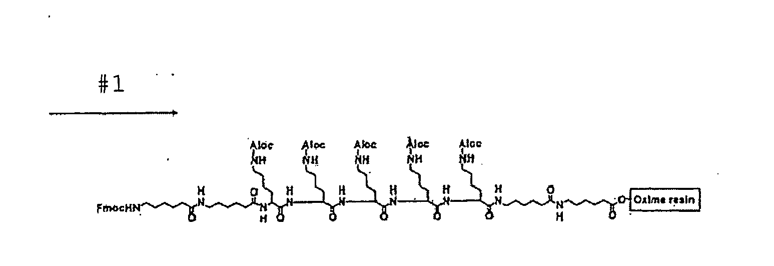 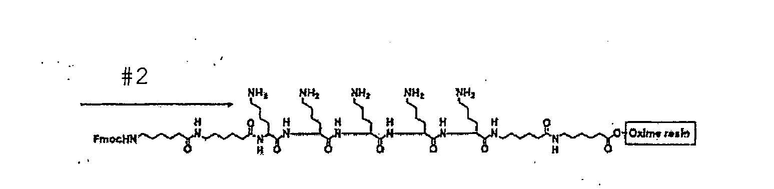 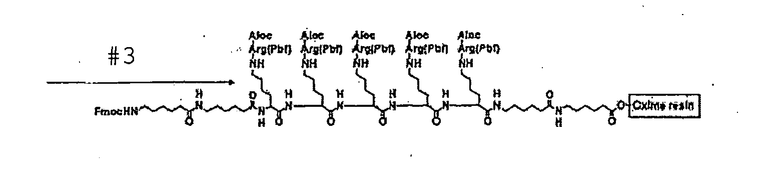 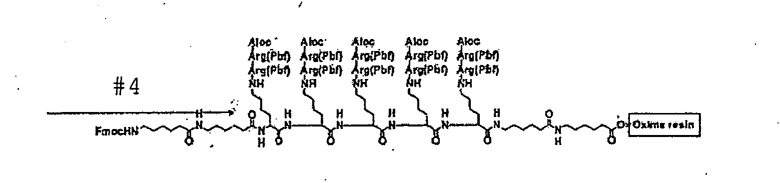 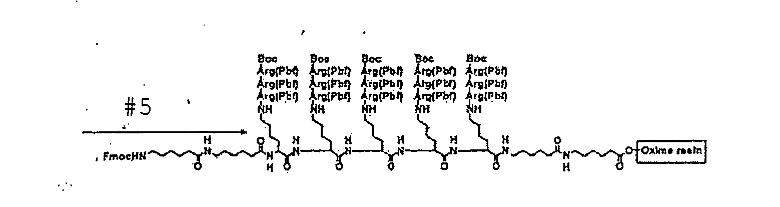 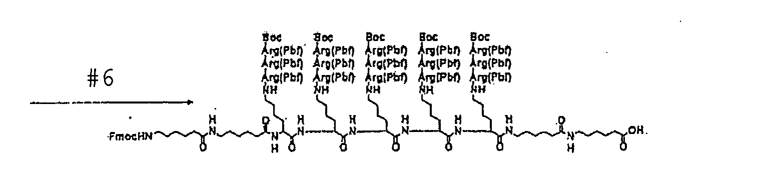
#7

#1 Dehydration-condensation reaction with 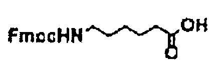 and the terminal amino group, using HATU (2-(1H-9-azabenzotriazol-1-yl)-1,1,3,3-tetramethyluronium hexafluorophosphate) as a catalyst
#2 Reduction to substitute Aloc groups with hydrogen atoms, using Pd(PPh₃)₄ and BH₃ as a catalyst
#3 Dehydration-condensation reaction with Aloc-Arg(Pdf)-OH and the terminal amino group, using HATU (2-(1H-9-azabenzotriazol-1-yl)-1,1,3,3-tetramethyluronium hexafluorophosphate) as a catalyst
#4 Substitution of Aloc groups with hydrogen atoms using Pd(PPh₃)₄ and BH₃ as a catalyst; dehydration-condensation reaction with Aloc-Arg(Pdf)-OH and the terminal amino group, using HATU (2-(1H-9-azabenzotriazol-1-yl)-1,1,3,3-tetramethyluronium hexafluorophosphate) as a catalyst
#5 Substitution of Aloc groups with hydrogen atoms using Pd(PPh₃)₄ and BH₃ as a catalyst; dehydration-condensation reaction with Boc-Arg(Pdf)-OH and the terminal amino group, using HATU (2-(1H-9-azabenzotriazol-1-yl)-1,1,3,3-tetramethyluronium hexafluorophosphate) as a catalyst
#6 Release from Oxime resin in an NaOH solution by adding thereto hydrogen ions in water
#7 The above oligomer having five Boc groups on side chains and one Fmoc group on the straight chain enables, using MBHA resin which has different reaction rates in the side-chain and straight-chain directions in an isolated state, further condensation reactions with PNA monomers or PNA oligomers in the side-chain or straight-chain directions.

### Example 12:

### Synthesis of compound represented by general formula (1a-3)

According to the reaction steps given below, the compound represented by the following general formula (1a-3) was synthesized. 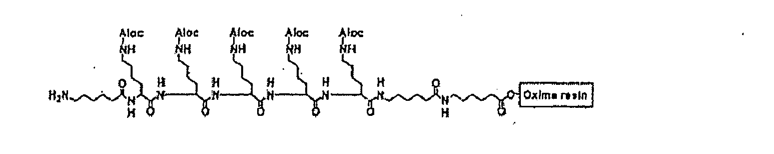 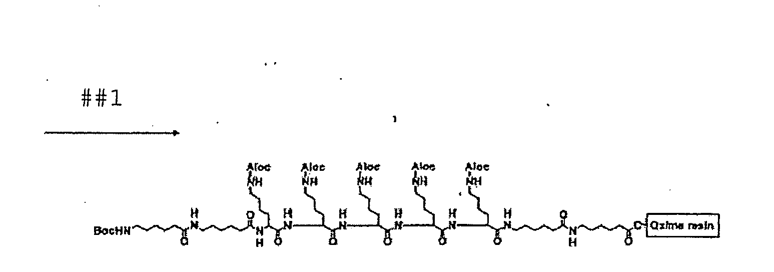 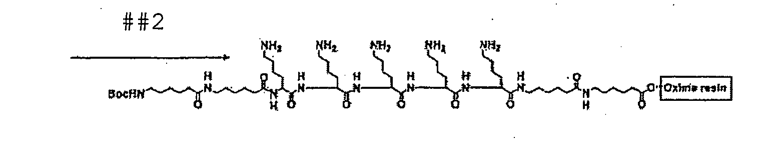 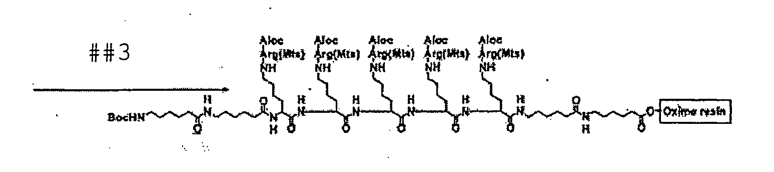 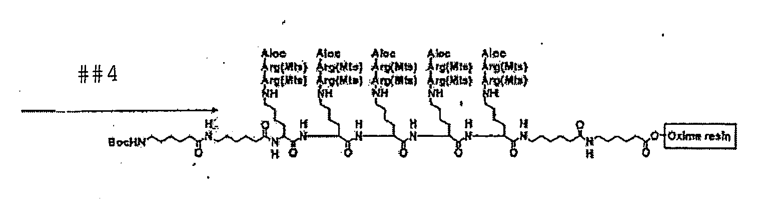 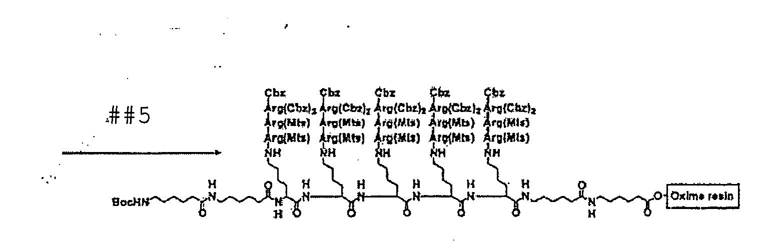 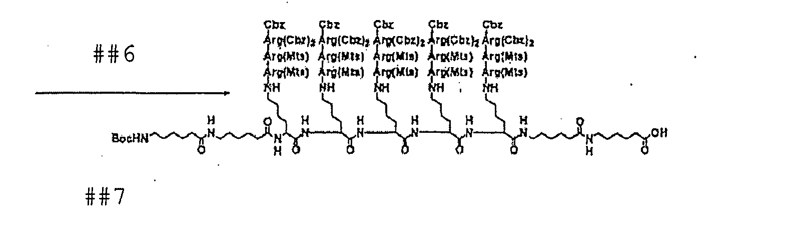
##1 Substitution of a Boc group with a hydrogen atom by treatment with TFA containing 5% m-cresol; dehydration-condensation reaction with 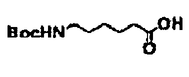 and the terminal amino group, using HATU (2-(1H-9-azabenzotriazol-1-yl)-1,1,3,3-tetramethyluronium hexafluorophosphate) as a catalyst
##2 Reduction to substitute Aloc groups with hydrogen atoms, using Pd(PPh₃)₄ and BH₃ as a catalyst
##3 Dehydration-condensation reaction with Aloc-Arg(Mts)-OH and the terminal amino group, using HATU (2-(1H-9-azabenzotriazol-1-yl)-1,1,3,3-tetramethyluronium hexafluorophosphate) as a catalyst
##4 Substitution of Aloc groups with hydrogen atoms using Pd(PPh₃)₄ and BH₃ as a catalyst; dehydration-condensation reaction with Aloc-Arg(Mts)-OH and the terminal amino group, using HATU (2-(1H-9-azabenzotriazol-1-yl)-1,1,3,3-tetramethyluronium hexafluorophosphate) as a catalyst
##5 Substitution of Aloc groups with hydrogen atoms using Pd(PPh₃)₄ and BH₃ as a catalyst; dehydration-condensation reaction with Cbz-Arg(Cbz)₂-OH and the terminal amino group, using HATU (2-(1H-9-azabenzotriazol-1-yl)-1,1,3,3-tetramethyluronium hexafluorophosphate) as a catalyst
##6 Release from Oxime resin in an NaOH solution by adding thereto hydrogen ions in water
##7 The above oligomer having five Boc groups on side chains and one Fmoc group on the straight chain enables, using MBHA resin which has different reaction rates in the side-chain and straight-chain directions in an isolated state, further condensation reactions with PNA monomers or PNA oligomers in the side-chain or straight-chain directions.

### Reference Test Example 1:

### Nucleic acid transfection experiment (oligo DNA level)

PC3 (human lung adenocarcinoma) cells, UMUC3 (human bladder carcinoma) cells, T24 (human urinary bladder carcinoma) cells and NIH-3T3 (mouse embryonic fibroblast) cells were separately precultured in a 12-well plate, either in a 10% FBS-containing RPMI medium (PC3 cells, NIH-3T3 cells, and T24 cells) or in a 10% FBS-containing DMEM medium (UMUC cells), to attain a 60 to 70% confluent state.

A 1 µl quantity of each of the compounds shown in Table 1 [solutions containing compound (1-A) or (1-B) at 0.37, 0.13 and 0.04 mM] was added to each of a corresponding number of containers each holding 50 µl of non-serum medium, mixed by tapping, and incubated at room temperature for 5 minutes. Subsequently, 0.3 µg of FITC-labeled single-strand DNA (20 mer) (FITC-TAATACGACTCACTATAGGG: product of Proligo) was added to each of the above-prepared media containing a carrier for nucleic acid transfection, mixed by tapping, and incubated at 37°C for 30 minutes (mixed by tapping every 10 minutes) . The obtained mixtures were separately added to the above wells in which the cell culture had been completed, leveled by lightly tapping, and then incubated at 37°C for 4 hours. The cells were collected, and FITC-positive cells were counted by FACS to calculate DNA transfection efficiency (%) in cells. Further, for the comparison with conventional carriers for nucleic acid transfection (Lipofectamine 2000, TransIt-TKO, and Fugene 6), siRNA transfection efficiency (%) in PC3 cells was calculated in the same manner as above using the protocol recommended by the respective manufacturer and corresponding to a transfection nucleic acid amount of 0.3 µg.

The obtained results are shown in Table 1. These results confirmed that compounds (1-A) and (1-B) are excellently suitable for introducing nucleic acids into cells, and are useful as carriers for nucleic acid transfection.

**[Table 1]**

| DNA transfection efficiency in cells (%) | | | | | | |
|---|---|---|---|---|---|---|
| Target gene | Compound | | | | | |
| | Compound of the invention | | | Comparative compound | | |
| | Concentration of compound | Compound (1-A) | Compound (1-B) | Lipofectamine 2000 | TransIT-TKO | FuGene 6 |
| PC3 | 0.37 mM | 86.0 | 83.8 | 35.75 | 44.73 | 0.52 |
| | 0.13 mM | 61.8 | 76.1 | | | |
| | 0.04 mM | 80.4 | 65.0 | | | |
| UMUC3 | 0.37 mM | 21.6 | 61.5 | - | - | - |
| | 0.13 mM | 49.8 | 77.2 | | | |
| | 0.04 mM | 49.6 | 61.7 | | | |
| T24 | 0.37 mM | 16.3 | 31.4 | - | - | - |
| | 0.13 mM | 10.4 | 26.0 | | | |
| | 0.04 mM | 10.6 | 13.4 | | | |
| NH-3T3 | 0.37 mM | 87.1 | 87.0 | - | - | - |
| | 0.13 mM | 93.1 | 93.2 | | | |
| | 0.04 mM | 94.7 | 84.0 | | | |

| | | | | | | |
|---|---|---|---|---|---|---|
| Lipofectamine 2000: product of Invitrogen TransIT-TKO: product of Mirus FuGene 6: product of Roche | | | | | | |

### Reference Examples 1 to 17:

### Production of carriers for nucleic acid transfection

Lipid dissolved in an organic solvent and each of the above cationic liposome compositions were placed in each of a corresponding number of glass vessels of a suitable size (short-neck flask, cuvette, etc.) (see Table 2 for the composition), and the solvent was distilled off using a rotary evaporator (film production). After the solvent was completely removed under vacuum conditions (more than 6 hours), hydration was performed with phosphate buffer (PBS buffer, pH 7.4) to give a final concentration of 2.0 mM. The formed films were completely removed to prepare lipid suspensions. Each of these suspensions was subjected to a "Freeze-thaw" procedure alternating immersion in a dry-ice bath and tepid water, repeatedly 10 times, thus preparing multi-layer liposomes (MLV: multilamellar vesicle).

Subsequently, the MLVs was transformed into LUVs (large unilamellar vesicles), having a particle diameter of about 200 to about 100 nm, or SUVs (small unilamellar vesicles), having a particle diameter of about 20 nm. The LUVs were prepared using standard production apparatus (Mini-Extruder; product of Avanti). The SUVs were prepared using probe tip sonicator (UD-220; product of TOMY) .

**[Table 2]**

| | Composition (%) | | | | | | Particle diameter (nm) |
|---|---|---|---|---|---|---|---|
| | Composition (1L-A) | Composition (1L-B) | DOPE | POPC | Composition (1 L-D) | Composition (1 L-C) | |
| Control | --- | --- | 50 | 50 | --- | --- | 100 |
| Ref. Ex.1 | 10 | --- | 40 | 50 | --- | --- | 100 |
| Ref. Ex. 2 | 1 | --- | 49 | 50 | --- | --- | 100 |
| Ref. Ex. 3 | 50 | --- | 50 | --- | --- | --- | 100 |
| Ref. Ex.4 | 30 | --- | 70 | --- | --- | --- | 200 |
| Ref. Ex.5 | 30 | --- | 70 | --- | --- | --- | 100 |
| Ref. Ex. 6 | 10 | --- | 70 | --- | 20 | --- | 100 |
| Ref. Ex. 7 | 10 | --- | 70 | --- | --- | 20 | 100 |
| Ref. Ex. 8 | 10 | --- | 90 | --- | --- | --- | 100 |
| Ref. Ex.9 | 50 | --- | 50 | --- | --- | --- | SUV |
| Ref. Ex.10 | 30 | --- | 70 | --- | --- | --- | SUV |
| Ref. Ex.11 | 15 | 15 | 70 | --- | --- | --- | SUV |
| Ref. Ex. 12 | --- | 30 | 70 | --- | --- | --- | SUV |
| Ref. Ex.13 | 30 | 10 | 60 | --- | --- | --- | SUV |
| Ref. Ex. 14 | 30 | 20 | 50 | --- | --- | --- | SUV |
| Ref. Ex. 15 | 30 | 30 | 40 | --- | --- | --- | SUV |
| Ref. Ex. 16 | 100 | --- | --- | --- | --- | --- | 100 |
| Ref. Ex.17 | 100 | --- | --- | --- | --- | --- | unchanged |

| | | | | | | | |
|---|---|---|---|---|---|---|---|
| DOPE: 1,2-dioleoyl-sn-grycelo-3-phosphatidylethanolamine POPC: 1-palmitoyl-2-oleoyl-sn-glycelo-3-phosphatidylcholine | | | | | | | |

### Reference Test Example 2:

### Nucleic acid transfection experiment (siRNA level)

In order to attain 50 to 60% confluence at the time of siRNA transfection, PC3 (human lung adenocarcinoma) cells, UMUC3 (human bladder carcinoma) cells and T24 (human urinary bladder carcinoma) cells were separately seeded in a 6-well plate on the day before the transfection and cultured at 37 °C in a 5% CO₂ incubator.

A 100-µl quantity of Opti-MEM was added to each of several 1.5-ml tubes, and two samples each of 1, 3 and 10 µl of PBS solution containing the carriers for nucleic acid transfection of Reference Example 3 in one of the two samples and Reference Example 8 in the other sample (each carrier for nucleic acid transfection content had been adjusted so that compound (1L-A) was at 0.067 mM) were separately added to each tube and then left to stand at room temperature for about 10 minutes. Further, 0.5 µg of FITC-labeled siRNA (fluorescent labeled siRNA ((sense)Fluo-GACCCGCGCCGAGGUGAAGUU/(antisense)CUUCACCUCGGCGCGGGUCUU: product of Proligo) was added to each tube and then left to stand for 15 minutes, forming a complex of siRNA and the carrier for gene transfection.

Separately, 50 µl of Opti-MEM was added to each of several 1.5-ml tubes, and two samples each of 1, 3 and 10 µl of PBS solution containing 0.6 mM compound (1-A) in one of the two samples and 0.6 mM compound (1-B) in the other sample were separately added to each tube, mixed by tapping, and then left to stand at room temperature for 5 minutes. Further, 0.5 µg of FITC-labeled siRNA (same as above) was added to each tube and then left to stand for 30 minutes, forming a complex of the siRNA and carrier for gene transfection.

After a while, the prepared solutions containing the siRNA and carrier for gene transfection were separately added to the wells in which the cells were present at 50 to 60% confluence. The plates were slowly pitched and rolled, and culture was then performed at 37°C under a condition of 5% CO₂ for 2 hours. After the culture was completed, FITC-positive cells were counted by a flow cytometer to calculate the siRNA transfection efficiency (%) in cells. Further, for comparison with conventional carriers for nucleic acid transfection (Lipofectamine 2000, TransIt-TKO, Metafectene, and oligofectamine), siRNA transfection efficiency (%) in cells was calculated in the same manner as above using the protocol recommended by the respective manufacture and corresponding to a transfection nucleic acid amount of 0.5 µg.

The obtained results are shown in Table 3. These results revealed that the use of compound (1L-A) leads to more efficient siRNA transfection in cells than conventional carriers for nucleic acid transfection.

**[Table 3]**

| siRNA transfection efficiency in cells (%) | | | | | | | | | |
|---|---|---|---|---|---|---|---|---|---|
| Target cells | Compound of present invention | | | | | Comparative examples | | | |
| | Amount (µl) | Reference Example 3 | Reference Example 8 | Compound (1-A) | Compound (1-B) | Lipofectamine Matafectene 2000 | Matafectene | Translt-TKO | Oligofectamine |
| PC3 | 1 | 72.7 | 83.2 | 4.3 | 3.5 | 63.4 | 82.2 | 99.4 | 39.3 |
| | 3 | 92.3 | 89.9 | 4.0 | 10.1 | | | | |
| | 10 | 90.9 | 89.6 | 16.1 | 42.0 | | | | |
| UMU C3 | 1 | 89.9 | 80.3 | 5.7 | 5.3 | 22.3 | 87.4 | 93.9 | 61.5 |
| | 3 | 94.5 | 82.1 | 5.4 | 4.4 | | | | |
| | 10 | 90.3 | 85.3 | 5.4 | 7.0 | | | | |
| T24 | 1 | 53.5 | 75.2 | 12.7 | 11.6 | 75.0 | 83.7 | - | 70.9 |
| | 3 | 87.4 | 81.3 | 18.5 | 19.2 | | | | |
| | 10 | 89.7 | 80.6 | 60.5 | 60.7 | | | | |

| | | | | | | | | | |
|---|---|---|---|---|---|---|---|---|---|
| Lipofectamine 2000: product of Invitrogen TransIT-TKO: product of Mirus Metafectene: product of Biontex Oligofectamine: product of Invitrogen | | | | | | | | | |

### Test Example 3:

### Nucleic acid transfection experiment (plasmid DNA level)

In order for PC3 (human lung adenocarcinoma) cells to be at 60 to 70% confluence at the time of plasmid DNA transfection, the cells were seeded in a 6-well plate on the day before the transfection and cultured at 37°C under a condition of 5% CO₂. The culture medium in each well was replaced with a serum-free medium (RPMI medium) two hours before the transfection.

A 250 µl quantity of Opti-MEM was added to a 1.5ml-tube, and 5 µl of PBS solution containing the carrier for nucleic acid transfection of Reference Example 3 (the carrier for nucleic acid transfection content had been adjusted so that compound (1L-A) was at 0.133 mM) was added thereto and then left to stand at room temperature for about 10 minutes. Further, 250 µl of Opti-MEM-containing EGFP recombinant plasmid DNA (pEGFP-N3) (concentration of pEGFP-N3 : 5.0 µg/250 ml) was added thereto, mixed by lightly tapping, and then left to stand for 15 minutes, forming a complex of the plasmid DNA and carrier for nucleic acid transfection.

After a while, the prepared solution containing the siRNA and carrier for gene transfection was added to the wells in which the cells were present at 60 to 70% confluence. The plate was slowly pitched and rolled, and culture was then performed at 37°C under a condition of 5% CO₂ for 2 hours. The medium was then replaced with complete medium, and the culture was further continued at 37°C under a condition of 5% CO₂ for 45 hours (47 hours in total). After the culture was completed, GFP (green-fluorescent protein)-positive cells were measured by a flow cytometer to calculate the plasmid DNA transfection efficiency (%) in cells.

Further, for the comparison with conventional carriers for nucleic acid transfection (FuGene 6, Lipofectamine 2000, and Metafectene), plasmid DNA was transfected into cells according to the protocol recommended by the respective manufacturer, and transfection efficiency (%) was determined in the same manner as above.

The obtained results are shown in Table 4. These results revealed that the use of compound (1L-A) enables plasmid DNA transfection in cells.

**[Table 4]**

| Plasmid DNA transfection efficiency in cells (%) | | | | |
|---|---|---|---|---|
| Carrier for nucleic acid transfection | Present invention | Comparative product | | |
| | Reference Example 3 | Fugene 6 | Lipofectamine 2000 | Metafectene |
| Transfection efficiency | 26.11 | 5.78 | 29.84 | 31.8 |

### Reference Test Example 4:

### Nucleic acid transfection experiment (siRNA level)

In order for PC3 (human lung adenocarcinoma)/Bcl-2 cells (cells in which a Bcl-2-inserted plasmid had been transfected into PC3) to be 50 to 60% confluent at the time of siRNA transfection, the cells were seeded in a 6-well plate on the day before the transfection and cultured at 37°C in a 5% CO₂ incubator.

A 100 µl quantity of Opti-MEM was added to a 1.5ml-tube, and 6 µl of PBS solution containing the nucleic acid of Reference Example 3 (the carrier for nucleic acid transfection content had been adjusted so that compound (1L-A) was at 0.067 mM) was added thereto and pre-incubated at room temperature for 10 minutes. A 13.5 to 450 pmol quantity of bcl-2-corresponding siRNA (siRNA((sense)GACCCGCGCCGAGGUGAAGUU/(Antisense)CUUCACCUCGGCGCGGGU CUU: product of Proligo) was added thereto and left to stand for 15 minutes, forming a complex of the siRNA and carrier for gene transfection. The obtained complex was added dropwise to the wells in which the cells were present in a 50 to 60% confluent state, and incubated at 37°C for about 48 hours. After the culture was completed, bcl-2 protein expression was detected by a standard western blot method.

For the comparison with a conventional carrier for nucleic acid transfection (XtremeGene: product of Roche), transfection efficiency (%) of bcl-2-corresponding siRNA in cells was calculated in the same manner as above using the recommended protocol corresponding to a transfection nucleic acid amount of 0.5 µg (lanes 2 and 3). Further, it was demonstrated that no inhibitory effects on bcl-2 protein expression are caused even when a complex of GFP-corresponding siRNA having no target sequence is transfected in the form of complex (lane 4). Detailed transfection conditions for each lane is shown in the following table.

The obtained results are shown in Fig. 1. These results revealed that when compound (1L-A) is used, siRNA transfected into cells shows protein expression inhibitory effects more efficiently than when the conventional carriers for nucleic acid transfection are used.

### INDUSTRIAL APPLICABILITY

Since the compounds represented by formula (1) of the present invention contain amino acid or oligopeptide side chains, they can very effectively exhibit useful activities attributed to these amino acids and oligopeptides. In particular, when a compound represented by formula (1) contains an amino acid selected from the group consisting of arginine, lysine, and serine or an oligopeptide containing these amino acids, the compound can effectively exhibit membrane permeability.

Further, the compounds represented by formula (1a) of the present invention contain a free amino group protected by a protecting group, and have a terminal free carboxyl group in repeating unit C. Therefore, the compounds represented by formula (1a) can easily be condensed with a target compound containing an amino or hydroxyl group, thereby imparting the useful activities attributed to amino acids and oligopeptides to such a target compound.

## Claims

1. A compound represented by the following formula (1) 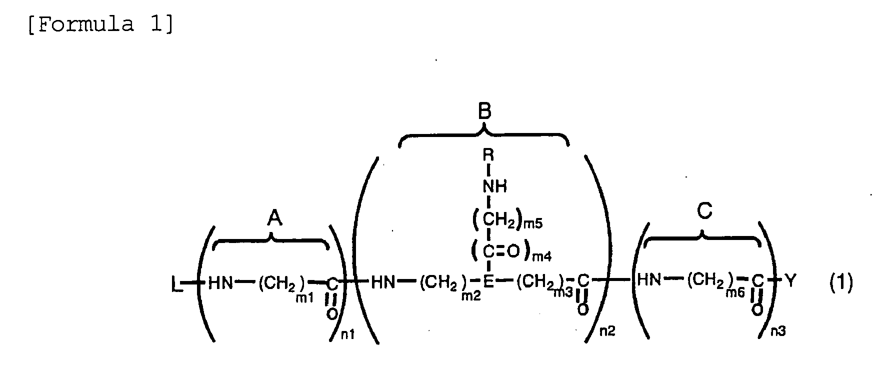 wherein n1 is an integer from 0 to 10, n2 is an integer from 1 to 50, and n3 is an integer from 1 to 10; m1 is an integer from 0 to 100, m2 is an integer from 0 to 100, m3 is an integer from 0 to 100, m4 is an integer of 0 or 1, m5 is an integer from 0 to 100, and m6 is an integer from 0 to 100;
Y is a hydroxyl group or an amino group;
E is N or CH;
R is an amino acid residue, or a peptide residue consisting of 2 to 100 amino acid residues;
L is a hydrogen atom, a group containing a lipid group, a group containing a fatty acid residue, or a group containing a fluorescent group;
when n1 is at least 2, each m1 in repeating unit A may be the same or different;
when n2 is at least 2, each of m2 to m5 and R may be the same or different in each repeating unit B; and
when n3 is at least 2, each m6 in repeating unit C may be the same or different.

2. A compound of claim 1, wherein L in formula (1) is a group containing a phospholipid group.

3. A compound of claim 1, wherein R in formula (1) is an amino acid residue selected from the group consisting of arginine, lysine and serine; or a peptide residue comprising at least one of these amino acid residues.

4. A compound of claim 1, wherein R in formula (1) is a peptide residue comprising at least one amino acid residue selected from the group consisting of arginine, lysine and serine; said pepetide residue consisting of a total of 2 to 20 amino acid residues.

5. A compound of claim 1, wherein R in formula (1) represents a peptide residue consisting of 2 to 5 arginine residues.

6. A compound of claim 1, wherein in formula (1) n1 is an integer from 0 to 2, n2 is an integer from 1 to 10, and n3 is an integer from 0 to 2.

7. A compound of claim 1, wherein in formula (1) E is N; m2 is 2, m3 and m4 are 1, and m5 is 5.

8. A compound of claim 1, wherein in formula (1) E is CH; m2, m3 and m4 are 0, and m5 is 4.

9. A compound represented by the following formula (1a): 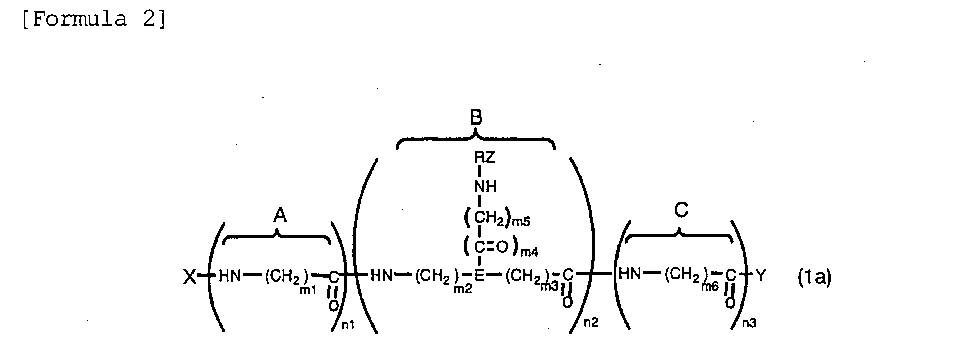 wherein n1 to n3, m1 to m6, E and R are as defined in claim 1,
Y is a hydroxyl group;
X is a protecting group;
Z is a protecting group Za different from protecting group X, or
a protecting group Zb identical to protecting group X;
RZ represents such a protecting group Z bonded to a functional group of amino acid or peptide residue R;
when n1 is at least 2, each m1 in repeating unit A may be the same or different;
when n2 is at least 2, each of m2 to m5 and RZ may be the same or different in each repeating unit B; and
when n3 is at least 2, each m6 in repeating unit C may be the same or different.

10. A compound of claim 9, wherein in formula (1a) X is a tertiary-butoxycarbonyl group or a 9-fluorenylmethoxycarbonyl group.

11. A compound of claim 9, wherein R in formula (1) is an amino acid residue selected from the group consisting of arginine, lysine and serine, or a peptide residue comprising at least one of these amino acid residues; said peptide residue consisting of a total of 2 to 20 amino acid residues.

12. A compound of claim 9, where R in formula (1) is a peptide residue comprising at least one amino acid residue selected from the group consisting of arginine, lysine and serine; said peptide residue consisting of a total of 2 to 20 amino acid residues.

13. A compound of claim 9, wherein R in formula (1) represents a peptide residue consisting of 2 to 5 arginine residues.

14. A compound of claim 9, wherein in formula (1) n1 is an integer from 0 to 2, n2 is an integer from 1 to 10, and n3 is an integer from 0 to 2.

15. A compound of claim 9, wherein in formula (1) E is N; m2 is 2, m3 and m4 are 1, and m5 is 5.

16. A compound of claim 9, wherein in formula (1) E is CH; m2, m3 and m4 are 0, and m5 is 4.

17. A conjugated compound synthesized by a condensation reaction between a compound of claim 9 and a compound containing an amino group or a hydroxyl group.

18. A conjugated compound of claim 17, wherein the compound containing an amino group is a PNA monomer or a PNA oligomer.

19. A process for producing a compound represented by the following formula (1) 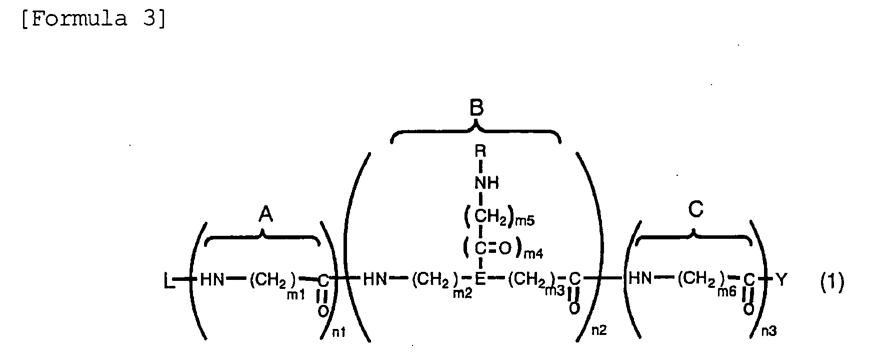 wherein n1 is an integer from 0 to 10, n2 is an integer from 1 to 50, and n3 is an integer from 1 to 10; m1 is an integer from 0 to 100, m2 is an integer from 0 to 100, m3 is an integer from 0 to 100, m4 is an integer of 0 or 1, m5 is an integer from 0 to 100, and m6 is an integer from 0 to 100;
Y is a hydroxy group or an amino group;
E is N or CH;
R is an amino acid residue, or a peptide residue consisting of 2 to 100 amino acid residues;
L is a hydrogen atom, a group containing a lipid group, a group containing a fatty acid residue, or a group containing a fluorescent group;
when n1 is at least 2, each m1 in repeating unit A may be the same or different;
when n2 is at least 2, each of m2 to m5 and R may be the same or different in each repeating unit B; and
when n3 is at least 2, each m6 in repeating unit C may be the same or different;
the process comprising the steps 1-1 to 1-5 below:
Step 1-1: a step of condensing a compound represented by the following formula (I) 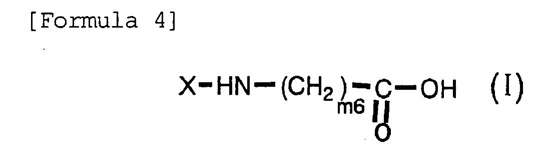 wherein m6 is as defined above,
with a solid-phase resin or a solid-phase compound, removing protecting group(s) from the compound condensed with solid-phase resin or solid-phase compound, and subjecting the compound represented by formula (I) to such condensation n3-1 more times so as to polymerize it, thereby obtaining a compound represented by the following formula (i) 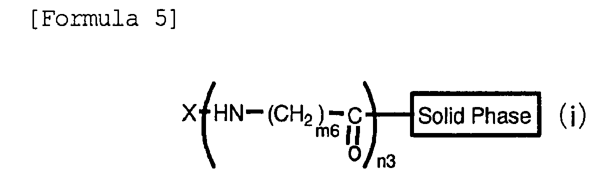 wherein n3, m6 and X are as defined above; and Solid Phase represents a solid-phase resin or a solid-phase compound;
Step 1-2: a step of condensing n2 times a compound represented by the following formula (II) 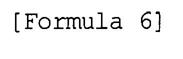 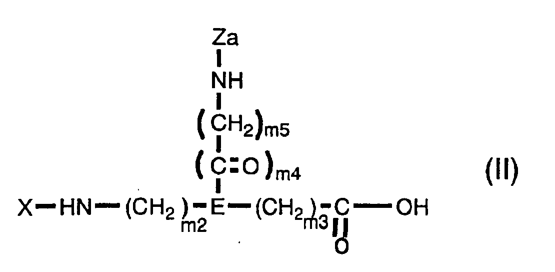 wherein m2 to m5 and X are as defined above, and Za is a protecting group different from X,
with the compound represented by formula (i), thereby obtaining a compound represented by the following formula (ii) 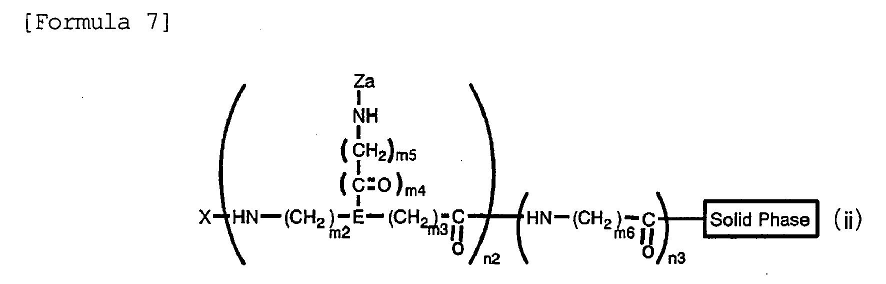 wherein n2, n3, m2 to m6, X, Za and Solid Phase are as defined above;
Step 1-3: a step of condensing n1 times a compound represented by the following formula (III) 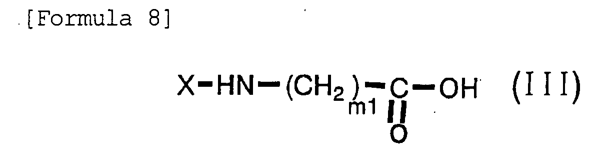 wherein m1 and X are as defined above,
with the compound represented by formula (ii), thereby obtaining a compound represented by the following formula (iii) 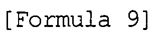 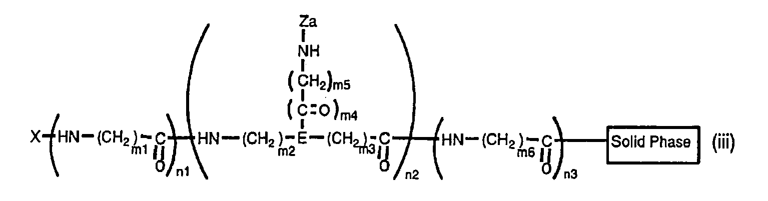 wherein n1 to n3, m1 to m6, X, Za and Solid Phase are as defined above;
Step 1-4: a step of condensing, 1 to 100 times with the compound represented by formula (iii), one or more amino acids wherein protecting group Za is bonded to a functional group other than a carboxyl group bonded to an α carbon atom, thereby obtaining a compound represented by the following formula (iv) 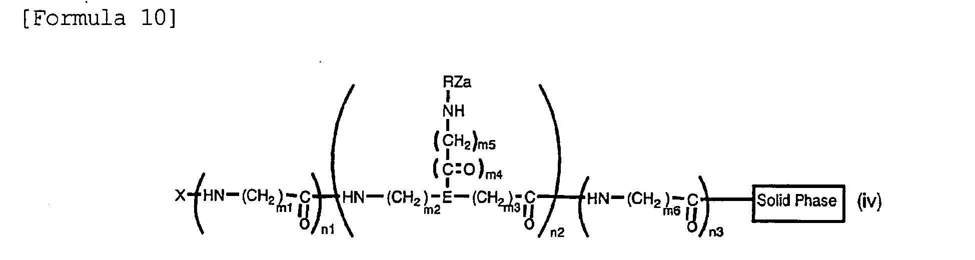 wherein n1 to n3, m1 to m6, X, RZa and Solid Phase are as defined above; and
Step 1-5: a step of obtaining a compound represented by formula (1) by removing the solid-phase resin or solid-phase compound from the compound represented by formula (iv) to form terminal - COOH or -CONH₂ groups, and removing protecting groups X and Za.

20. A process for producing a compound represented by the following formula (1a) 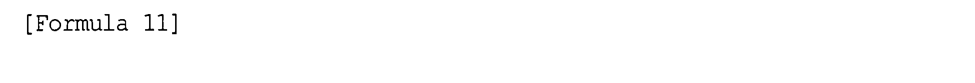 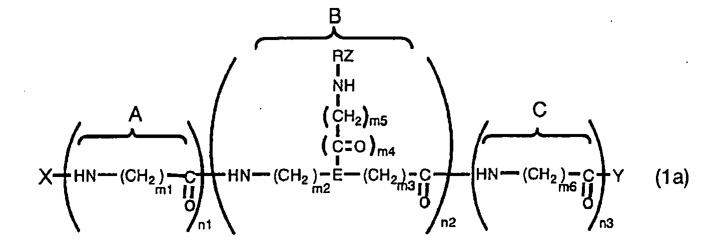 wherein n1 to n3, m1 to m6, E, X and RZ are as defined above;
Y is a hydroxyl group;
when n1 is at least 2, each m1 in repeating unit A may be the same or different;
when n2 is at least 2, each of m2 to m5 and RZ may be the same or different in each repeating unit B; and
when n3 is at least 2, each m6 in repeating unit C may be the same or different;
the process comprising the steps 3-1 to 3-5 below:
Step 3-1: a step of subjecting a compound represented by the following formula (I) 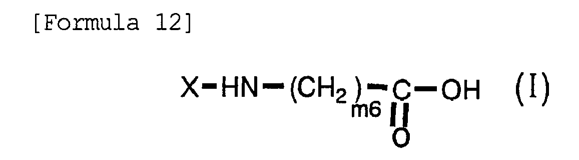 wherein m6 is as defined above,
to condensation polymerization with a solid-phase resin or a solid-phase compound containing an amino group, removing protecting group X from the compound condensed with the solid-phase resin or solid-phase compound, and subjecting the compound represented by formula (I) to such condensation n3-1 more times so as to polymerize it, thereby obtaining a compound represented by the following formula (i) 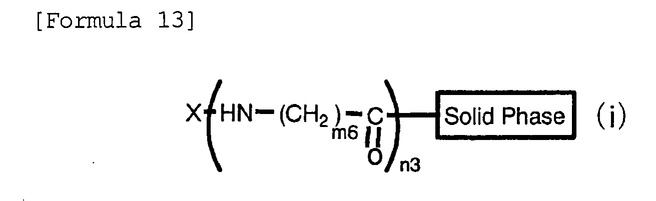 wherein n3, m6 and X are as defined above; and Solid Phase represents a solid-phase resin or a solid-phase compound;
Step 3-2: a step of condensing n2 times a compound represented by the following formula (II) 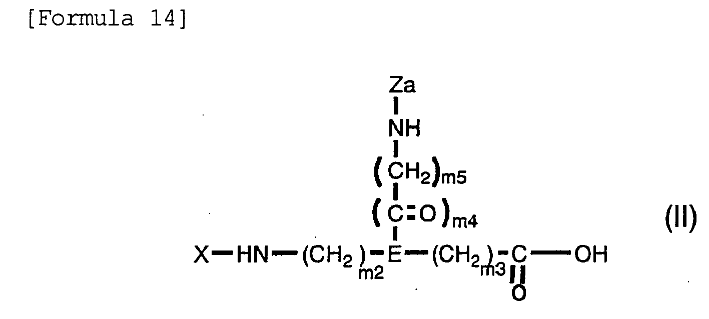 wherein m2 to m5, X and Za are as defined above,
with the compound represented by formula (i), thereby obtaining a compound represented by the following formula (ii) 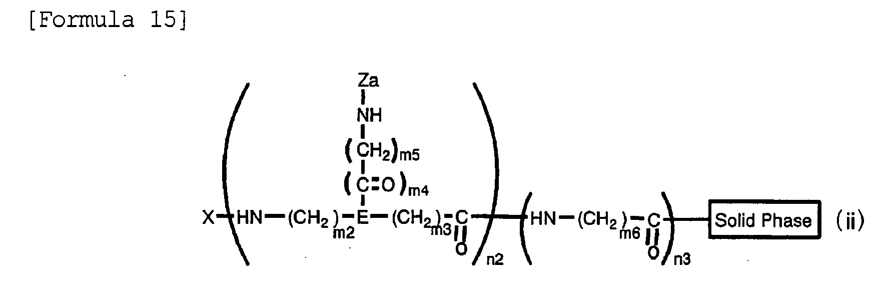 wherein n2, n3, m2 to m6, X, Za and Solid Phase are as defined above;
Step 3-3: a step of condensing n1 times a compound represented by the following formula (III) 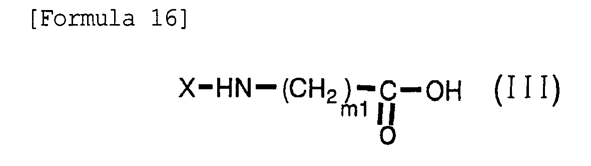 wherein m1 and X are as defined above,
with the compound represented by formula (ii), thereby obtaining a compound represented by the following formula (iii) 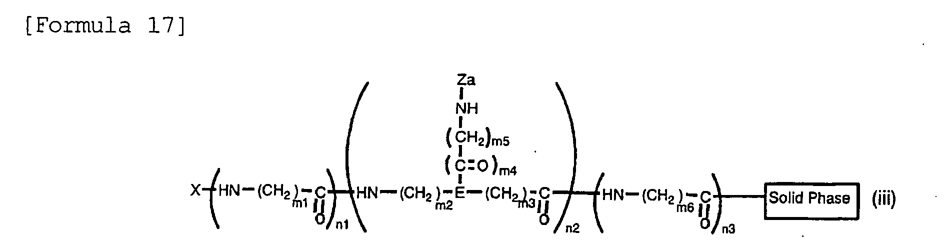 wherein n1 to n3, m1 to m6, X, Za and Solid Phase are as defined above;
Step 3-4: a step of obtaining a compound represented by the following formula (iv) 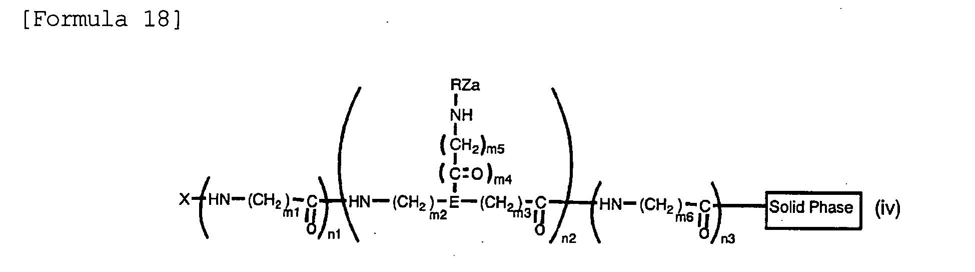 wherein n1 to n3, m1 to m6, X, RZa and Solid Phase are as defined above,
by condensing, 1 to 100 times the compound represented by formula (iii), with one or more amino acids in which protecting group Z is bonded to an amino group; and
Step 3-5: a step of obtaining a compound represented by formula (1a) by substituting as necessary protecting group Za of the compound represented by formula (iv) with protecting group Zb, and removing the solid-phase resin or solid-phase compound to form terminal -COOH without removing protecting groups X and Z.
